# EUROPEAN PATENT APPLICATION

(11) **EP 4 813 983 A1**
(43) Date of publication of application: **30.09.2026**
(21) Application number: 24894344.1
(22) Date of filing: 23.09.2024
(51) Int. Cl.: C07D 405/14, C07D 409/14, C07D 403/14, H10K 85/60, H10K 50/11

(54) **HETEROCYCLIC COMPOUND, AND ORGANIC LIGHT-EMITTING DEVICE AND COMPOSITION FOR ORGANIC LAYER, EACH COMPRISING SAME**

(30) Priority: 22.11.2023 KR 20230163718
(71) Applicant: LT Materials Co., Ltd., Yongin-si, Gyeonggi-do 17118 (KR)
(72) Inventor: LEE, Kyung Yeon, Yongin-si, Gyeonggi-do 17118 (KR); PARK, Geon Yu, Yongin-si, Gyeonggi-do 17118 (KR); NO, Young Seok, Yongin-si, Gyeonggi-do 17118 (KR); KIM, Dong Jun, Yongin-si, Gyeonggi-do 17118 (KR); CHOI, Dae Hyuk, Yongin-si, Gyeonggi-do 17118 (KR)
(74) Representative: Meissner Bolte Partnerschaft mbB
(86) International application number: PCT/KR2024/014250
(87) International publication number: WO 2025/110448

(57) **Abstract**

The present invention relates to a heterocyclic compound represented by Chemical Formula 1, an organic light-emitting device including the same, and a composition for an organic material layer including the same.

## Description

### [Technical Field]

The present application claims priority to Korean Patent Application No. 10-2023-0163718, filed on November 22, 2023, and all contents disclosed in the specification of the Korean patent application are incorporated herein by reference.

The present invention relates to a heterocyclic compound, an organic light-emitting device comprising the same, and a composition for an organic material layer.

### [Background Art]

An organic light-emitting device is a type of self-emissive display device, and has advantages such as a wide viewing angle, excellent contrast, and a fast response speed.

The organic light-emitting device has a structure in which an organic thin film is disposed between two electrodes. When a voltage is applied to the organic light-emitting device having such a structure, electrons and holes injected from the two electrodes recombine in the organic thin film to form pairs, and then emit light while annihilating. The organic thin film may be composed of a single layer or multiple layers as needed.

The material of the organic thin film may have a light-emitting function as needed. For example, as the organic thin film material, a compound that itself can constitute a emission layer alone may be used, or a compound that can serve as a host or a dopant in a host-dopant type emission layer may be used. In addition, as the material of the organic thin film, compounds capable of performing roles such as hole injection, hole transport, electron blocking, hole blocking, electron transport, and electron injection may be used.

In order to improve the performance, lifetime, or efficiency of the organic light-emitting device, continuous development of materials for the organic thin film is required.

### [Prior Art]

[Patent Document]
U.S. Patent No. 4,356,429

### [Detailed Description of the Invention]

### [Technical Problem]

An object of the present invention is to provide a heterocyclic compound, an organic light-emitting device comprising the same, and a composition for an organic material layer.

### [Technical Solution]

In order to achieve the above object, the present invention provides a heterocyclic compound represented by the following Chemical Formula 1. 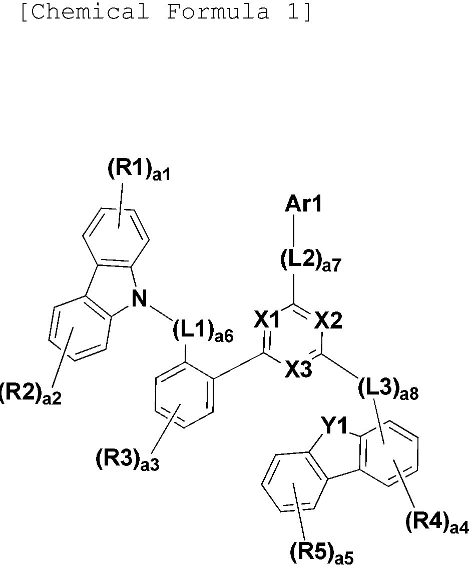 in Chemical Formula 1,
X1 is N; or CRa,
X2 is N; or CRb,
X3 is N; or CRc,
at least two of X1 to X3 are N,
Y1 is O; S; or NRd,
R1 to R5 and Ra to Rd are the same as or different from each other, and are each independently selected from the group consisting of hydrogen; deuterium; halogen; cyano group; substituted or unsubstituted C1 to C60 alkyl group; substituted or unsubstituted C2 to C60 alkenyl group; substituted or unsubstituted C2 to C60 alkynyl group; substituted or unsubstituted C1 to C60 alkoxy group; substituted or unsubstituted C3 to C60 cycloalkyl group; substituted or unsubstituted C2 to C60 heterocycloalkyl group; substituted or unsubstituted C6 to C60 aryl group; substituted or unsubstituted C2 to C60 heteroaryl group; -P(=O)R101R102; -SiR101R102R103; and -NR101R102, or two or more adjacent groups may be bonded to each other to form a substituted or unsubstituted C6 to C60 aromatic hydrocarbon ring; or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group.
a1 is an integer of 0 to 4, and when a1 is 2 or more, R1s are the same as or different from each other,
a2 is an integer of 0 to 4, and when a2 is 2 or more, R2s are the same as or different from each other,
a3 is an integer of 0 to 4, and when a3 is 2 or more, R3s are the same as or different from each other,
a4 is an integer of 0 to 3, and when a4 is 2 or more, R4s are the same as or different from each other,
a5 is an integer of 0 to 4, and when a5 is 2 or more, R5s are the same as or different from each other,
Ar1 is a substituted or unsubstituted C6 to C60 aryl group,
L1 and L2 are the same as or different from each other, and each independently a direct bond; a substituted or unsubstituted C6 to C60 arylene group; or a C2 to C60 heteroarylene group,
a6 is an integer of 0 to 5, and when a6 is 2 or more, L1s are the same as or different from each other,
a7 is an integer of 0 to 5, and when a7 is 2 or more, L2s are the same as or different from each other,
L3 is a substituted or unsubstituted C6 to C60 arylene group; or a C2 to C60 heteroarylene group,
a8 is an integer of 1 to 5, and when a8 is 2 or more, L3s are the same as or different from each other.

Further, the present invention provides an organic light-emitting device in which the organic material layer further comprises a heterocyclic compound represented by Chemical Formula 2 or Chemical Formula 3 below. 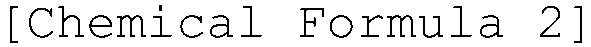 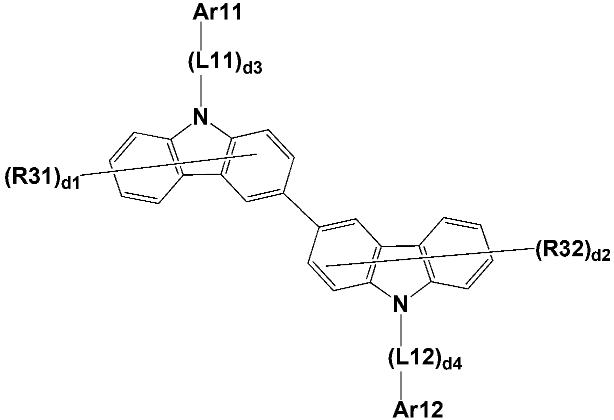 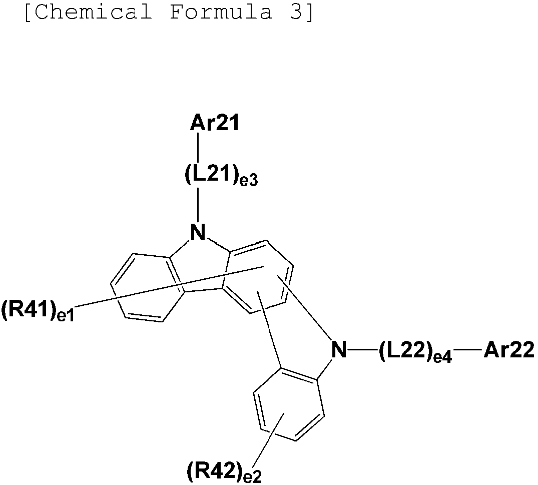 in Chemical Formulas 2 and 3,
R31, R32, R41, and R42 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - P(=O)R201R202; -SiR201R202R203; and -NR201R202, or two or more adjacent groups may be bonded to each other to form a substituted or unsubstituted C6 to C60 aromatic hydrocarbon ring or a substituted or unsubstituted C2 to C60 heterocyclic ring, wherein R201, R202, and R203 are the same as or different from each other, and each independently a substituted or unsubstituted C1 to C60 alkyl group, a substituted or unsubstituted C6 to C60 aryl group, or a substituted or unsubstituted C2 to C60 heteroaryl group,
d1 is an integer of 0 to 7, and when d1 is 2 or more, R31s are the same as or different from each other,
d2 is an integer of 0 to 7, and when d2 is 2 or more, R32s are the same as or different from each other,
e1 is an integer of 0 to 6, and when e1 is 2 or more, R41s are the same as or different from each other,
e2 is an integer of 0 to 4, and when e2 is 2 or more, R42s are the same as or different from each other,
Ar11, Ar12, Ar21, and Ar22 are the same as or different from each other, and each independently a cyano group; - SiR201R202R203; a substituted or unsubstituted C6 to C60 aryl group; or a substituted or unsubstituted C2 to C60 heteroaryl group, wherein R201, R202, and R203 are the same as or different from each other, and each independently a substituted or unsubstituted C1 to C60 alkyl group, a substituted or unsubstituted C6 to C60 aryl group, or a substituted or unsubstituted C2 to C60 heteroaryl group,
L11, L12, L21, and L22 are the same as or different from each other, and each independently a direct bond; a substituted or unsubstituted C6 to C60 arylene group; or a substituted or unsubstituted C2 to C60 heteroarylene group,
d3 is an integer of 0 to 5, and when d3 is 2 or more, Llls are the same as or different from each other,
d4 is an integer of 0 to 5, and when d4 is 2 or more, L12s are the same as or different from each other,
e3 is an integer of 0 to 5, and when e3 is 2 or more, L21s are the same as or different from each other.
e4 is an integer of 0 to 5, and when e4 is 2 or more, L22s are the same as or different from each other.

Further, the present invention provides a composition for an organic material layer comprising the heterocyclic compound represented by Chemical Formula 1 and the heterocyclic compound represented by Chemical Formula 2 or Chemical Formula 3.

### [Advantageous Effects]

The compounds described in the present specification may be used as materials for the organic material layer of an organic light-emitting device. The compounds may serve as a hole injection layer material, a hole transport layer material, a emission layer material, an electron transport layer material, or an electron injection layer material in the organic light-emitting device. In particular, the compounds may be used as materials for the emission layer of the organic light-emitting device, and may be used alone as light-emitting materials, or may be used as a host material or a dopant material of the emission layer.

Specifically, the compound may be used alone as a light-emitting material, or may be used as a host material or a dopant material of the emission layer. When the heterocyclic compound represented by Chemical Formula 1 is used in the organic material layer, the driving voltage of the organic light-emitting device may be reduced, the luminous efficiency may be improved, and the lifetime characteristics may be enhanced.

### [Brief Description of Drawings]

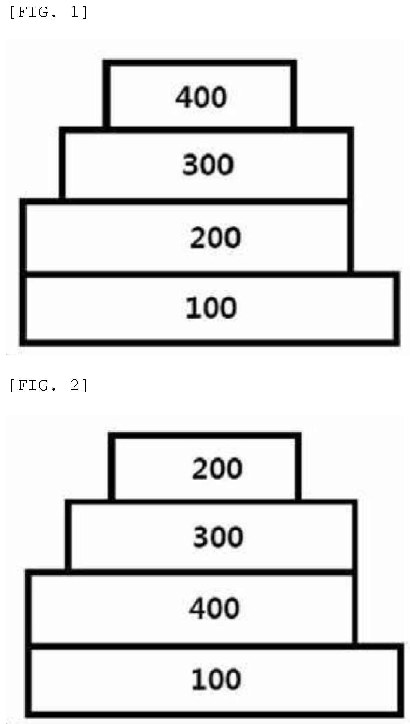

FIGS. 1 to 3 are schematic diagrams each illustrating a stacked structure of an organic light-emitting device according to an embodiment of the present invention.

### [Best Mode]

Hereinafter, the present specification will be described in more detail.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, the term "substituted or unsubstituted" means substituted with or unsubstituted with one or more substituents selected from the group consisting of deuterium; halogen; cyano; a linear or branched C1 to C60 alkyl group; a linear or branched C2 to C60 alkenyl group; a linear or branched C2 to C60 alkynyl group; a linear, branched, or cyclic C1 to C60 alkoxy group; a monocyclic or polycyclic C3 to C60 cycloalkyl group; a monocyclic or polycyclic C2 to C60 heterocycloalkyl group; a monocyclic or polycyclic C6 to C60 aryl group; a monocyclic or polycyclic C2 to C60 heteroaryl group; -SiRR'R"; -P(=O)RR'; a C1 to C20 alkylamino group; a monocyclic or polycyclic C6 to C60 arylamino group; and a monocyclic or polycyclic C2 to C60 heteroarylamino group; or substituted with or unsubstituted with a substituent in which two or more substituents selected from the substituents exemplified above are linked to each other, wherein R, R', and R" are the same as or different from each other and are each independently a substituted or unsubstituted C1 to C60 alkyl group, a substituted or unsubstituted C6 to C60 aryl group, or a substituted or unsubstituted C2 to C60 heteroaryl group.

In the present specification, the halogen may be fluorine, chlorine, bromine or iodine.

In the present specification, the alkyl group includes a linear or branched chain having 1 to 60 carbon atoms, and may be further substituted with another substituent. The alkyl group may have 1 to 60 carbon atoms, specifically 1 to 40 carbon atoms, and more specifically 1 to 20 carbon atoms. Specific examples thereof include a methyl group; an ethyl group; an n-propyl group; an isopropyl group; an n-butyl group; an isobutyl group; a tert-butyl group; a sec-butyl group; a 1-methylbutyl group; a 1-ethylbutyl group; an n-pentyl group; an isopentyl group; a neopentyl group; a tert-pentyl group; an n-hexyl group; a 1-methylpentyl group; a 2-methylpentyl group; a 4-methyl-2-pentyl group; a 3,3-dimethylbutyl group; a 2-ethylbutyl group; an n-heptyl group; a 1-methylhexyl group; a cyclopentylmethyl group; a cyclohexylmethyl group; an n-octyl group; a tert-octyl group; a 1-methylheptyl group; a 2-ethylhexyl group; a 2-propylpentyl group; an n-nonyl group; a 2,2-dimethylheptyl group; a 1-ethylpropyl group; a 1,1-dimethylpropyl group; an isohexyl group; a 4-methylhexyl group; and a 5-methylhexyl group, but are not limited thereto.

In the present specification, the alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group; a 1-propenyl group; an isopropenyl group; a 1-butenyl group; a 2-butenyl group; a 3-butenyl group; a 1-pentenyl group; a 2-pentenyl group; a 3-pentenyl group; a 3-methyl-1-butenyl group; a 1,3-butadienyl group; an allyl group; a 1-phenylvinyl-1-yl group; a 2-phenylvinyl-1-yl group; a 2,2-diphenylvinyl-1-yl group; a 2-phenyl-2-(naphthyl-1-yl)vinyl-1-yl group; a 2,2-bis(diphenyl-1-yl)vinyl-1-yl group; a stilbenyl group; a styrenyl group, and the like, but are not limited thereto.

In the present specification, the alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, the alkoxy group may be linear, branched, or cyclic. The number of carbon atoms in the alkoxy group is not particularly limited, but is preferably 1 to 20 carbon atoms. Specifically, examples thereof include a methoxy group; an ethoxy group; an n-propoxy group; an isopropoxy group; an n-butoxy group; an isobutoxy group; a tert-butoxy group; a sec-butoxy group; an n-pentyloxy group; a neopentyloxy group; an isopentyloxy group; an n-hexyloxy group; a 3,3-dimethylbutoxy group; a 2-ethylbutoxy group; an n-octyloxy group; an n-nonyloxy group; an n-decyloxy group; a benzyloxy group; and a p-methylbenzyloxy group, but are not limited thereto.

In the present specification, the cycloalkyl group includes a monocyclic or polycyclic group having 3 to 60 carbon atoms, and m ay be further substituted with another substituent. Herein, the term "polycyclic" means a group in which the cycloalkyl group is directly linked to or fused with another ring group. Herein, the other ring group may be a cycloalkyl group, or may be another type of ring group, for example, a heterocycloalkyl group, an aryl group, or a heteroaryl group. The cycloalkyl group may have 3 to 60 carbon atoms, specifically 3 to 40 carbon atoms, and more specifically 5 to 20 carbon atoms. Specific examples thereof include a cyclopropyl group; a cyclobutyl group; a cyclopentyl group; a 3-methylcyclopentyl group; a 2,3-dimethylcyclopentyl group; a cyclohexyl group; a 3-methylcyclohexyl group; a 4-methylcyclohexyl group; a 2,3-dimethylcyclohexyl group; a 3,4,5-trimethylcyclohexyl group; a 4-tert-butylcyclohexyl group; a cycloheptyl group; and a cyclooctyl group, but are not limited thereto.

In the present specification, the heterocycloalkyl group includes a monocyclic or polycyclic group having 2 to 60 carbon atoms and comprising O, S, Se, N, or Si as a heteroatom, and may be further substituted with another substituent. Herein, the term "polycyclic" means a group in which the heterocycloalkyl group is directly linked to or fused with another ring group. Herein, the other ring group may be a heterocycloalkyl group, or may be another type of ring group, for example, a cycloalkyl group, an aryl group, or a heteroaryl group. The heterocycloalkyl group may have 2 to 60 carbon atoms, specifically 2 to 40 carbon atoms, and more specifically 3 to 20 carbon atoms.

In the present specification, the aryl group includes a monocyclic or polycyclic group having 6 to 60 carbon atoms, and may be further substituted with another substituent. Herein, the term "polycyclic" means a group in which the aryl group is directly linked to or fused with another ring group. Herein, the other ring group may be an aryl group, or may be another type of ring group, for example, a cycloalkyl group, a heterocycloalkyl group, or a heteroaryl group. The aryl group may include a spiro group. The aryl group may have 6 to 60 carbon atoms, specifically 6 to 40 carbon atoms, and more specifically 6 to 20 carbon atoms. Specific examples of the aryl group include a phenyl group; a biphenyl group; a triphenyl group; a naphthyl group; an anthryl group; a chrysenyl group; a phenanthrenyl group; a perylenyl group; a fluoranthenyl group; a triphenylenyl group; a phenalenyl group; a pyrenyl group; a tetracenyl group; a pentacenyl group; a fluorenyl group; an indenyl group; an acenaphthylenyl group; a benzofluorenyl group; a spirobifluorenyl group; a 2,3-dihydro-1H-indenyl group; fused ring groups thereof; and the like, but are not limited thereto.

In the present specification, the phosphine oxide group is represented by -P(=O)R101R102, wherein R101 and R102 are the same as or different from each other and are each independently hydrogen; deuterium; a halogen group; an alkyl group; an alkenyl group; an alkoxy group; a cycloalkyl group; an aryl group; or a substituent comprising at least one heterocyclic group. Specifically, the phosphine oxide group may be substituted with an aryl group, wherein the examples described above for the aryl group may be applied. For example, the phosphine oxide group includes, but is not limited to, a diphenylphosphine oxide group and a dinaphthylphosphine oxide group.

In the present specification, the silyl group is a substituent comprising Si, wherein the Si atom is directly linked as a radical, and is represented by -SiR101R102R103, wherein R101 to R103 are the same as or different from each other and are each independently hydrogen; deuterium; a halogen group; an alkyl group; an alkenyl group; an alkoxy group; a cycloalkyl group; an aryl group; or a substituent comprising at least one heterocyclic group. Specific examples of the silyl group include a trimethylsilyl group; a triethylsilyl group; a tert-butyldimethylsilyl group; a vinyldimethylsilyl group; a propyldimethylsilyl group; a triphenylsilyl group; a diphenylsilyl group; and a phenylsilyl group, but are not limited thereto.

In the present specification, the fluorenyl group may be substituted, and adjacent substituents may be bonded to each other to form a ring.

When the fluorenyl group is substituted, it may be, for example, 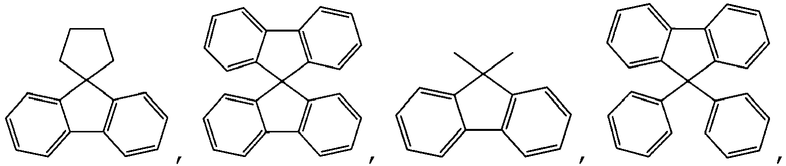 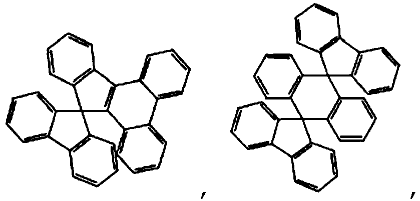 but is not limited thereto.

In the present specification, the spiro group is a group comprising a spiro structure and may have 15 to 60 carbon atoms. For example, the spiro group may include a structure in which a 2,3-dihydro-1H-indene group or a cyclohexane group is spiro-bonded to a fluorenyl group. Specifically, the spiro group may include any one of the groups represented by the following structural formulas.

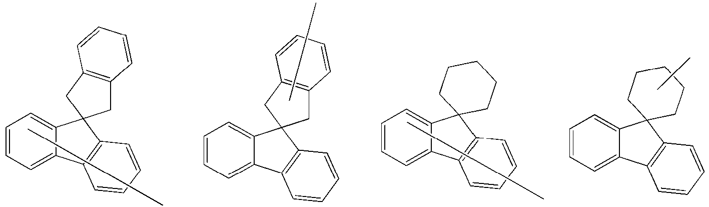

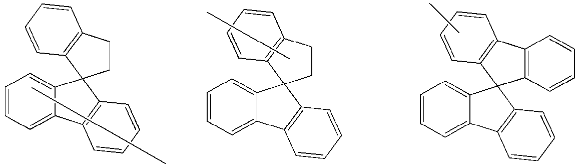

In the present specification, the heteroaryl group includes a monocyclic or polycyclic group having 2 to 60 carbon atoms and comprising S, O, Se, N, or Si as a heteroatom, and may be further substituted with another substituent. Herein, the term "polycyclic" means a group in which the heteroaryl group is directly linked to or fused with another ring group. Herein, the other ring group may be a heteroaryl group or may be another type of ring group, for example, a cycloalkyl group; a heterocycloalkyl group; or an aryl group. The heteroaryl group may have 2 to 60 carbon atoms, specifically 2 to 40 carbon atoms, and more specifically 3 to 25 carbon atoms. Specific examples of the heteroaryl group include a pyridyl group; a pyrrolyl group; a pyrimidyl group; a pyridazinyl group; a furanyl group; a thiophenyl group; an imidazolyl group; a pyrazolyl group; an oxazolyl group; an isoxazolyl group; a thiazolyl group; an isothiazolyl group; a triazolyl group; a furazanyl group; an oxadiazolyl group; a thiadiazolyl group; a dithiazolyl group; a tetrazolyl group; a pyranyl group; a thiopyranyl group; a diazinyl group; an oxazinyl group; a thiazinyl group; a dioxinyl group; a triazinyl group; a tetrazinyl group; a quinolyl group; an isoquinolyl group; a quinazolinyl group; an isoquinazolinyl group; a quinazolyl group; a naphthyridyl group; an acridinyl group; a phenanthridinyl group; an imidazopyridinyl group; a diazanaphthalenyl group; a triazaindenyl group; a 2-indolyl group; an indolizinyl group; a benzothiazolyl group; a benzoxazolyl group; a benzimidazolyl group; a benzothiophenyl group; a benzofuranyl group; a dibenzothiophenyl group; a dibenzofuranyl group; a carbazolyl group; a benzocarbazolyl group; a dibenzocarbazolyl group; a phenazinyl group; a dibenzosilolyl group; a spirobi(dibenzosilolyl) group; a dihydrophenazinyl group; a phenoxazinyl group; a phenanthridyl group; a thienyl group; an indolo[2,3-a]carbazolyl group; an indolo[2,3-b]carbazolyl group; an indolinyl group; a 10,11-dihydrodibenzo[b,f]azepinyl group; a 9,10-dihydroacridinyl group; a phenanthrazinyl group; a phenothiazinyl group; a phthalazinyl group; a naphthyridinyl group; a phenanthrolinyl group; a benzo[c][1,2,5]thiadiazolyl group; a 5,10-dihydrodibenzo[b,e][1,4]azasilinyl group; a pyrazolo[1,5-c]quinazolinyl group; a pyrido[1,2-b]indazolyl group; a pyrido[1,2-a]imidazo[1,2-e]indolinyl group; a 5,11-dihydroindeno[1,2-b]carbazolyl group; and the like, but are not limited thereto.

In the present specification, the amine group may be selected from the group consisting of a monoalkylamino group; a monoarylamino group; a monoheteroarylamino group; -NH₂; a dialkylamino group; a diarylamino group; a diheteroarylamino group; an alkylarylamino group; an alkylheteroarylamino group; and an arylheteroarylamino group, and the number of carbon atoms is not particularly limited, but is preferably 1 to 30. Specific examples of the amine group include a methylamino group; a dimethylamino group; an ethylamino group; a diethylamino group; a phenylamino group; a naphthylamino group; a biphenylamino group; a dibiphenylamino group; an anthracenylamino group; a 9-methyl-anthracenylamino group; a diphenylamino group; a phenylnaphthylamino group; a ditolylamino group; a phenyltolylamino group; a triphenylamino group; a biphenylnaphthylamino group; a phenylbiphenylamino group; a biphenylfluorenylamino group; a phenyltriphenylenylamino group; and a biphenyltriphenylenylamino group, but are not limited thereto.

In the present specification, the arylene group refers to a divalent group having two bonding sites in an aryl group, i.e., a divalent radical. Except for being divalent, the description of the aryl group described above may be applied thereto. Further, the heteroarylene group refers to a divalent group having two bonding sites in a heteroaryl group, i.e., a divalent radical. Except for being divalent, the description of the heteroaryl group described above may be applied thereto.

In the present specification, an "adjacent" group may refer to a substituent substituted on an atom directly connected to the atom on which the corresponding substituent is substituted; a substituent positioned closest to the corresponding substituent in terms of steric structure; or another substituent substituted on the atom on which the corresponding substituent is substituted. For example, two substituents substituted at the ortho position on a benzene ring, and two substituents substituted on the same carbon in a nonaromatic ring may be interpreted as being "adjacent" to each other.

In the present specification, "when a substituent is not indicated in the structure of a Formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a Formula or compound" may mean that all the positions that may be reached by the substituent are hydrogen or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a Formula or compound", when the content of deuterium is 0%, the content of hydrogen is 100%, and all the substituents do not explicitly exclude deuterium such as hydrogen, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or 2H.

In an exemplary embodiment of the present application, the isotope means an atom with the same atomic number (Z), but different mass numbers (A), and the isotope may also be interpreted as an element which has the same number of protons, but different number of neutrons.

In an exemplary embodiment of the present application, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 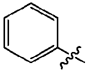 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formulas.

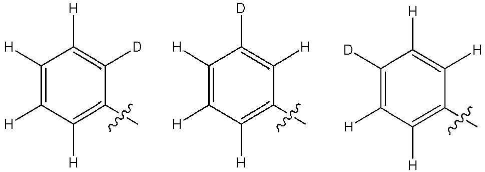

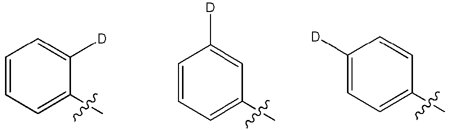

Further, in an exemplary embodiment of the present application, "a phenyl group having a deuterium content of 0%" may mean a phenyl group that does not include a deuterium atom, that is, has five hydrogen atoms.

In the present invention, the C6 to C60 aromatic hydrocarbon ring refers to a compound comprising an aromatic ring consisting of 6 to 60 carbon atoms and hydrogen atoms. Examples thereof include, but are not limited to, phenyl, biphenyl, terphenyl, triphenylenyl, naphthyl, anthracenyl, phenalenyl, phenanthrenyl, fluorenyl, pyrenyl, chrysenyl, perylenyl, and azuleny, as well as all aromatic hydrocarbon ring compounds known in the art that satisfy the above carbon number.

the present invention provides a heterocyclic compound represented by the following Chemical Formula 1. 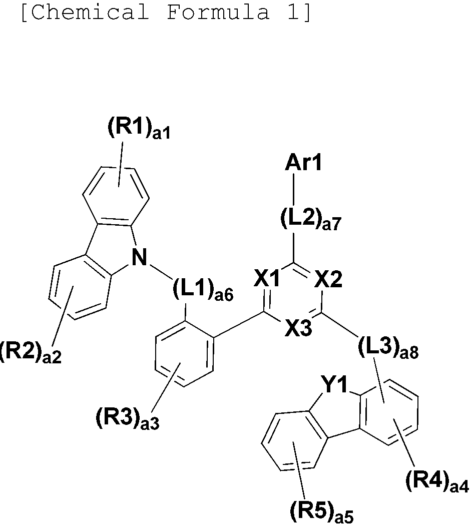 in Chemical Formula 1,
X1 is N; or CRa,
X2 is N; or CRb,
X3 is N; or CRc,
at least two of X1 to X3 are N,
Y1 is O; S; or NRd,
R1 to R5 and Ra to Rd are the same as or different from each other, and are each independently selected from the group consisting of hydrogen; deuterium; halogen; cyano group; substituted or unsubstituted C1 to C60 alkyl group; substituted or unsubstituted C2 to C60 alkenyl group; substituted or unsubstituted C2 to C60 alkynyl group; substituted or unsubstituted C1 to C60 alkoxy group; substituted or unsubstituted C3 to C60 cycloalkyl group; substituted or unsubstituted C2 to C60 heterocycloalkyl group; substituted or unsubstituted C6 to C60 aryl group; substituted or unsubstituted C2 to C60 heteroaryl group; -P(=O)R101R102; -SiR101R102R103; and -NR101R102, or two or more adjacent groups may be bonded to each other to form a substituted or unsubstituted C6 to C60 aromatic hydrocarbon ring; or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group.
a1 is an integer of 0 to 4, and when a1 is 2 or more, R1s are the same as or different from each other,
a2 is an integer of 0 to 4, and when a2 is 2 or more, R2s are the same as or different from each other,
a3 is an integer of 0 to 4, and when a3 is 2 or more, R3s are the same as or different from each other,
a4 is an integer of 0 to 3, and when a4 is 2 or more, R4s are the same as or different from each other,
a5 is an integer of 0 to 4, and when a5 is 2 or more, R5s are the same as or different from each other,
Ar1 is a substituted or unsubstituted C6 to C60 aryl group,
L1 and L2 are the same as or different from each other, and each independently a direct bond; a substituted or unsubstituted C6 to C60 arylene group; or a C2 to C60 heteroarylene group,
a6 is an integer of 0 to 5, and when a6 is 2 or more, L1s are the same as or different from each other,
a7 is an integer of 0 to 5, and when a7 is 2 or more, L2s are the same as or different from each other,
L3 is a substituted or unsubstituted C6 to C60 arylene group; or a C2 to C60 heteroarylene group,
a8 is an integer of 1 to 5, and when a8 is 2 or more, L3s are the same as or different from each other.

In one embodiment of the present invention, the heterocyclic compound represented by Chemical Formula 1 may be represented by any one of the following Chemical Formulas 1-1 to 1-4.

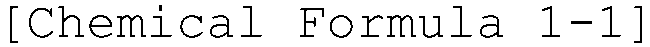

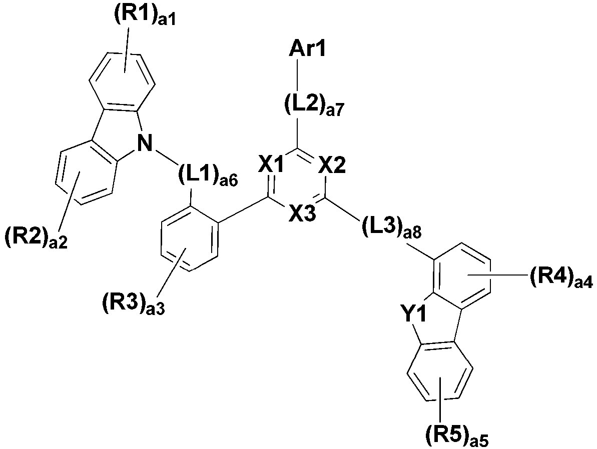

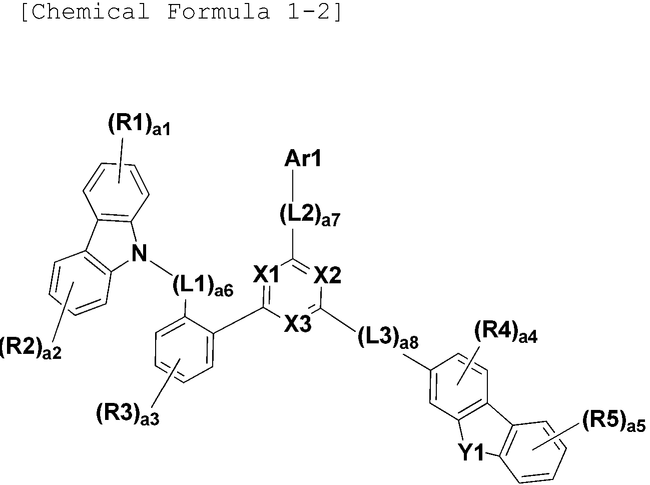

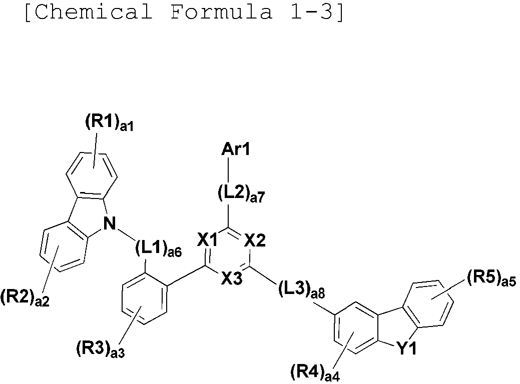

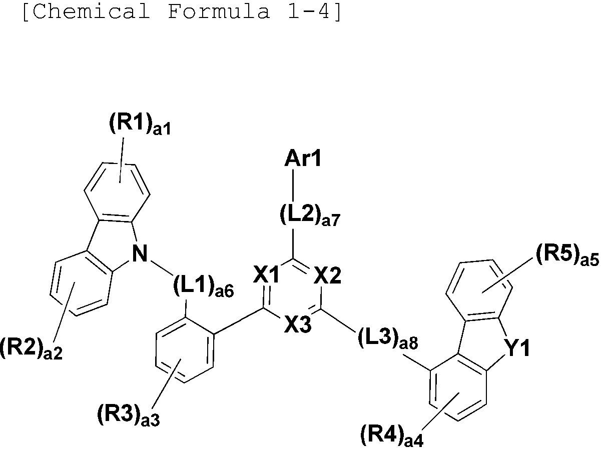

In Chemical Formulas 1-1 to 1-4,
X1 to X3, Y1, R1 to R5, L1 to L3, Ar1, and a1 to a8 are the same as defined in Chemical Formula 1.

In one embodiment of the present invention, X1 to X3 may all be N.

In another embodiment of the present invention, X1 and X2 may be N, and X3 may be CRc.

In another embodiment of the present invention, X1 and X3 may be N, and X2 may be CRb.

In another embodiment of the present invention, X2 and X3 may be N, and X1 may be CRa.

In one embodiment of the present invention, the heterocyclic compound represented by Chemical Formula 1 may be represented by any one of the following Chemical Formulas 1-5 to 1-7. 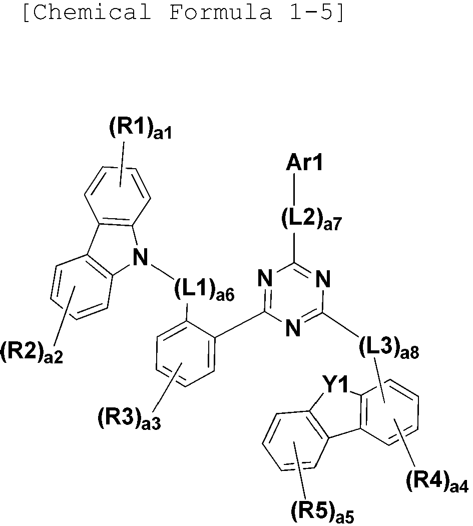 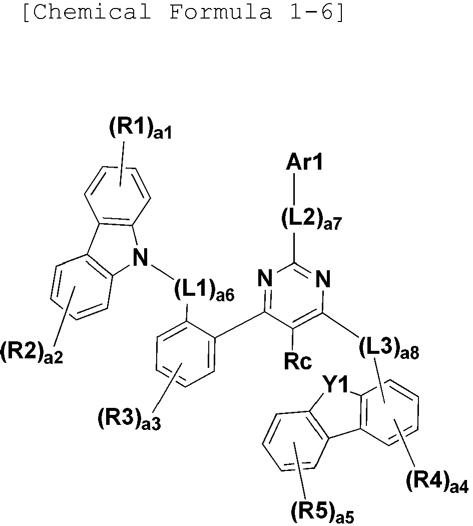 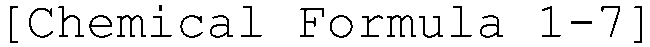 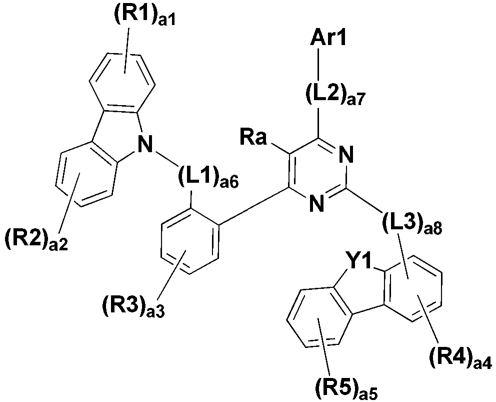 in Chemical Formulas 1-5 to 1-7,
R1 to R5, Ra, Rc, Y1, L1 to L3, Ar1, and a1 to a8 are the same as defined in Chemical Formula 1.

In one embodiment of the present invention, Y1 may be O.

In another embodiment of the present invention, Y1 may be S.

In another embodiment of the present invention, Y1 may be NRd.

In one embodiment of the present invention, R1 to R5 and Ra to Rd are the same as or different from each other, and are each independently selected from the group consisting of hydrogen; deuterium; halogen; cyano group; substituted or unsubstituted C1 to C30 alkyl group; substituted or unsubstituted C2 to C30 alkenyl group; substituted or unsubstituted C2 to C30 alkynyl group; substituted or unsubstituted C1 to C30 alkoxy group; substituted or unsubstituted C3 to C30 cycloalkyl group; substituted or unsubstituted C2 to C30 heterocycloalkyl group; substituted or unsubstituted C6 to C30 aryl group; substituted or unsubstituted C2 to C30 heteroaryl group; -P(=O)R101R102; -SiR101R102R103; and -NR101R102, or two or more adjacent groups may be bonded to each other to form a substituted or unsubstituted C6 to C30 aromatic hydrocarbon ring; or a substituted or unsubstituted C2 to C30 heterocycle, wherein R101, R102, and R103 are the same as or different from each other, and are each independently a substituted or unsubstituted C1 to C30 alkyl group; a substituted or unsubstituted C6 to C30 aryl group; or a substituted or unsubstituted C2 to C30 heteroaryl group.

In another embodiment of the present invention, R1 to R5 and Ra to Rd are the same as or different from each other, and each independently selected from the group consisting of hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R101R102; - SiR101R102R103; or -NR101R102, or two or more adjacent groups may be bonded to each other to form a substituted or unsubstituted C6 to C20 aromatic hydrocarbon ring or a substituted or unsubstituted C2 to C20 heterocyclic ring, wherein R101, R102, and R103 are the same as or different from each other, and each independently a substituted or unsubstituted C1 to C20 alkyl group; a substituted or unsubstituted C6 to C20 aryl group; or a substituted or unsubstituted C2 to C20 heteroaryl group.

In another embodiment of the present invention, R1 to R5 and Ra to Rd are the same as or different from each other, and each independently hydrogen; deuterium; halogen; a cyano group; a substituted or unsubstituted C1 to C20 alkyl group; a substituted or unsubstituted C6 to C20 aryl group; or a substituted or unsubstituted C2 to C20 heteroaryl group, or two or more adjacent groups may be bonded to each other to form a substituted or unsubstituted C6 to C20 aromatic hydrocarbon ring; or a substituted or unsubstituted C2 to C20 heterocyclic ring.

In another embodiment of the present invention, R1 to R5 are the same as or different from each other, and each independently hydrogen; deuterium; halogen; a cyano group; a substituted or unsubstituted C1 to C20 alkyl group; a substituted or unsubstituted C6 to C20 aryl group; or a substituted or unsubstituted C2 to C20 heteroaryl group, or two or more adjacent groups may be bonded to each other to form a substituted or unsubstituted C6 to C20 aromatic hydrocarbon ring; or a substituted or unsubstituted C2 to C20 heterocyclic ring.

In another embodiment of the present invention, R1 to R5 are the same as or different from each other, and each independently hydrogen; deuterium; a substituted or unsubstituted C6 to C20 aryl group; or a substituted or unsubstituted C2 to C20 heteroaryl group, or two or more adjacent groups may be bonded to each other to form a substituted or unsubstituted C6 to C20 aromatic hydrocarbon ring; or a substituted or unsubstituted C2 to C20 heterocyclic ring.

In another embodiment of the present invention, R1 to R2 are the same as or different from each other, and each independently hydrogen; deuterium; a substituted or unsubstituted C6 to C20 aryl group; or a substituted or unsubstituted C2 to C20 heteroaryl group, or two or more adjacent groups may be bonded to each other to form a substituted or unsubstituted C6 to C20 aromatic hydrocarbon ring; or a substituted or unsubstituted C2 to C20 heterocyclic ring.

In another embodiment of the present invention, R1 and R2 may be the same as or different from each other and may each independently be hydrogen; deuterium; a substituted or unsubstituted phenyl group; a substituted or unsubstituted terphenyl group; a substituted or unsubstituted triphenylenyl group; or a substituted or unsubstituted dibenzothiophenyl group; or two or more adjacent groups may be bonded to each other to form a substituted or unsubstituted C6 to C10 aromatic hydrocarbon ring; or a substituted or unsubstituted C2 to C10 heterocyclic ring.

In another embodiment of the present invention, R3 may be hydrogen; deuterium; a substituted or unsubstituted C6 to C20 aryl group; or a substituted or unsubstituted C2 to C20 heteroaryl group.

In another embodiment of the present invention, R3 may be hydrogen; deuterium; or a substituted or unsubstituted C6 to C20 aryl group.

In another embodiment of the present invention, R3 may be hydrogen; deuterium; or a substituted or unsubstituted phenyl group.

In another embodiment of the present invention, R4 may be hydrogen; deuterium; a substituted or unsubstituted C6 to C20 aryl group; or a substituted or unsubstituted C2 to C20 heteroaryl group.

In another embodiment of the present invention, R4 may be hydrogen; deuterium; a substituted or unsubstituted phenyl group; a substituted or unsubstituted carbazolyl group; or a substituted or unsubstituted dibenzofuranyl group.

In another embodiment of the present invention, R5 may be hydrogen; deuterium; a substituted or unsubstituted C6 to C20 aryl group; or a substituted or unsubstituted C2 to C20 heteroaryl group; or two or more adjacent groups may be bonded to each other to form a substituted or unsubstituted C6 to C20 aromatic hydrocarbon ring; or a substituted or unsubstituted C2 to C20 heterocyclic ring.

In another embodiment of the present invention, R5 may be hydrogen; deuterium; a substituted or unsubstituted phenyl group; a substituted or unsubstituted biphenyl group; a substituted or unsubstituted carbazolyl group; or a substituted or unsubstituted dibenzofuranyl group; or two or more adjacent groups may be bonded to each other to form a substituted or unsubstituted C6 to C10 aromatic hydrocarbon ring; or a substituted or unsubstituted C2 to C10 heterocyclic ring.

In another embodiment of the present invention, Ra to Rc may be the same as or different from each other and may each independently be hydrogen; deuterium; a substituted or unsubstituted C6 to C20 alkyl group; a substituted or unsubstituted C6 to C20 aryl group; or a substituted or unsubstituted C2 to C20 heteroaryl group.

In another embodiment of the present invention, Ra to Rc may be the same as or different from each other and may each independently be hydrogen; deuterium; a substituted or unsubstituted C6 to C20 aryl group; or a substituted or unsubstituted C2 to C20 heteroaryl group.

In another embodiment of the present invention, Ra to Rc may be the same as or different from each other and may each independently be hydrogen; or deuterium.

In another embodiment of the present invention, Rd may be a substituted or unsubstituted C6 to C20 aryl group; or a substituted or unsubstituted C2 to C20 heteroaryl group.

In another embodiment of the present invention, Rd may be a substituted or unsubstituted C6 to C20 aryl group.

In another embodiment of the present invention, Rd may be a substituted or unsubstituted phenyl group; or a substituted or unsubstituted biphenyl group.

In one embodiment of the present invention, Ar1 may be a substituted or unsubstituted C6 to C40 aryl group.

In another embodiment of the present invention, Ar1 may be a substituted or unsubstituted C6 to C30 aryl group.

In another embodiment of the present invention, Ar1 may be a substituted or unsubstituted C6 to C20 aryl group.

In another embodiment of the present invention, Ar1 may be a substituted or unsubstituted phenyl group; a substituted or unsubstituted biphenyl group; a substituted or unsubstituted terphenyl group; a substituted or unsubstituted naphthyl group; a substituted or unsubstituted triphenylenyl group; a substituted or unsubstituted phenanthrenyl group; a substituted or unsubstituted fluorenyl group; a substituted or unsubstituted benzofluorenyl group; a substituted or unsubstituted spirobifluorenyl group; a substituted or unsubstituted spirofluorenebenzofluorenyl group; or a substituted or unsubstituted spirofluorenedibenzofluorenyl group.

In one embodiment of the present invention, L1 and L2 may be the same as or different from each other and may each independently be a direct bond; a substituted or unsubstituted C6 to C30 arylene group; or a C2 to C30 heteroarylene group.

In another embodiment of the present invention, L1 and L2 may be the same as or different from each other and may each independently be a direct bond; a substituted or unsubstituted C6 to C20 arylene group; or a C2 to C20 heteroarylene group.

In another embodiment of the present invention, L1 and L2 may be the same as or different from each other and may each independently be a direct bond; a substituted or unsubstituted C6 to C20 arylene group.

In another embodiment of the present invention, L1 may be a direct bond; a substituted or unsubstituted phenylene group; or a substituted or unsubstituted biphenylene group.

In another embodiment of the present invention, L2 may be a direct bond; a substituted or unsubstituted phenylene group; a substituted or unsubstituted biphenylene group; a substituted or unsubstituted naphthylene group; a substituted or unsubstituted triphenylene group; or a substituted or unsubstituted phenanthrylene group.

In one embodiment of the present invention, L3 may be a substituted or unsubstituted C6 to C30 arylene group; or a C2 to C30 heteroarylene group.

In another embodiment of the present invention, L3 may be a substituted or unsubstituted C6 to C20 arylene group; or a C2 to C20 heteroarylene group.

In another embodiment of the present invention, L3 may be a substituted or unsubstituted C6 to C20 arylene group.

In another embodiment of the present invention, L3 may be a substituted or unsubstituted phenylene group; a substituted or unsubstituted biphenylene group; or a substituted or unsubstituted terphenylene group.

In one embodiment of the present invention, the above 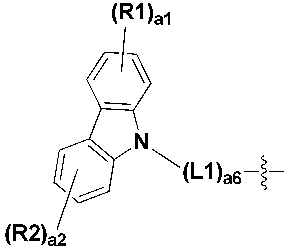 may be represented by any one of Formulas A-1 to A-3 below.

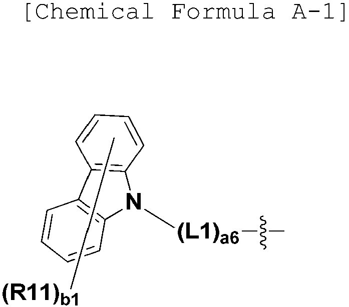

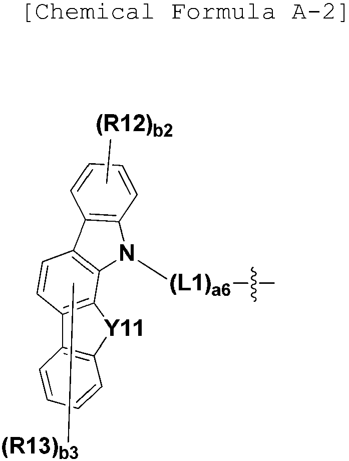

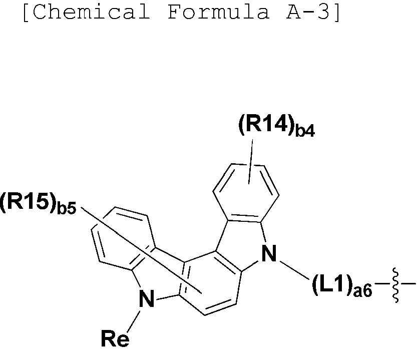

In Formulas A-1 to A-3,
Y11 is O; or S,
R11 to R15 and Re may be the same as or different from each other and are each independently selected from the group consisting of hydrogen; deuterium; halogen; cyano; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, wherein R101, R102, and R103 may be the same as or different from each other and are each independently a substituted or unsubstituted C1 to C60 alkyl group; a substituted or unsubstituted C6 to C60 aryl group; or a substituted or unsubstituted C2 to C60 heteroaryl group,
b1 is an integer of 0 to 8, and when b1 is 2 or greater, R11 may be the same as or different from each other,
b2 is an integer of 0 to 4, and when b2 is 2 or greater, R12 may be the same as or different from each other,
b3 is an integer of 0 to 6, and when b3 is 2 or greater, R13 may be the same as or different from each other,
b4 is an integer of 0 to 4, and when b4 is 2 or greater, R14 may be the same as or different from each other,
b5 is an integer of 0 to 6, and when b5 is 2 or greater, R15 may be the same as or different from each other, and
L1 and a6 are as defined in Chemical Formula 1.

In one embodiment of the present invention, Y11 may be O.

In another embodiment of the present invention, Y11 may be S.

In one embodiment of the present invention, R11 to R15 and Re may be the same as or different from each other and are each independently hydrogen; deuterium; halogen; cyano; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; - P(=O)R101R102; -SiR101R102R103; or -NR101R102, wherein R101, R102, and R103 may be the same as or different from each other and may each independently be a substituted or unsubstituted C1 to C30 alkyl group; a substituted or unsubstituted C6 to C30 aryl group; or a substituted or unsubstituted C2 to C30 heteroaryl group.

In one embodiment of the present invention, R11 to R15 and Re may be the same as or different from each other and are each independently hydrogen; deuterium; halogen; cyano; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; - P(=O)R101R102; -SiR101R102R103; or -NR101R102, wherein R101, R102, and R103 may be the same as or different from each other and may each independently be a substituted or unsubstituted C1 to C20 alkyl group; a substituted or unsubstituted C6 to C20 aryl group; or a substituted or unsubstituted C2 to C20 heteroaryl group.

In another embodiment of the present invention, R11 to R15 and Re may be the same as or different from each other and may each independently be hydrogen; deuterium; halogen; cyano; a substituted or unsubstituted C1 to C20 alkyl group; a substituted or unsubstituted C6 to C20 aryl group; or a substituted or unsubstituted C2 to C20 heteroaryl group.

In another embodiment of the present invention, R11 to R15 and Re may be the same as or different from each other and may each independently be hydrogen; deuterium; a substituted or unsubstituted C6 to C20 aryl group; or a substituted or unsubstituted C2 to C20 heteroaryl group.

In another embodiment of the present invention, R11 to R15 may be the same as or different from each other and may each independently be hydrogen; or deuterium.

In another embodiment of the present invention, Re may be a substituted or unsubstituted C6 to C20 aryl group; or a substituted or unsubstituted C2 to C20 heteroaryl group.

In another embodiment of the present invention, Re may be a substituted or unsubstituted C6 to C20 aryl group.

In another embodiment of the present invention, Re may be a substituted or unsubstituted phenyl; or a substituted or unsubstituted biphenyl.

In one embodiment of the present invention, the above 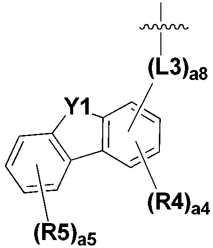 may be represented by any one of Formulas B-1 to B-3 below. 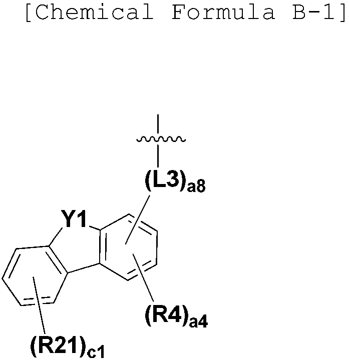 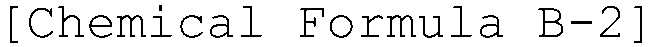 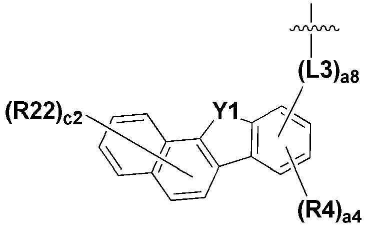 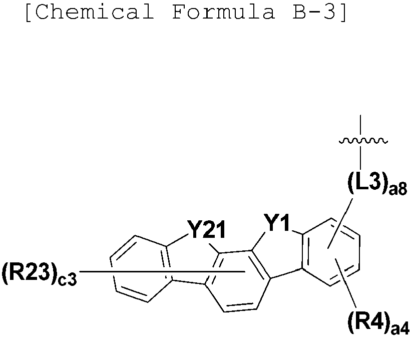 in Formulas B-1 to B-3,
Y21 is O; or NRf,
R21 to R23 and Rf may be the same as or different from each other and are each independently selected from the group consisting of hydrogen; deuterium; halogen; cyano; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, wherein R101, R102, and R103 may be the same as or different from each other and are each independently a substituted or unsubstituted C1 to C60 alkyl group; a substituted or unsubstituted C6 to C60 aryl group; or a substituted or unsubstituted C2 to C60 heteroaryl group,
c1 is an integer of 0 to 4, and when c1 is 2 or greater, R21 may be the same as or different from each other,
c2 is an integer of 0 to 6, and when c2 is 2 or greater, R22 may be the same as or different from each other,
c3 is an integer of 0 to 6, and when c3 is 2 or greater, R23 may be the same as or different from each other, and
Y1, R4, L3, a4, and a8 are as defined in Chemical Formula 1.

In one embodiment of the present invention, Y1 may be S, and Y21 may be NRf.

In another embodiment of the present invention, Y1 may be NRd, and Y21 may be O.

In one embodiment of the present invention, R21 to R23 and Rf may be the same as or different from each other and are each independently hydrogen; deuterium; halogen; cyano; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; - P(=O)R101R102; -SiR101R102R103; or -NR101R102, wherein R101, R102, and R103 may be the same as or different from each other and may each independently be a substituted or unsubstituted C1 to C30 alkyl group; a substituted or unsubstituted C6 to C30 aryl group; or a substituted or unsubstituted C2 to C30 heteroaryl group.

In another embodiment of the present invention, R21 to R23 and Rf may be the same as or different from each other and are each independently hydrogen; deuterium; halogen; cyano; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; - P(=O)R101R102; -SiR101R102R103; or -NR101R102, wherein R101, R102, and R103 may be the same as or different from each other and may each independently be a substituted or unsubstituted C1 to C20 alkyl group; a substituted or unsubstituted C6 to C20 aryl group; or a substituted or unsubstituted C2 to C20 heteroaryl group.

In another embodiment of the present invention, R21 to R23 and Rf may be the same as or different from each other and may each independently be hydrogen; deuterium; halogen; cyano; a substituted or unsubstituted C1 to C20 alkyl group; a substituted or unsubstituted C6 to C20 aryl group; or a substituted or unsubstituted C2 to C20 heteroaryl group.

In another embodiment of the present invention, R21 to R23 and Rf may be the same as or different from each other and may each independently be hydrogen; deuterium; a substituted or unsubstituted C6 to C20 aryl group; or a substituted or unsubstituted C2 to C20 heteroaryl group.

In another embodiment of the present invention, R21 to R23 may be the same as or different from each other and may each independently be hydrogen; or deuterium.

In another embodiment of the present invention, Rf may be a substituted or unsubstituted C6 to C20 aryl group; or a substituted or unsubstituted C2 to C20 heteroaryl group.

In another embodiment of the present invention, Rf may be a substituted or unsubstituted C6 to C20 aryl group.

In another embodiment of the present invention, Rf may be a substituted or unsubstituted phenyl group.

In one embodiment of the present invention, R1 to R5, Ra to Rf, R11 to R15, R21 to R23, Ar1, and L1 to L3 may all comprise non-deuterated hydrogen (H).

In another embodiment of the present invention, at least one of R1 to R5, Ra to Rf, R11 to R15, R21 to R23, Ar1, and L1 to L3 may comprise deuterium (D), and at least one of R1 to R5, Ra to Rf, R11 to R15, R21 to R23, Ar1, and L1 to L3 may comprise non-deuterated hydrogen.

In another embodiment of the present invention, R1 to R5, Ra to Rf, R11 to R15, R21 to R23, Ar1, and L1 to L3 may all comprise deuterium.

In one embodiment of the present invention, the heterocyclic compound represented by Chemical Formula 1 may not include deuterium as a substituent, or the deuterium content relative to the total number of hydrogen atoms and deuterium atoms may be, for example, greater than 0%, 1% or more, 10% or more, 20% or more, 30% or more, 40% or more, or 50% or more, and may be 100% or less, 90% or less, 80% or less, 70% or less, or 60% or less.

In another embodiment of the present invention, the heterocyclic compound represented by Chemical Formula 1 may not include deuterium as a substituent, or the deuterium content relative to the total number of hydrogen atoms and deuterium atoms may be 1% to 100%.

In another embodiment of the present invention, the heterocyclic compound represented by Chemical Formula 1 may not include deuterium as a substituent, or the deuterium content relative to the total number of hydrogen atoms and deuterium atoms may be 20% to 90%.

In another embodiment of the present invention, the heterocyclic compound represented by Chemical Formula 1 may not include deuterium as a substituent, or the deuterium content relative to the total number of hydrogen atoms and deuterium atoms may be 30% to 80%.

In another embodiment of the present invention, the heterocyclic compound represented by Chemical Formula 1 may not include deuterium as a substituent, or the deuterium content relative to the total number of hydrogen atoms and deuterium atoms may be 50% to 70%.

In one embodiment of the present invention, the heterocyclic compound represented by Chemical Formula 1 may be represented by any one of the following compounds.

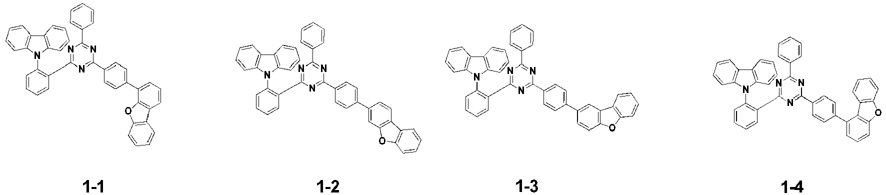

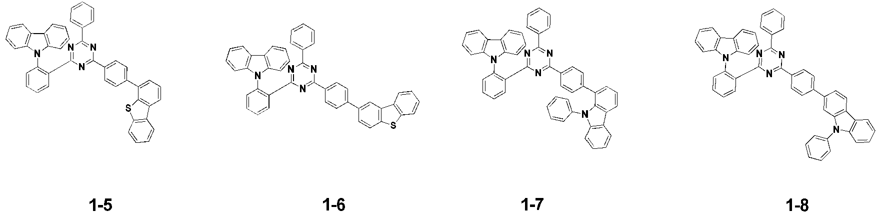

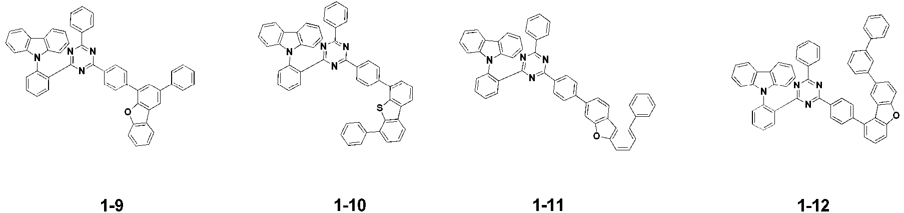

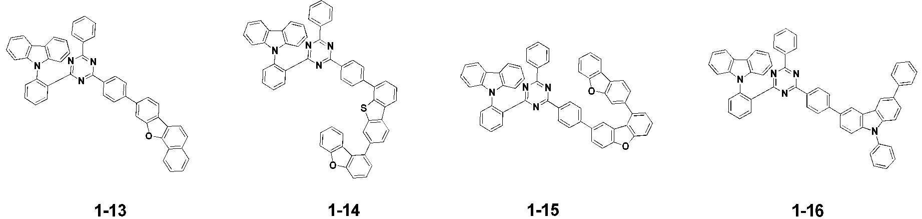

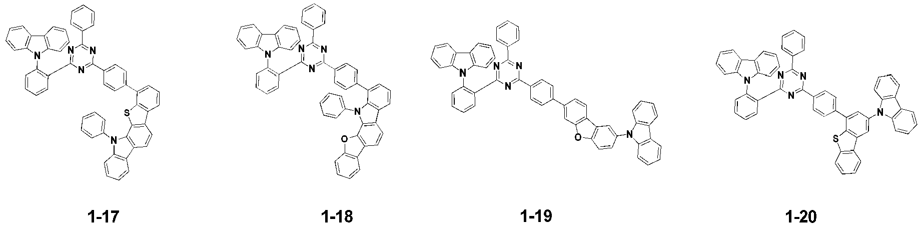

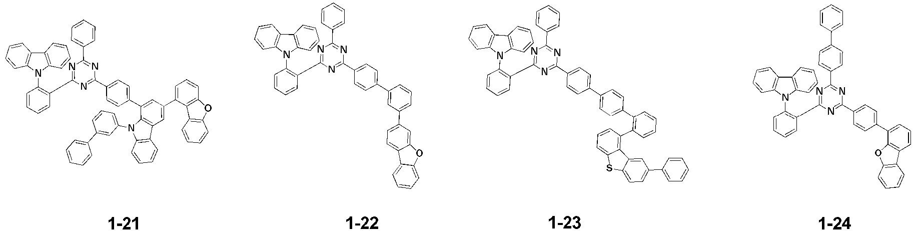

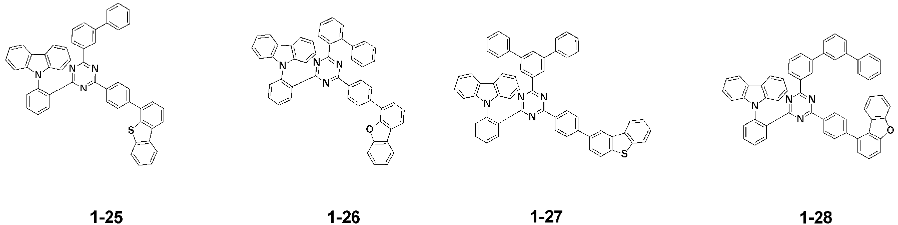

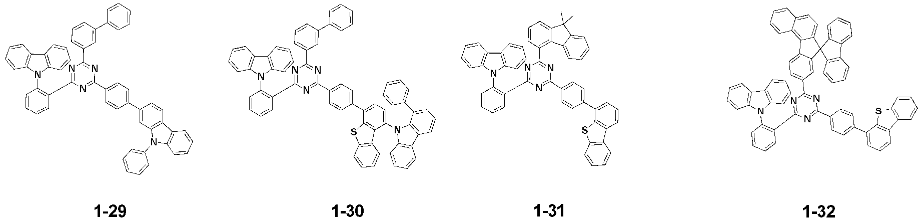

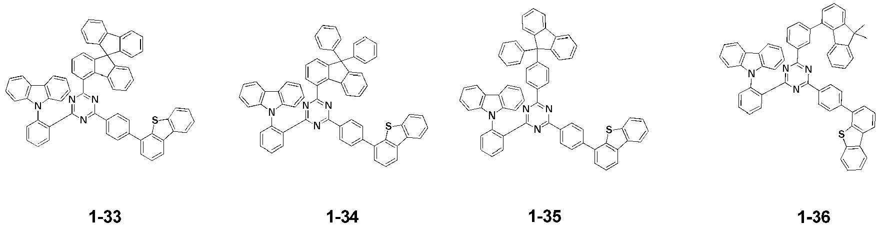

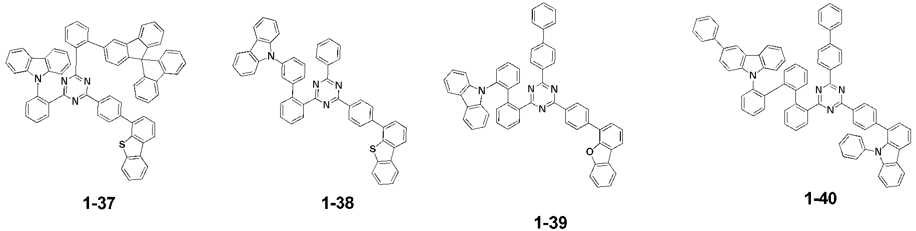

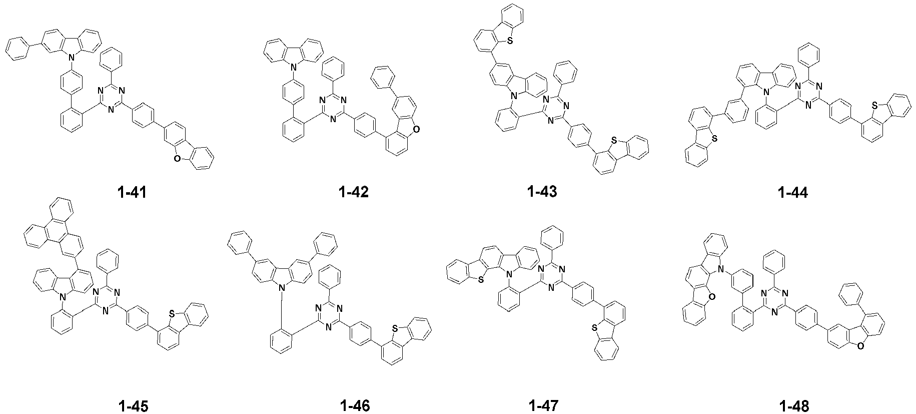

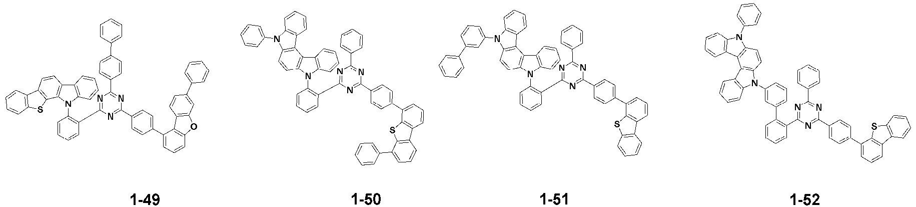

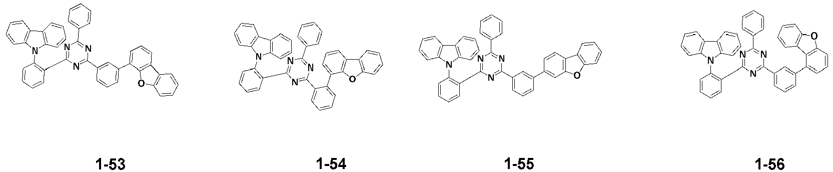

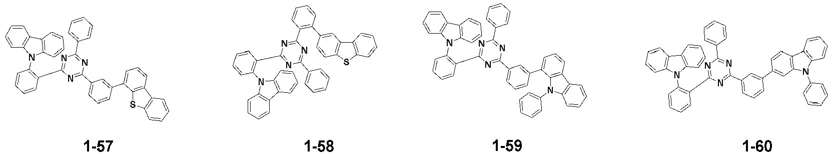

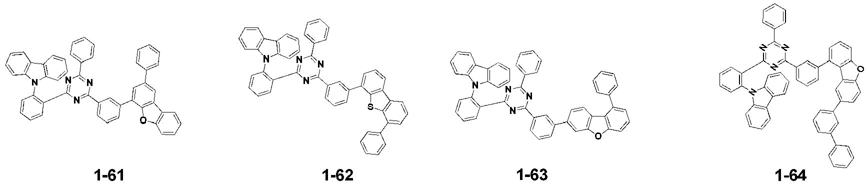

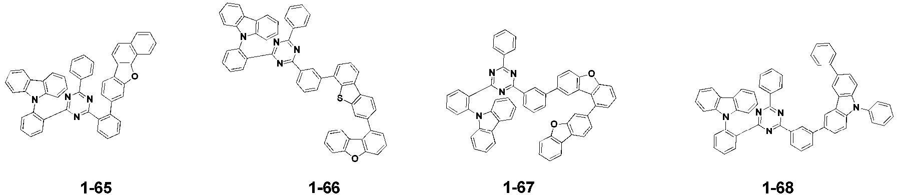

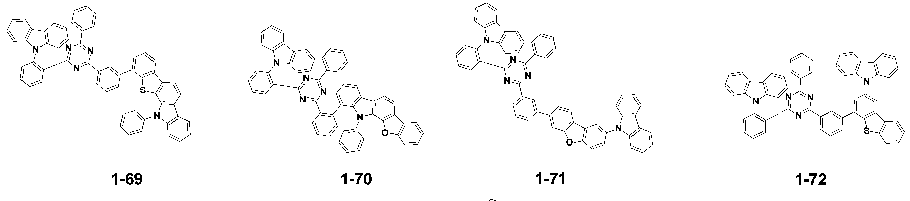

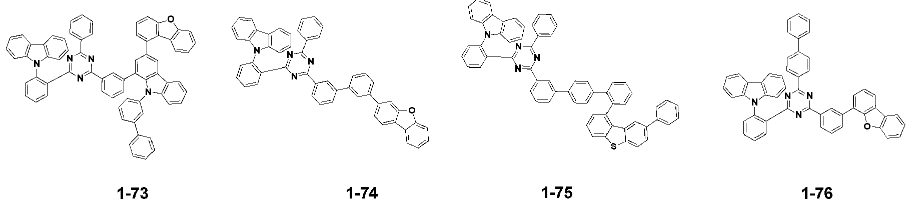

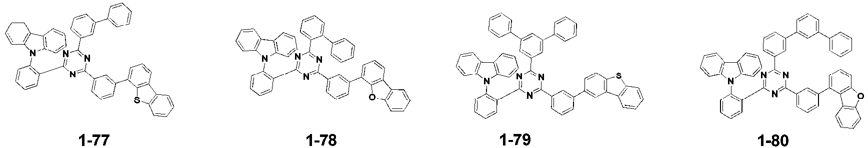

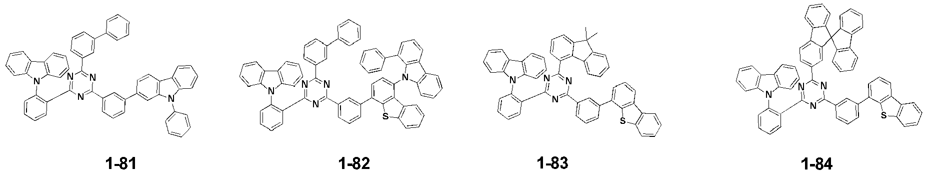

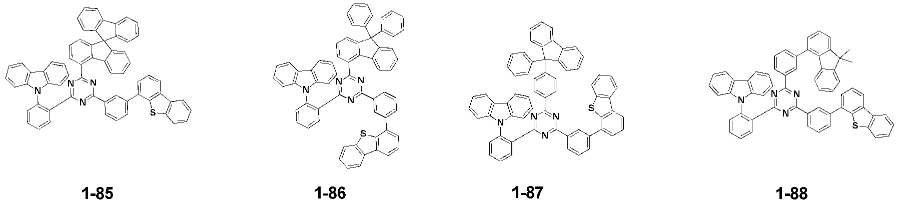

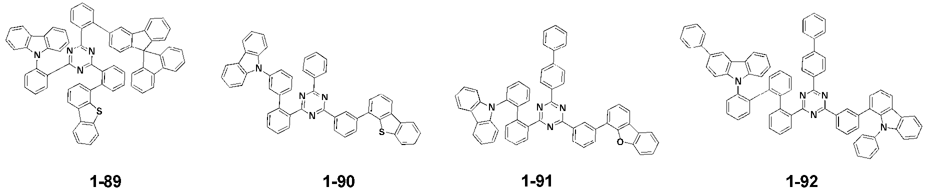

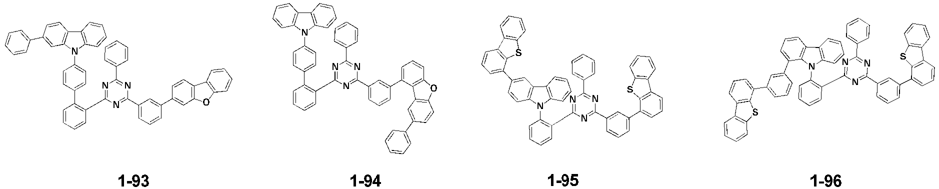

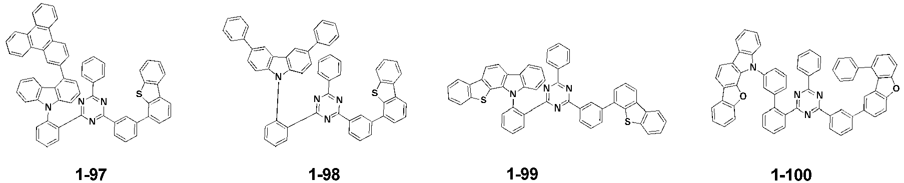

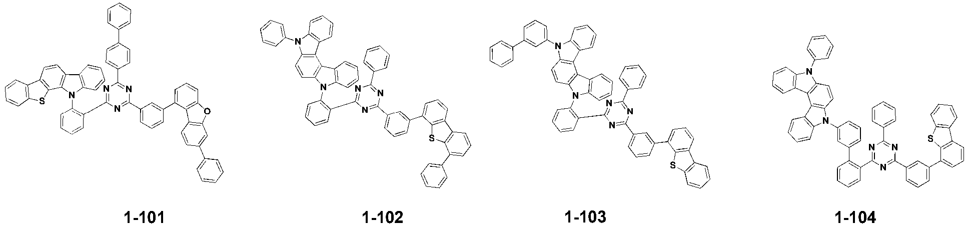

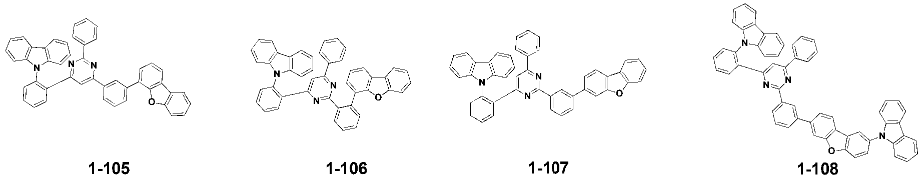

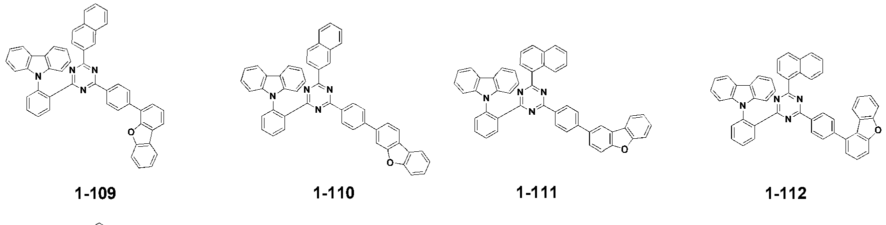

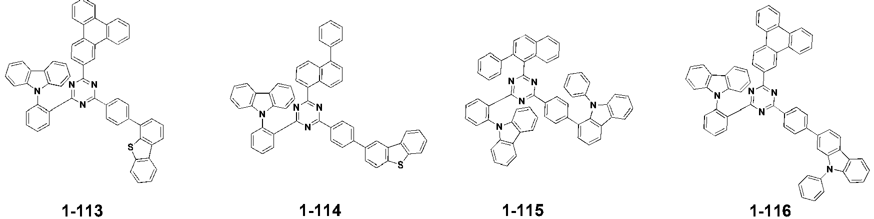

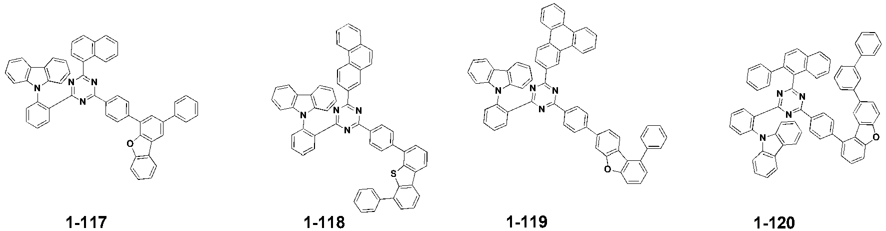

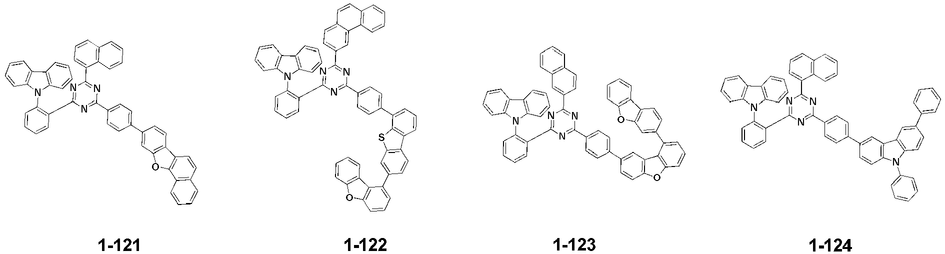

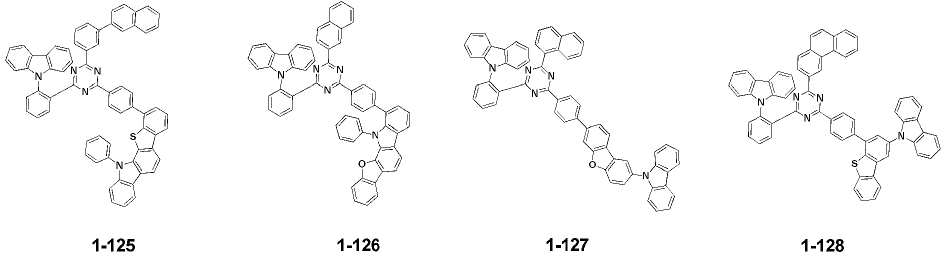

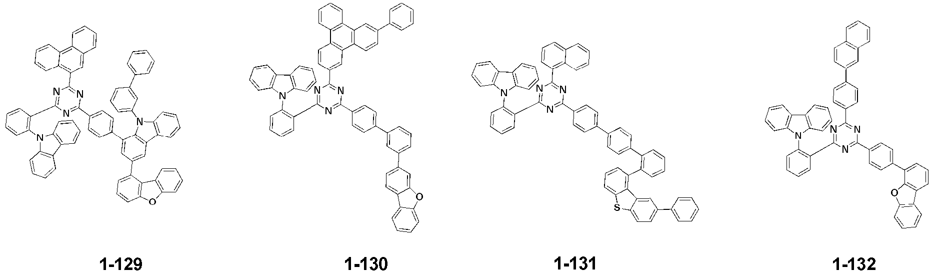

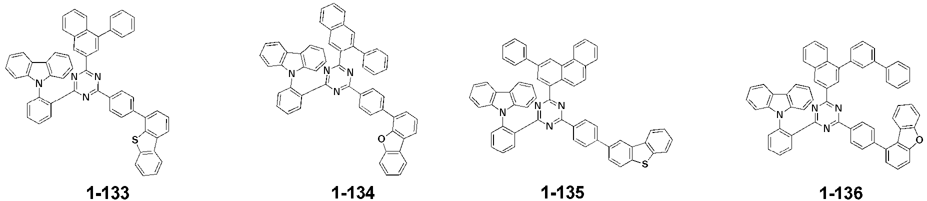

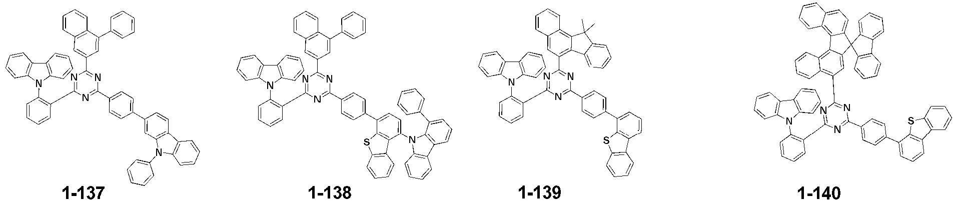

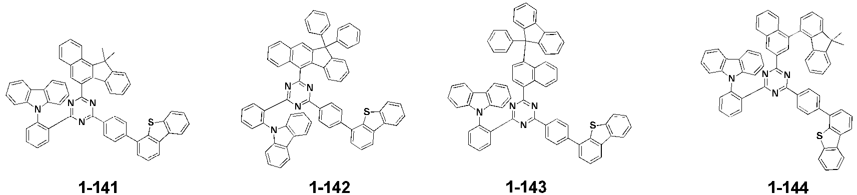

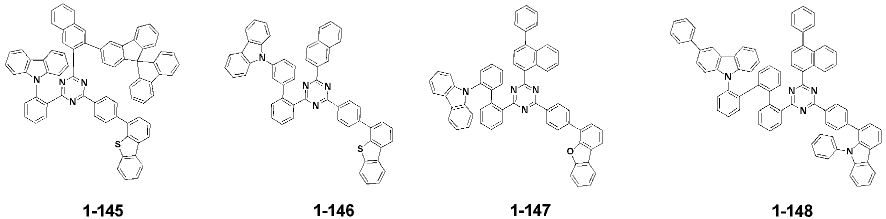

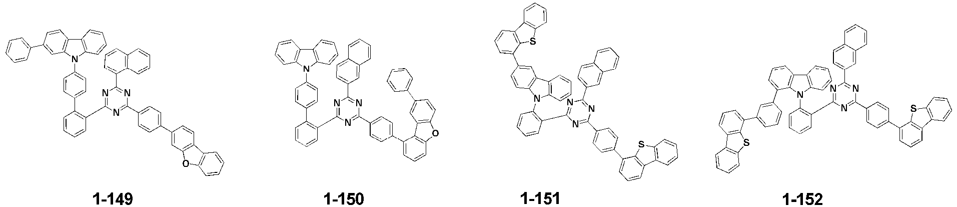

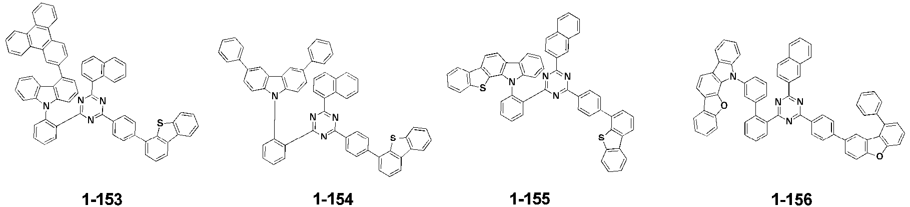

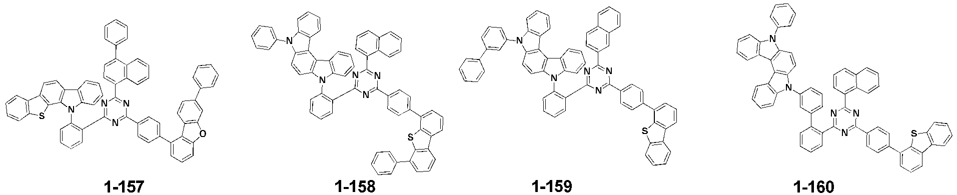

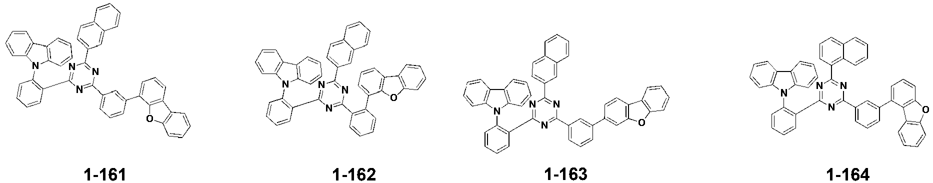

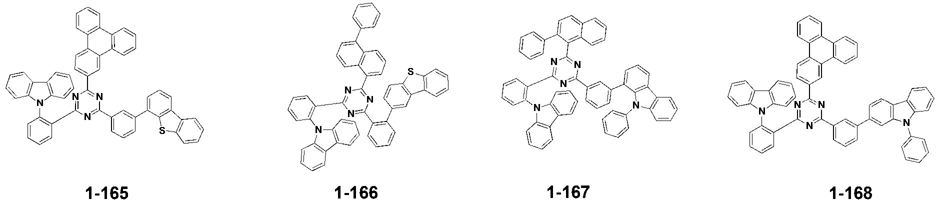

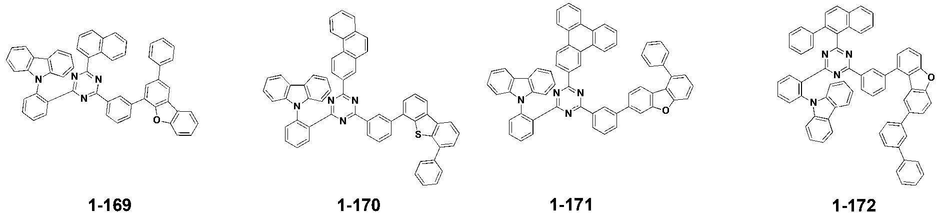

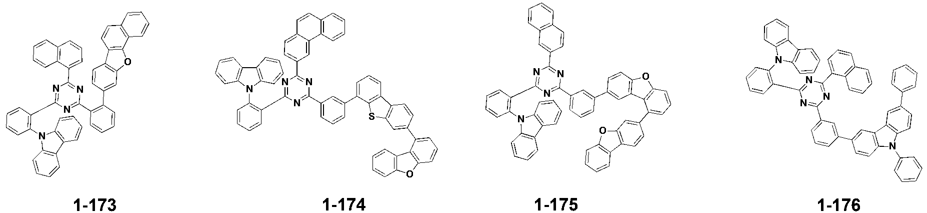

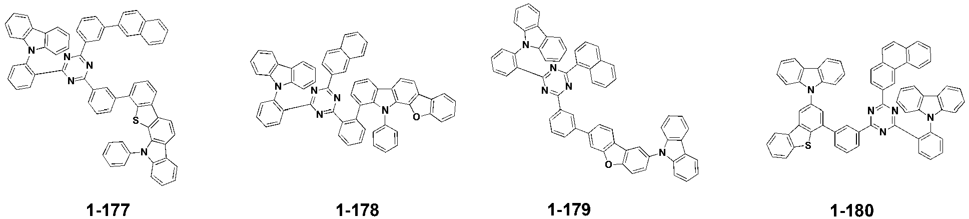

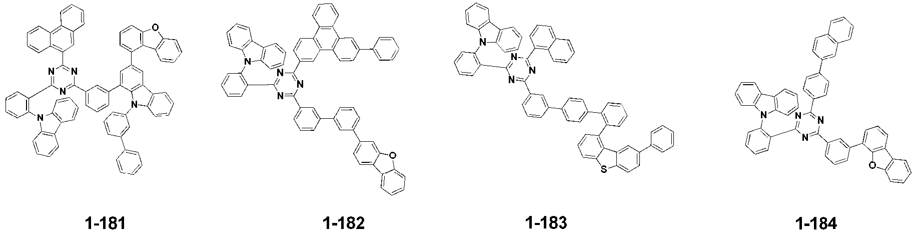

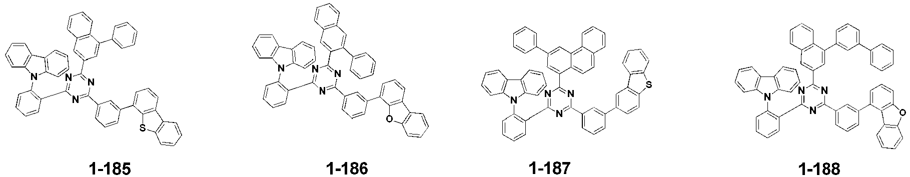

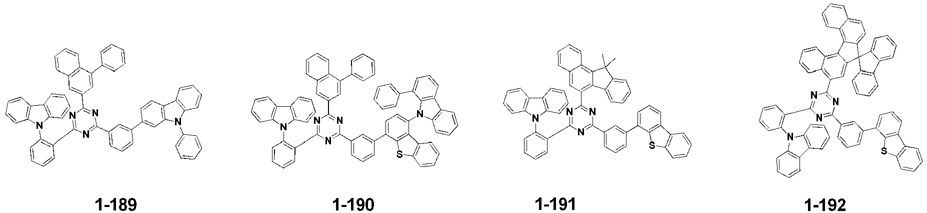

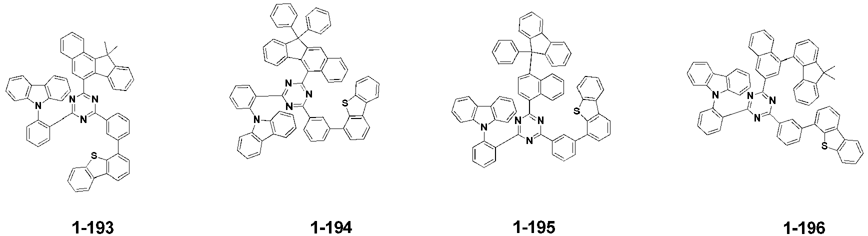

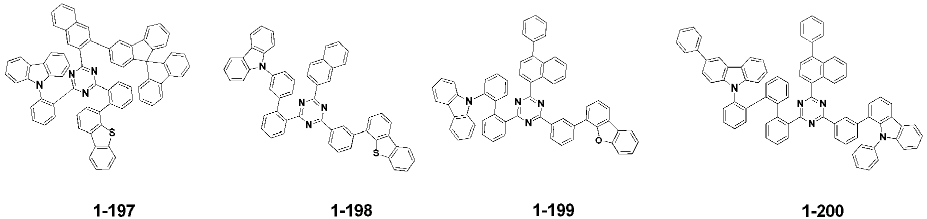

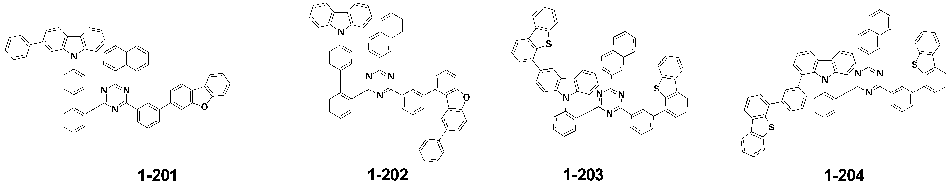

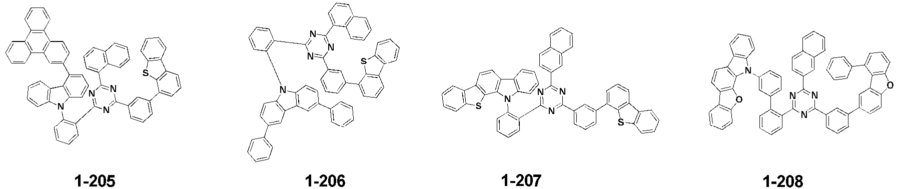

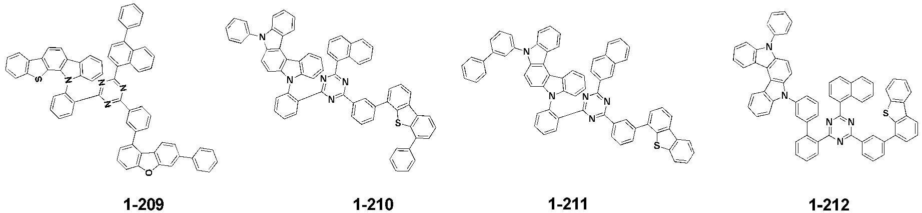

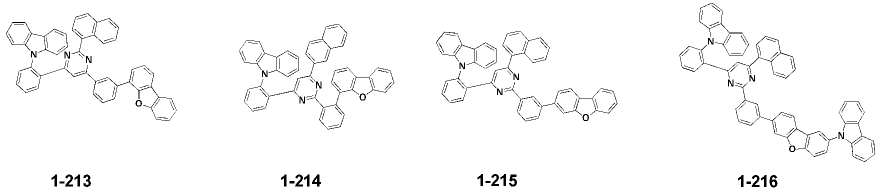

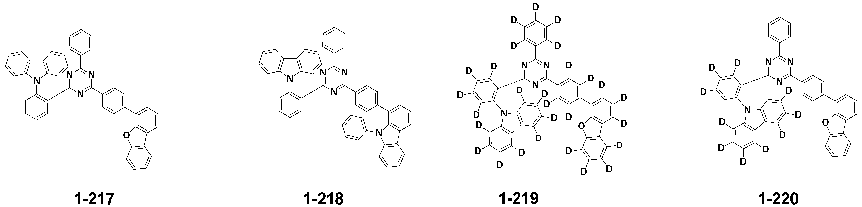

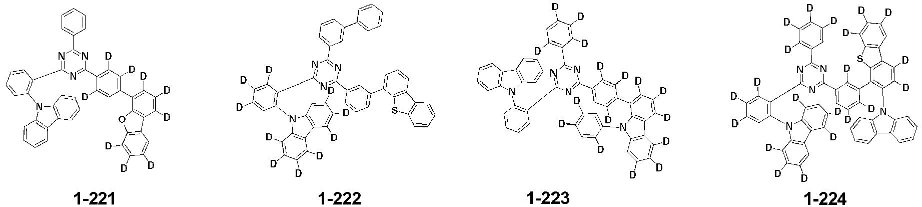

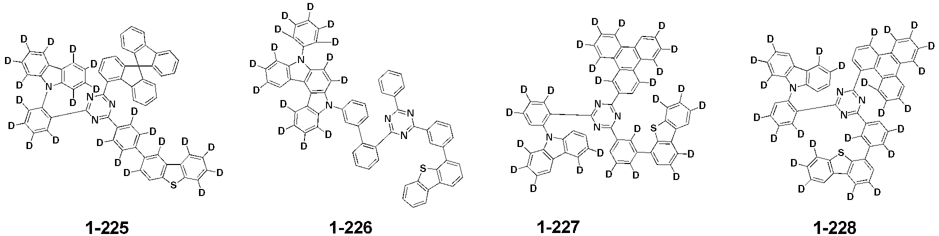

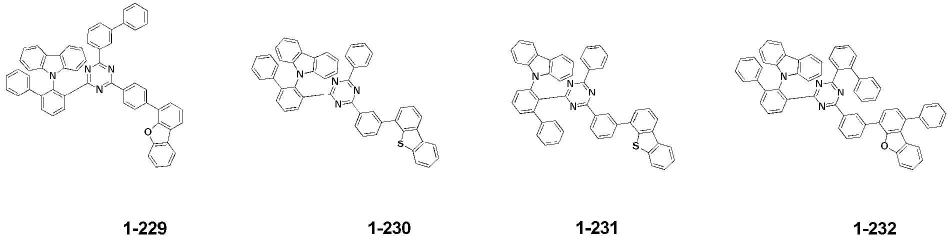

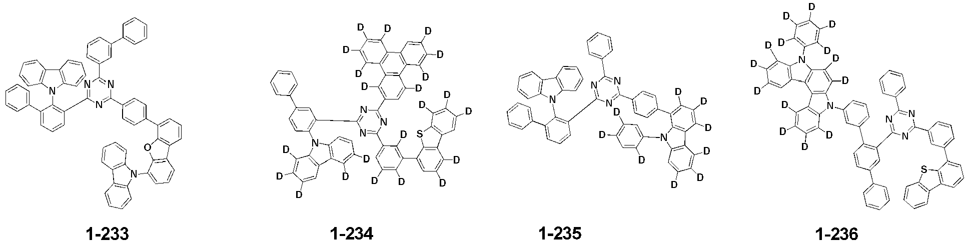

Further, by introducing various substituents into the structure of Chemical Formula 1, compounds having intrinsic properties imparted by the introduced substituents may be synthesized. For example, by introducing into the core structure substituents mainly used in materials for a hole injection layer, a hole transport layer, an emission layer, an electron transport layer, an electron blocking layer, and a charge generation layer used in manufacturing an organic light-emitting device, materials satisfying the requirements demanded in each organic material layer may be synthesized.

Further, by introducing various substituents into the structure of Chemical Formula 1, the energy bandgap may be finely controlled, while at the same time interfacial properties between organic materials may be improved, thereby diversifying the applications of the material.

Meanwhile, the heterocyclic compound has a high glass transition temperature (Tg) and thus exhibits excellent thermal stability. Such an increase in thermal stability is an important factor in providing driving stability to a device.

The heterocyclic compound according to one embodiment of the present invention may be prepared through multi-step chemical reactions. Some intermediate compounds may first be prepared, and the compound of Chemical Formula 1 may be prepared from those intermediate compounds. More specifically, the heterocyclic compound according to one embodiment of the present invention may be prepared based on the Preparation Examples described below.

Another embodiment of the present invention provides an organic light-emitting device comprising the heterocyclic compound represented by Chemical Formula 1. The term "organic light-emitting device" may be referred to as "organic light-emitting diode," "OLED (Organic Light Emitting Diode)," "OLED device," or "organic electroluminescent device."

Further, the present invention provides an organic light-emitting device comprising:
a first electrode;
a second electrode disposed to face the first electrode; and
one or more organic material layers disposed between the first electrode and the second electrode,
wherein at least one of the organic material layers comprises the heterocyclic compound represented by Chemical Formula 1.

In one embodiment of the present invention, the first electrode may be an anode, and the second electrode may be a cathode.

In another embodiment, the first electrode may be a cathode, and the second electrode may be an anode.

In one embodiment of the present invention, the organic light-emitting device may be a red organic light-emitting device, and the heterocyclic compound represented by Chemical Formula 1 may be used as a material for a red organic light-emitting device.

In another embodiment of the present invention, the organic light-emitting device may be a blue organic light-emitting device, and the heterocyclic compound represented by Chemical Formula 1 may be used as a material for a blue organic light-emitting device.

In another embodiment of the present invention, the organic light-emitting device may be a green organic light-emitting device, and the heterocyclic compound represented by Chemical Formula 1 may be used as a material for a green organic light-emitting device.

In one embodiment of the present invention, the organic light-emitting device may be a red organic light-emitting device, and the heterocyclic compound represented by Chemical Formula 1 may be used as a emission layer material of the red organic light-emitting device.

In another embodiment of the present invention, the organic light-emitting device may be a blue organic light-emitting device, and the heterocyclic compound represented by Chemical Formula 1 may be used as a emission layer material of the blue organic light-emitting device.

In another embodiment of the present invention, the organic light-emitting device may be a green organic light-emitting device, and the heterocyclic compound represented by Chemical Formula 1 may be used as a emission layer material of the green organic light-emitting device.

Specific details of the heterocyclic compound represented by Chemical Formula 1 are the same as described above."

The organic light emitting device of the present invention may be manufactured by conventional methods and materials for manufacturing organic light emitting devices, except that one or more organic material layers are formed using the aforementioned heterocyclic compound.

The heterocyclic compound may be formed into an organic material layer through a solution coating method as well as a vacuum deposition method during the manufacture of an organic light emitting device. Here, the solution coating method refers to spin coating, dip coating, inkjet printing, screen printing, spraying, roll coating, and the like, but is not limited thereto.

The organic material layer of the organic light-emitting device according to the present invention may have a single-layer structure, or may have a multilayer structure in which two or more organic material layers are stacked. For example, the organic light-emitting device of the present invention may have a structure including, as organic material layers, a hole injection layer, an electron blocking layer, a hole transport layer, a emission layer, an electron transport layer, a hole blocking layer, and an electron injection layer. However, the structure of the organic light-emitting device is not limited thereto and may include a smaller number of organic material layers.

In the organic light-emitting device of the present invention, the organic material layer may include a emission layer, and the emission layer may include the heterocyclic compound represented by Chemical Formula 1. When the heterocyclic compound is used in the emission layer, strong charge transfer is possible due to spatial separation of the HOMO (Highest Occupied Molecular Orbital) and the LUMO (Lowest Unoccupied Molecular Orbital), thereby improving the driving efficiency and lifetime of the organic light-emitting device.

In an organic light-emitting device according to one embodiment of the present invention, there is provided an organic light-emitting device in which the organic material layer including the heterocyclic compound represented by Chemical Formula 1 further includes a heterocyclic compound represented by Chemical Formula 2 or Chemical Formula 3. 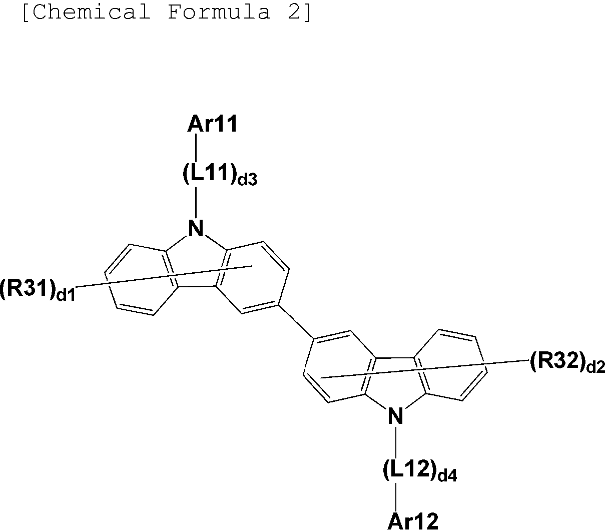 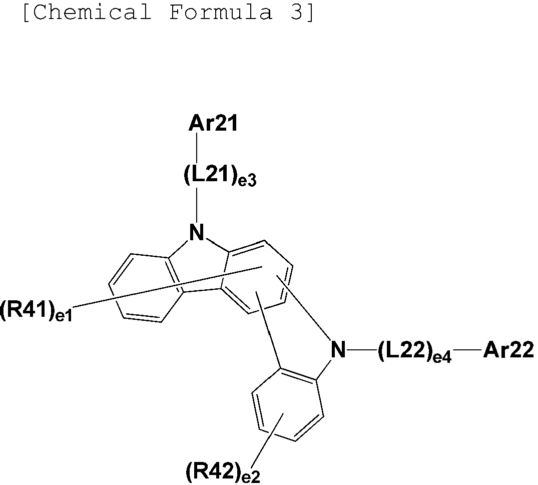 in Chemical Formulas 2 and 3,
R31, R32, R41, and R42 are the same as or different from each other, and each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - P(=O)R201R202; -SiR201R202R203; and -NR201R202; or two or more adjacent groups may be bonded to each other to form a substituted or unsubstituted C6 to C60 aromatic hydrocarbon ring or a substituted or unsubstituted C2 to C60 heterocyclic ring, wherein R201, R202, and R203 are the same as or different from each other, and each independently a substituted or unsubstituted C1 to C60 alkyl group; a substituted or unsubstituted C6 to C60 aryl group; or a substituted or unsubstituted C2 to C60 heteroaryl group,
d1 is an integer of 0 to 7, and when d1 is 2 or more, R31 are the same as or different from each other,
d2 is an integer of 0 to 7, and when d2 is 2 or more, R32 are the same as or different from each other,
e1 is an integer of 0 to 6, and when e1 is 2 or more, R41 are the same as or different from each other,
e2 is an integer of 0 to 4, and when e2 is 2 or more, R42 are the same as or different from each other,
Ar11, Ar12, Ar21, and Ar22 are the same as or different from each other, and each independently a cyano group; - SiR201R202R203; a substituted or unsubstituted C6 to C60 aryl group; or a substituted or unsubstituted C2 to C60 heteroaryl group, wherein R201, R202, and R203 are the same as or different from each other, and each independently a substituted or unsubstituted C1 to C60 alkyl group; a substituted or unsubstituted C6 to C60 aryl group; or a substituted or unsubstituted C2 to C60 heteroaryl group,
L11, L12, L21, and L22 are the same as or different from each other, and each independently a direct bond; a substituted or unsubstituted C6 to C60 arylene group; or a substituted or unsubstituted C2 to C60 heteroarylene group,
d3 is an integer of 0 to 5, and when d3 is 2 or more, L11 are the same as or different from each other,
d4 is an integer of 0 to 5, and when d4 is 2 or more, L12 are the same as or different from each other,
e3 is an integer of 0 to 5, and when e3 is 2 or more, L21 are the same as or different from each other,
e4 is an integer of 0 to 5, and when e4 is 2 or more, L22 are the same as or different from each other.

In one embodiment of the present invention, R31, R32, R41, and R42 are the same as or different from each other, and each independently selected from the group consisting of hydrogen; deuterium; a halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; -P(=O)R201R202; - SiR201R202R203; or -NR201R202; or two or more adjacent groups may be bonded to each other to form a substituted or unsubstituted C6 to C30 aromatic hydrocarbon ring or a substituted or unsubstituted C2 to C30 heterocyclic ring, wherein R201, R202, and R203 are the same as or different from each other, and each independently a substituted or unsubstituted C1 to C30 alkyl group; a substituted or unsubstituted C6 to C30 aryl group; or a substituted or unsubstituted C2 to C30 heteroaryl group.

In another embodiment of the present invention, R31, R32, R41, and R42 are the same as or different from each other, and each independently selected from the group consisting of hydrogen; deuterium; a halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R201R202; - SiR201R202R203; or -NR201R202; or two or more adjacent groups may be bonded to each other to form a substituted or unsubstituted C6 to C20 aromatic hydrocarbon ring or a substituted or unsubstituted C2 to C20 heterocyclic ring, wherein R201, R202, and R203 are the same as or different from each other, and each independently a substituted or unsubstituted C1 to C20 alkyl group; a substituted or unsubstituted C6 to C20 aryl group; or a substituted or unsubstituted C2 to C20 heteroaryl group.

In another embodiment of the present invention, R31, R32, R41, and R42 are the same as or different from each other, and each independently selected from the group consisting of hydrogen; deuterium; a halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; or a substituted or unsubstituted C2 to C20 heteroaryl group.

In another embodiment of the present invention, R31, R32, R41, and R42 are the same as or different from each other, and each independently selected from the group consisting of hydrogen; deuterium; a halogen; a cyano group; a substituted or unsubstituted C1 to C20 alkyl group; a substituted or unsubstituted C6 to C20 aryl group; or a substituted or unsubstituted C2 to C20 heteroaryl group.

In another embodiment of the present invention, R31, R32, R41, and R42 are the same as or different from each other, and each independently hydrogen or deuterium.

In one embodiment of the present invention, Ar11, Ar12, Ar21, and Ar22 are the same as or different from each other, and each independently a cyano group; a substituted or unsubstituted C6 to C30 aryl group; a substituted or unsubstituted C2 to C30 heteroaryl group; or -SiR201R202R203, wherein R201, R202, and R203 are the same as or different from each other, and each independently a substituted or unsubstituted C1 to C30 alkyl group; a substituted or unsubstituted C6 to C30 aryl group; or a substituted or unsubstituted C2 to C30 heteroaryl group.

In another embodiment of the present invention, Ar11, Ar12, Ar21, and Ar22 are the same as or different from each other, and each independently a cyano group; a substituted or unsubstituted C6 to C20 aryl group; a substituted or unsubstituted C2 to C20 heteroaryl group; or -SiR201R202R203, wherein R201, R202, and R203 are the same as or different from each other, and each independently a substituted or unsubstituted C1 to C20 alkyl group; a substituted or unsubstituted C6 to C20 aryl group; or a substituted or unsubstituted C2 to C20 heteroaryl group.

In another embodiment of the present invention, Ar11 and Ar12 are the same as or different from each other, and each independently a cyano group; a substituted or unsubstituted phenyl group; a substituted or unsubstituted biphenyl group; a substituted or unsubstituted terphenyl group; a substituted or unsubstituted naphthyl group; a substituted or unsubstituted triphenylenyl group; a substituted or unsubstituted fluorenyl group; a substituted or unsubstituted spirobifluorenyl group; a substituted or unsubstituted dibenzofuranyl group; a substituted or unsubstituted dibenzothiophenyl group; or -SiR201R202R203, wherein R201, R202, and R203 may be a substituted or unsubstituted phenyl group.

In another embodiment of the present invention, Ar21 and Ar22 are the same as or different from each other, and each independently a substituted or unsubstituted phenyl group; a substituted or unsubstituted biphenyl group; a substituted or unsubstituted terphenyl group; a substituted or unsubstituted triphenylenyl group; a substituted or unsubstituted dibenzofuranyl group; or a substituted or unsubstituted dibenzothiophenyl group.

In one embodiment of the present invention, L11, L12, L21, and L22 are the same as or different from each other, and each independently a direct bond; a substituted or unsubstituted C6 to C30 arylene group; or a substituted or unsubstituted C2 to C30 heteroarylene group.

In another embodiment of the present invention, L11, L12, L21, and L22 are the same as or different from each other, and each independently a direct bond; a substituted or unsubstituted C6 to C20 arylene group; or a substituted or unsubstituted C2 to C20 heteroarylene group.

In another embodiment of the present invention, L11 and L12 are the same as or different from each other, and each independently a direct bond; or a substituted or unsubstituted C6 to C20 arylene group.

In another embodiment of the present invention, L11 and L12 are the same as or different from each other, and each independently a direct bond; a substituted or unsubstituted phenylene group; or a substituted or unsubstituted biphenylene group.

In another embodiment of the present invention, L21 and L22 are the same as or different from each other, and each independently a direct bond; a substituted or unsubstituted C6 to C20 arylene group; or a substituted or unsubstituted C2 to C20 heteroarylene group.

In another embodiment of the present invention, L21 and L22 are the same as or different from each other, and each independently a direct bond; a substituted or unsubstituted phenylene group; a substituted or unsubstituted biphenylene group; or a substituted or unsubstituted dibenzofuranylene group.

In one embodiment of the present invention, R31, R32, R41, R42, Ar11, Ar12, Ar21, Ar22, L11, L12, L21, and L22 may all include non-deuterated hydrogen (H).

In another embodiment of the present invention, at least one of R31, R32, R41, R42, Ar11, Ar12, Ar21, Ar22, L11, L12, L21, and L22 includes deuterium (D), and at least one thereof includes non-deuterated hydrogen.

In another embodiment of the present invention, R31, R32, R41, R42, Ar11, Ar12, Ar21, Ar22, L11, L12, L21, and L22 may all include deuterium.

In one embodiment of the present invention, the heterocyclic compound represented by Chemical Formula 2 or Chemical Formula 3 may not include deuterium as a substituent, or the deuterium content relative to the total number of hydrogen atoms and deuterium atoms may be greater than 0%, 1% or more, 10% or more, 20% or more, 30% or more, 40% or more, or 50% or more, and may be 100% or less, 90% or less, 80% or less, 70% or less, or 60% or less.

In another embodiment of the present invention, the heterocyclic compound represented by Formula 2 or the heterocyclic compound represented by Chemical Formula 3 may not include deuterium as a substituent, or the content of deuterium relative to the total number of hydrogen atoms and deuterium atoms may be 1% to 100%.

In another embodiment of the present invention, the heterocyclic compound represented by Formula 2 or the heterocyclic compound represented by Chemical Formula 3 may not include deuterium as a substituent, or the content of deuterium relative to the total number of hydrogen atoms and deuterium atoms may be 20% to 90%.

In another embodiment of the present invention, the heterocyclic compound represented by Chemical Formula 2 or the heterocyclic compound represented by Chemical Formula 3 may not include deuterium as a substituent, or the content of deuterium relative to the total number of hydrogen atoms and deuterium atoms may be 30% to 80%.

In another embodiment of the present invention, the heterocyclic compound represented by Chemical Formula 2 or the heterocyclic compound represented by Chemical Formula 3 may not include deuterium as a substituent, or the content of deuterium relative to the total number of hydrogen atoms and deuterium atoms may be 50% to 70%.

In one embodiment of the present invention, the heterocyclic compound represented by Chemical Formula 2 may not include deuterium as a substituent, or the content of deuterium relative to the total number of hydrogen atoms and deuterium atoms may be, for example, greater than 0%, 1% or more, 10% or more, 20% or more, 30% or more, 40% or more, or 50% or more, and 100% or less, 90% or less, 80% or less, 70% or less, or 60% or less.

In another embodiment of the present invention, the heterocyclic compound represented by Chemical Formula 2 may not include deuterium as a substituent, or the content of deuterium relative to the total number of hydrogen atoms and deuterium atoms may be 1% to 100%.

In another embodiment of the present invention, the heterocyclic compound represented by Chemical Formula 2 may not include deuterium as a substituent, or the content of deuterium relative to the total number of hydrogen atoms and deuterium atoms may be 20% to 90%.

In another embodiment of the present invention, the heterocyclic compound represented by Chemical Formula 2 may not include deuterium as a substituent, or the content of deuterium relative to the total number of hydrogen atoms and deuterium atoms may be 30% to 80%.

In another embodiment of the present invention, the heterocyclic compound represented by Chemical Formula 2 may not include deuterium as a substituent, or the content of deuterium relative to the total number of hydrogen atoms and deuterium atoms may be 50% to 70%.

In one embodiment of the present invention, the heterocyclic compound represented by Chemical Formula 3 may not include deuterium as a substituent, or the content of deuterium relative to the total number of hydrogen atoms and deuterium atoms may be, for example, greater than 0%, 1% or more, 10% or more, 20% or more, 30% or more, 40% or more, or 50% or more, and 100% or less, 90% or less, 80% or less, 70% or less, or 60% or less.

In another embodiment of the present invention, the heterocyclic compound represented by Chemical Formula 3 may not include deuterium as a substituent, or the content of deuterium relative to the total number of hydrogen atoms and deuterium atoms may be 1% to 100%.

In another embodiment of the present invention, the heterocyclic compound represented by Chemical Formula 3 may not include deuterium as a substituent, or the content of deuterium relative to the total number of hydrogen atoms and deuterium atoms may be 20% to 90%.

In another embodiment of the present invention, the heterocyclic compound represented by Chemical Formula 3 may not include deuterium as a substituent, or the content of deuterium relative to the total number of hydrogen atoms and deuterium atoms may be 30% to 80%.

In another embodiment of the present invention, the heterocyclic compound represented by Chemical Formula 3 may not include deuterium as a substituent, or the content of deuterium relative to the total number of hydrogen atoms and deuterium atoms may be 50% to 70%.

When the heterocyclic compound represented by Chemical Formula 1 and the heterocyclic compound represented by Chemical Formula 2, or the heterocyclic compound represented by Chemical Formula 1 and the heterocyclic compound represented by Chemical Formula 3 are simultaneously included, more excellent efficiency and lifetime characteristics are exhibited. From this, it can be expected that an exciplex phenomenon occurs when the two compounds are simultaneously included.

The exciplex phenomenon refers to a process in which energy corresponding to the HOMO energy level of a donor (donor, p-host) and the LUMO energy level of an acceptor (acceptor, n-host) is emitted through electron exchange between two molecules. When the exciplex phenomenon occurs between two molecules, reverse intersystem crossing (RISC) takes place, thereby allowing the internal quantum efficiency of fluorescence to increase up to 100%. When a donor (p-host) having excellent hole-transporting ability and an acceptor (n-host) having excellent electron-transporting ability are used as host materials in the emission layer, holes are injected into the p-host and electrons are injected into the n-host. Accordingly, the driving voltage can be reduced, which contributes to improved device lifetime. That is, when the heterocyclic compound represented by Chemical Formula 1 is used as the acceptor and the heterocyclic compound represented by Chemical Formula 2 or the heterocyclic compound represented by Chemical Formula 3 is used as the donor, excellent device characteristics can be achieved.

In one embodiment of the present invention, when the heterocyclic compound represented by Chemical Formula 1 and the heterocyclic compound represented by Chemical Formula 2 are simultaneously included, at least one of the compounds may not include deuterium as a substituent, or the content of deuterium relative to the total number of hydrogen atoms and deuterium atoms may be greater than 0%, 1% or more, 10% or more, 20% or more, 30% or more, 40% or more, or 50% or more, and 100% or less, 90% or less, 80% or less, 70% or less, or 60% or less.

In another embodiment of the present invention, at least one of the compounds may not include deuterium as a substituent, or the content of deuterium relative to the total number of hydrogen atoms and deuterium atoms may be 1% to 100%.

In another embodiment of the present invention, at least one of the compounds may not include deuterium as a substituent, or the content of deuterium relative to the total number of hydrogen atoms and deuterium atoms may be 20% to 90%.

In another embodiment of the present invention, at least one of the compounds may not include deuterium as a substituent, or the content of deuterium relative to the total number of hydrogen atoms and deuterium atoms may be 30% to 80%.

In another embodiment of the present invention, at least one of the compounds may not include deuterium as a substituent, or the content of deuterium relative to the total number of hydrogen atoms and deuterium atoms may be 50% to 70%.

In one embodiment of the present invention, when the heterocyclic compound represented by Chemical Formula 1 and the heterocyclic compound represented by Chemical Formula 3 are simultaneously included, at least one of the compounds may not include deuterium as a substituent, or the content of deuterium relative to the total number of hydrogen atoms and deuterium atoms may be greater than 0%, 1% or more, 10% or more, 20% or more, 30% or more, 40% or more, or 50% or more, and 100% or less, 90% or less, 80% or less, 70% or less, or 60% or less.

In another embodiment of the present invention, at least one of the compounds may not include deuterium as a substituent, or the content of deuterium relative to the total number of hydrogen atoms and deuterium atoms may be 1% to 100%.

In another embodiment of the present invention, at least one of the compounds may not include deuterium as a substituent, or the content of deuterium relative to the total number of hydrogen atoms and deuterium atoms may be 20% to 90%.

In another embodiment of the present invention, at least one of the compounds may not include deuterium as a substituent, or the content of deuterium relative to the total number of hydrogen atoms and deuterium atoms may be 30% to 80%.

In another embodiment of the present invention, at least one of the compounds may not include deuterium as a substituent, or the content of deuterium relative to the total number of hydrogen atoms and deuterium atoms may be 50% to 70%.

In one embodiment of the present invention, the heterocyclic compound represented by Chemical Formula 2 may be represented by any one of the following compounds.

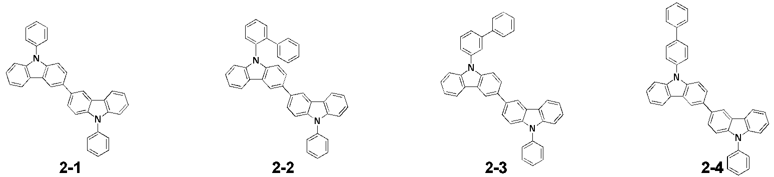

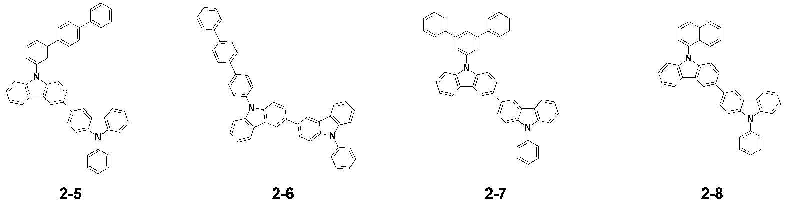

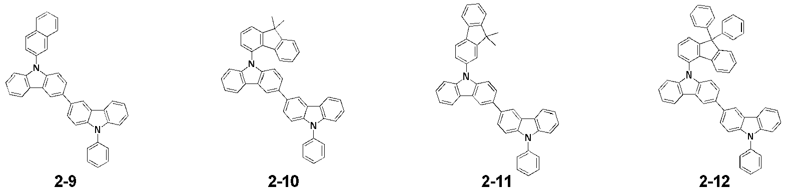

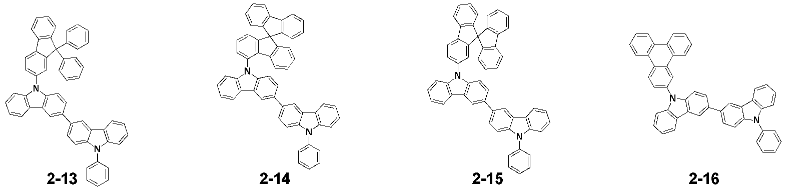

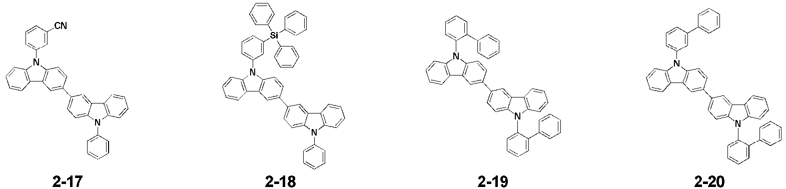

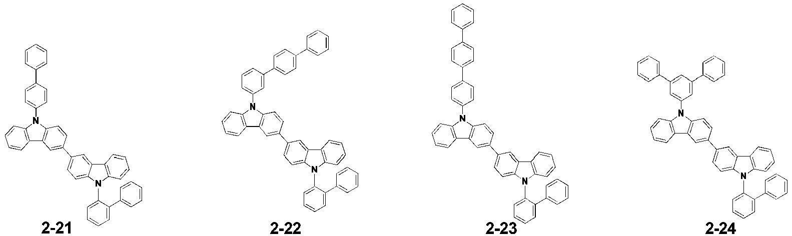

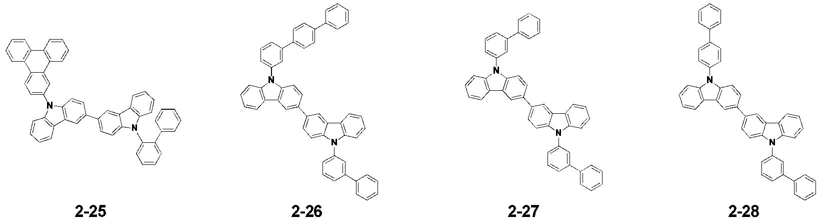

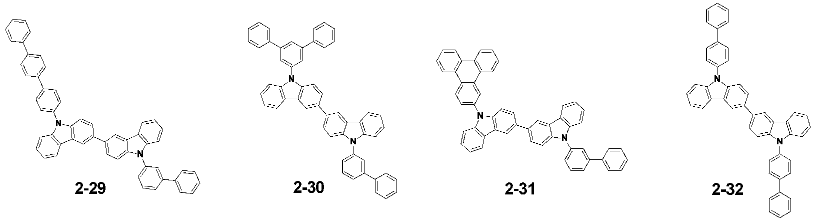

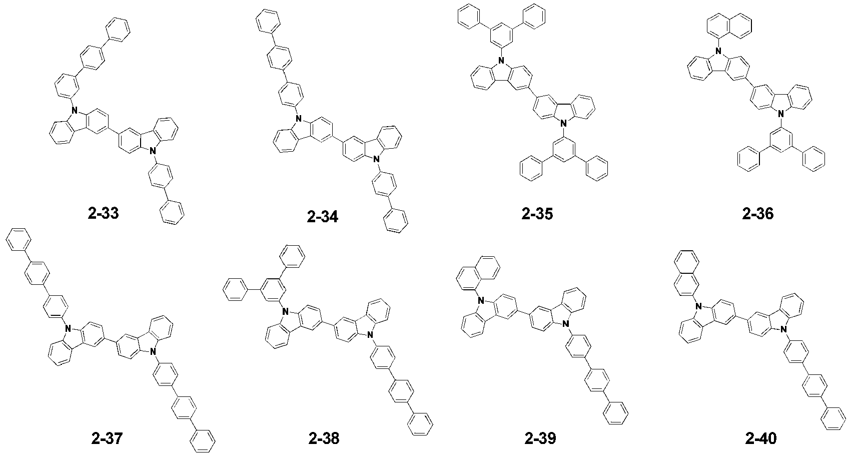

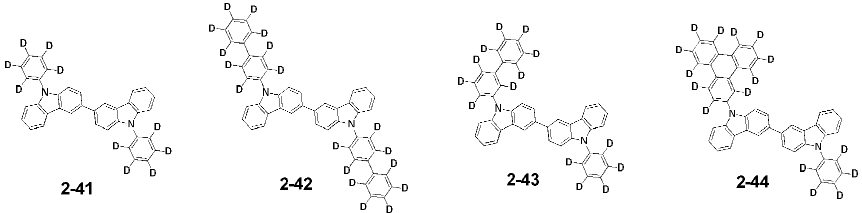

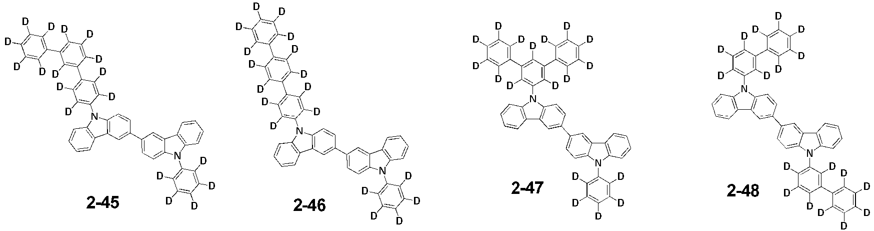

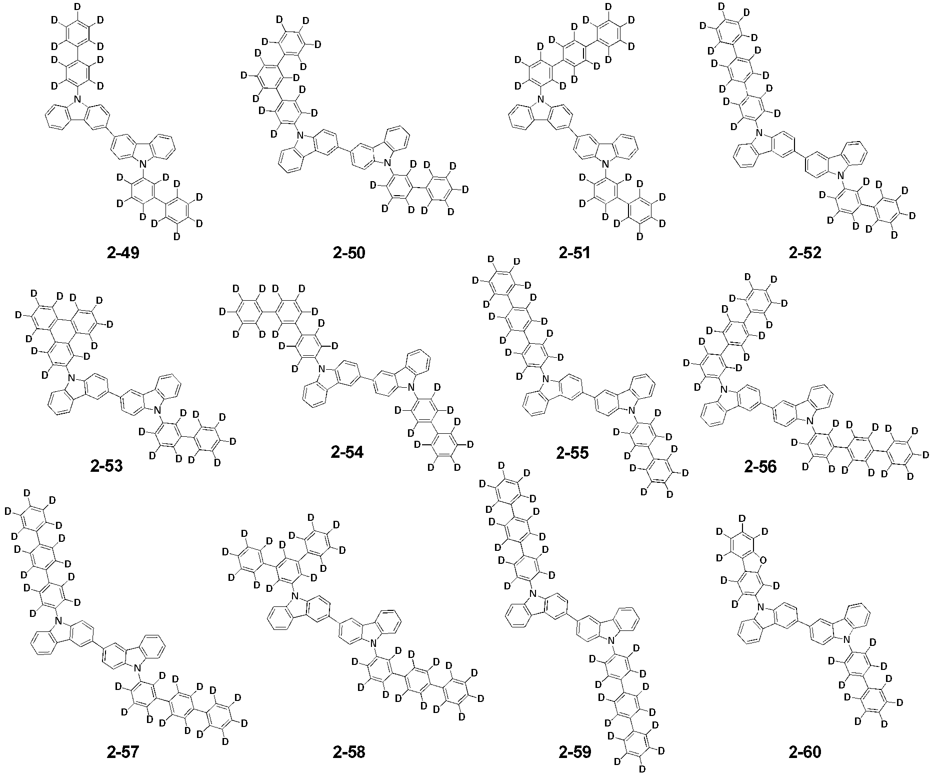

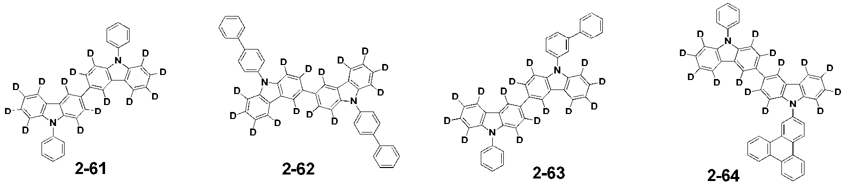

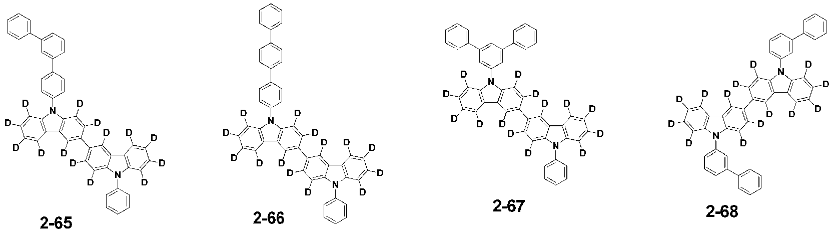

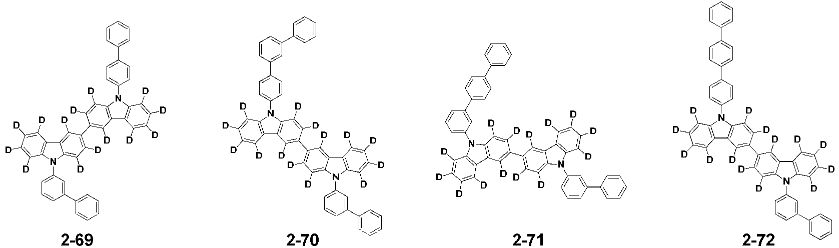

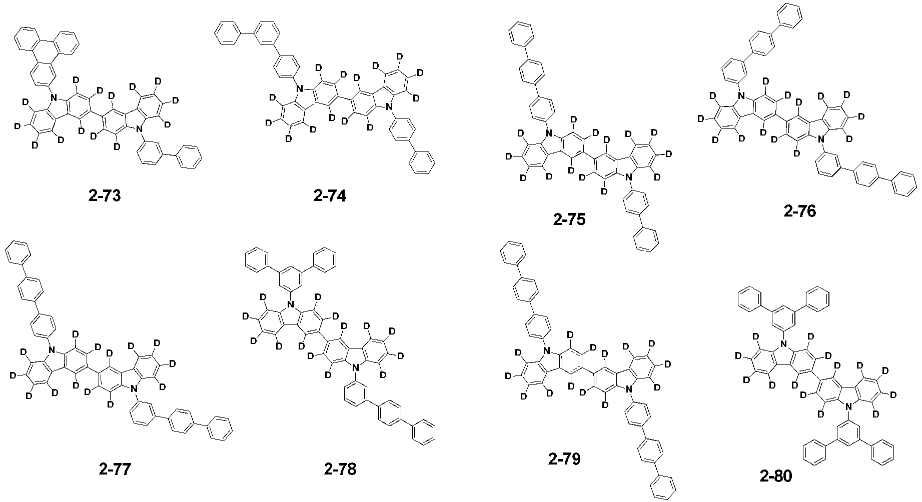

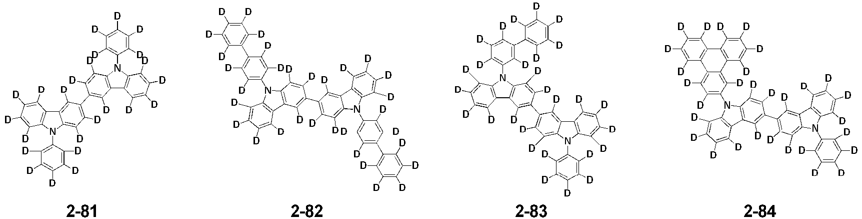

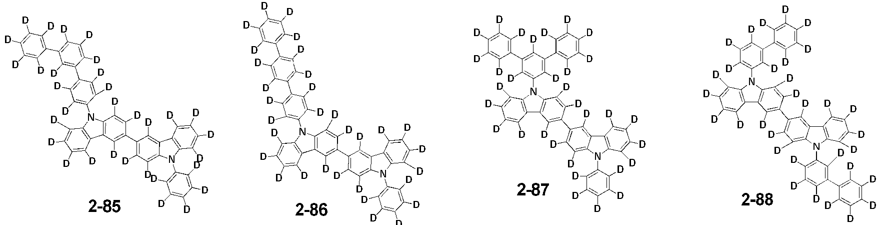

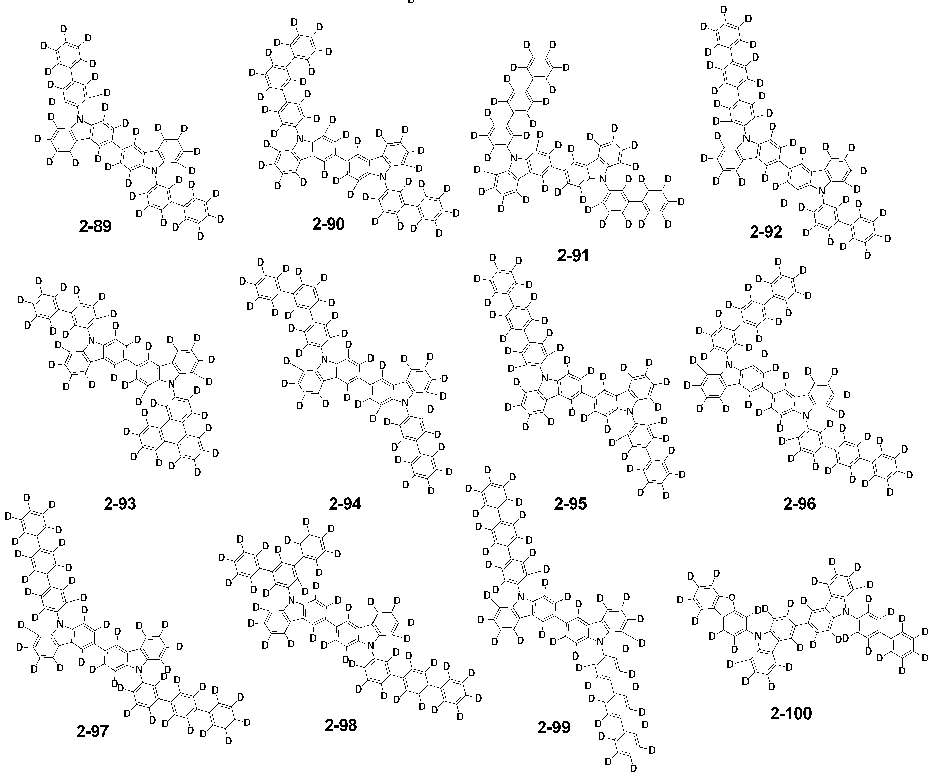

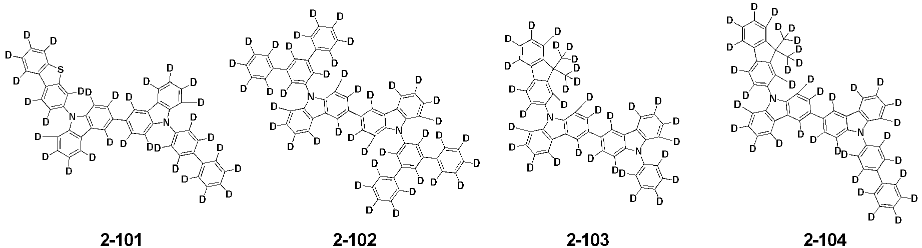

In one embodiment of the present invention, the heterocyclic compound represented by Chemical Formula 3 may be represented by any one of the following Formula 3-1 to 3-6. 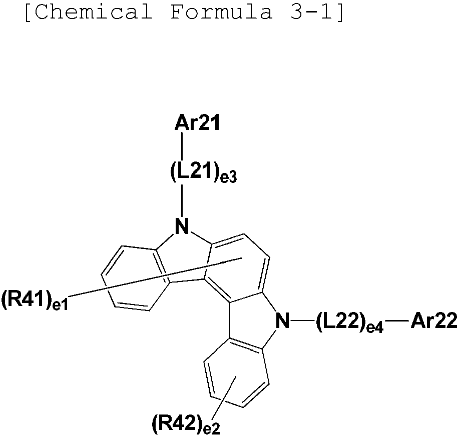 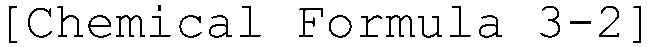 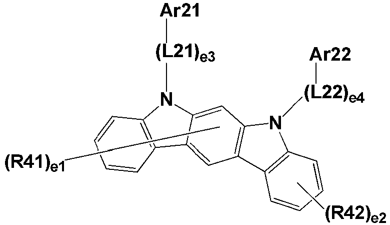 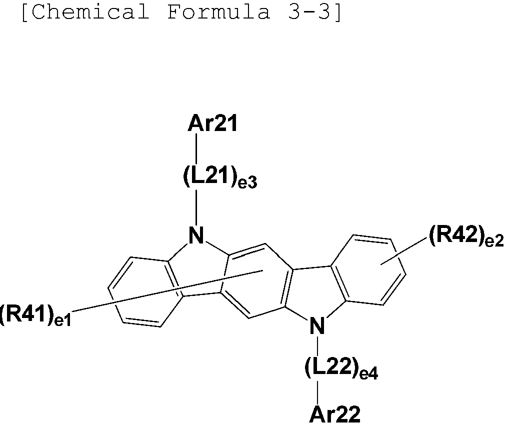 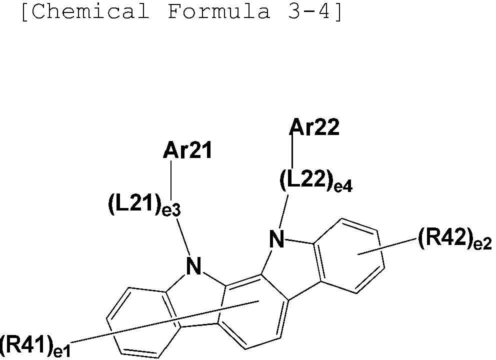 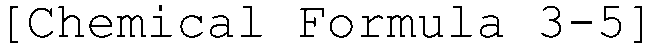 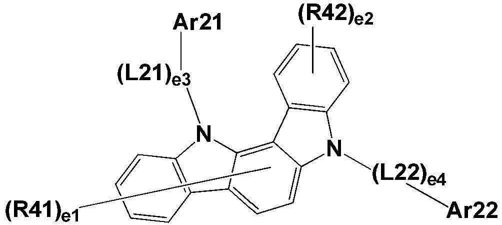 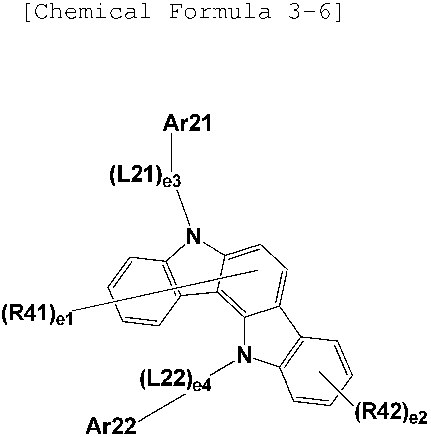 in Formulae 3-1 to 3-6,
R41, R42, Ar21, Ar22, L21, L22, and e1 to e4 are the same as defined in Chemical Formula 3.

In one embodiment of the present invention, the heterocyclic compound represented by Chemical Formula 3 may be represented by any one of the following compounds.

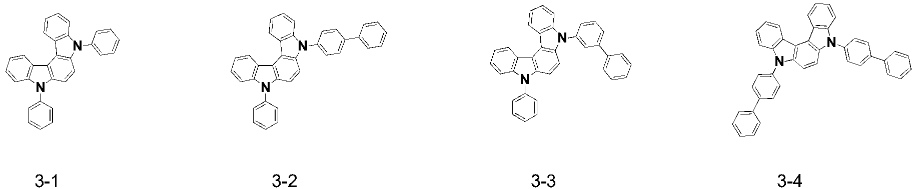

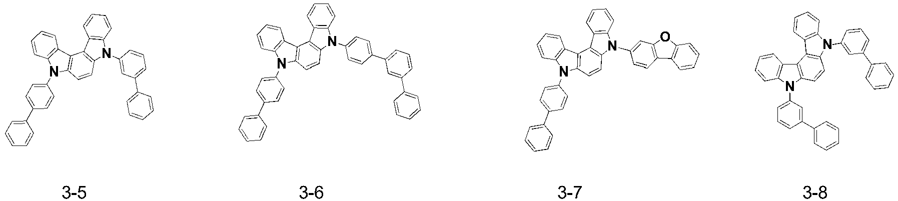

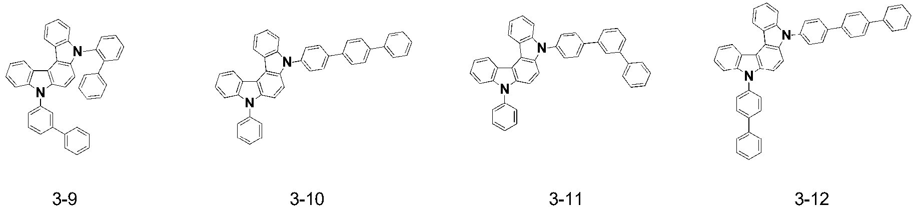

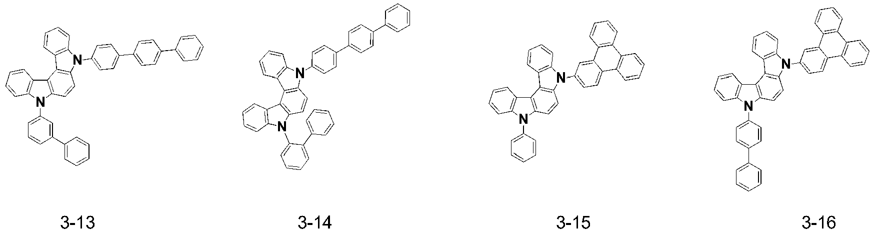

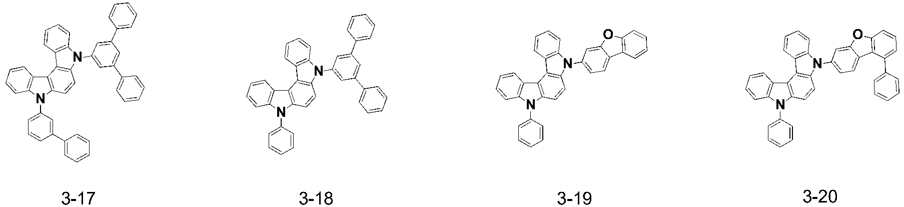

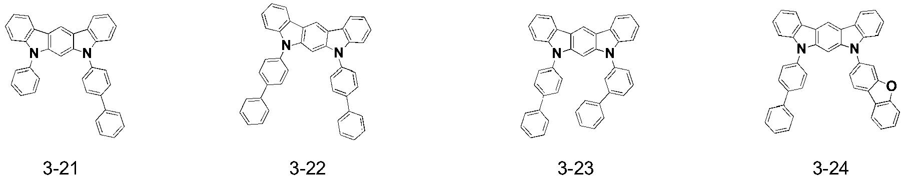

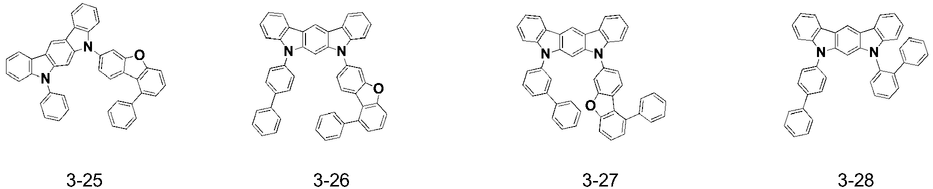

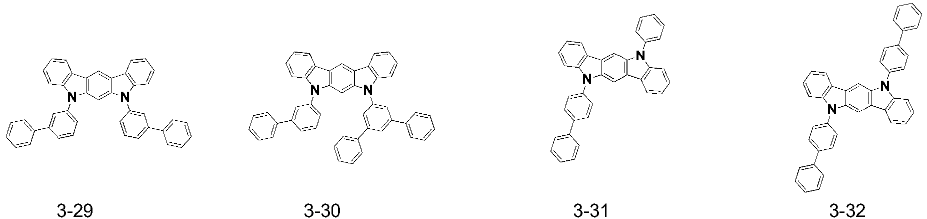

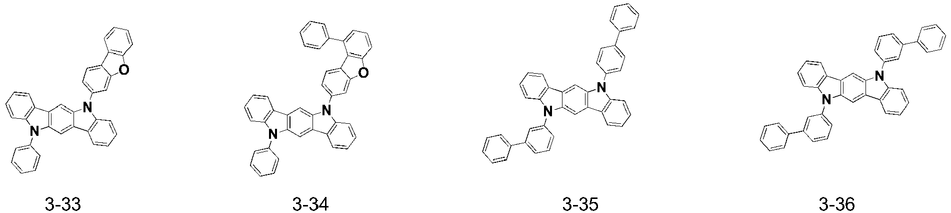

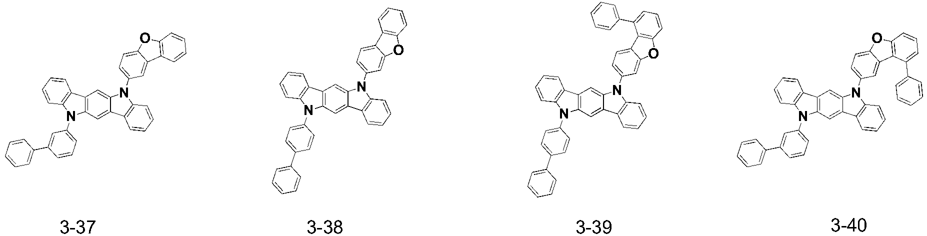

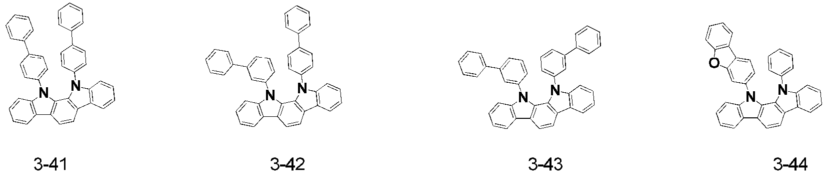

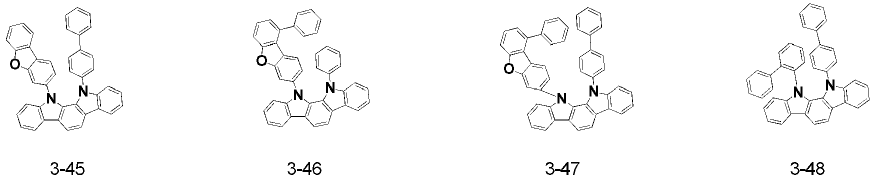

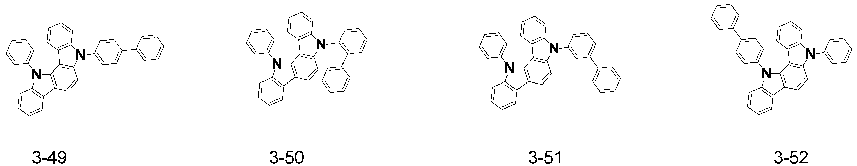

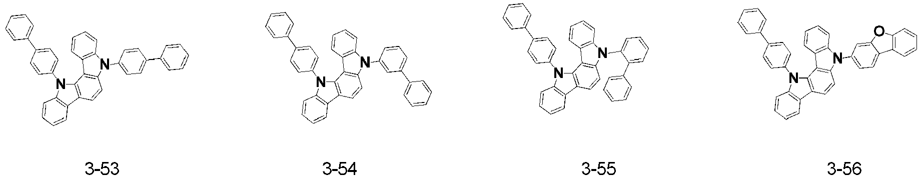

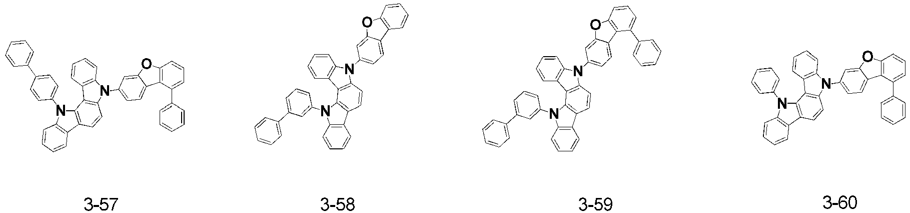

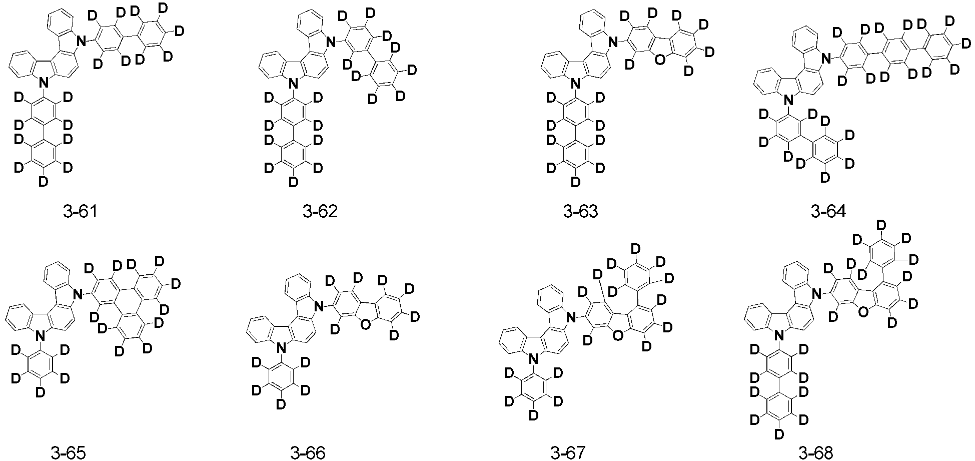

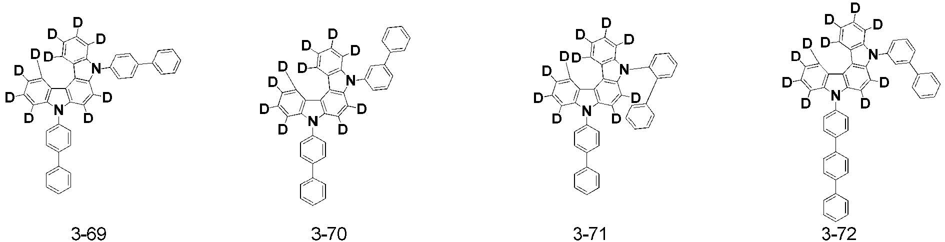

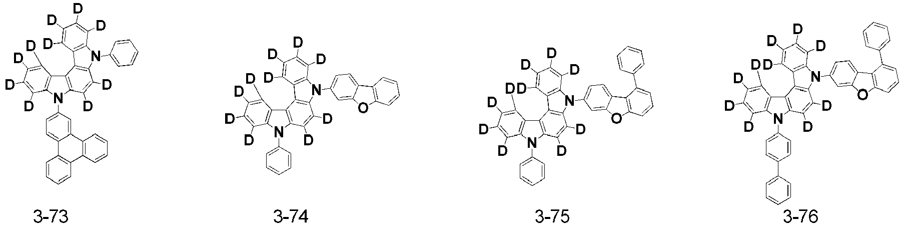

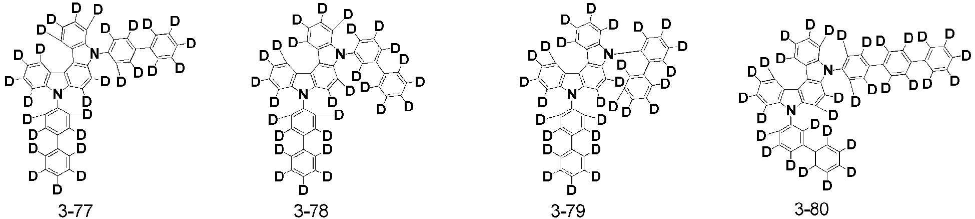

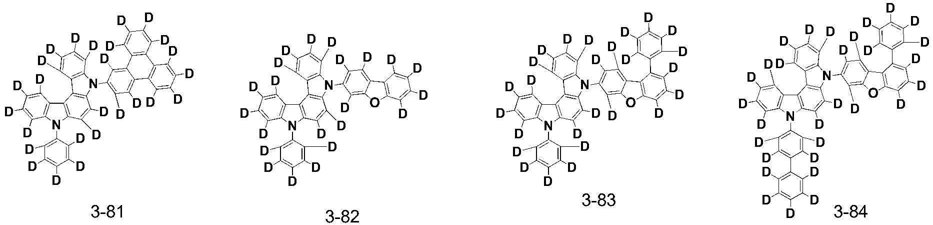

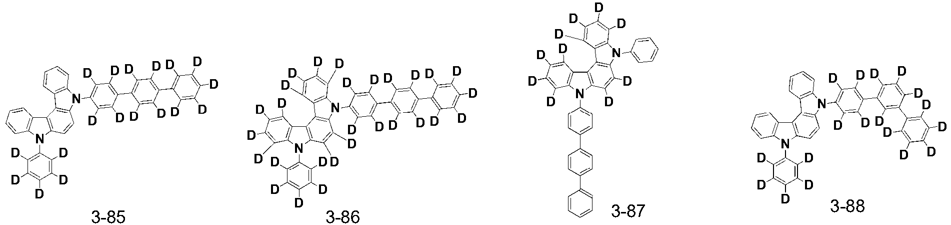

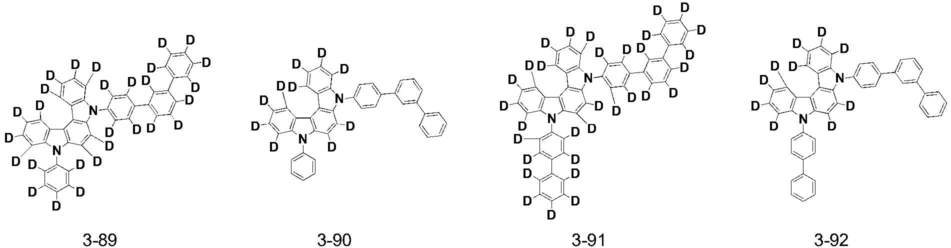

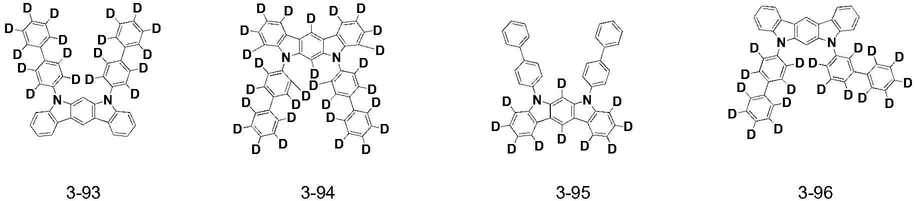

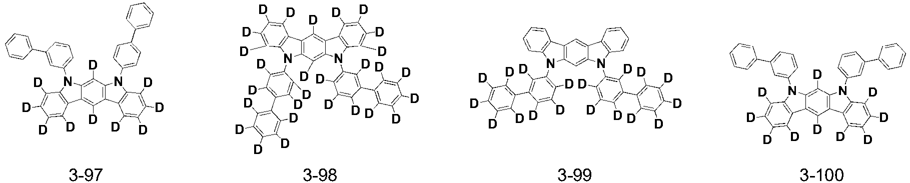

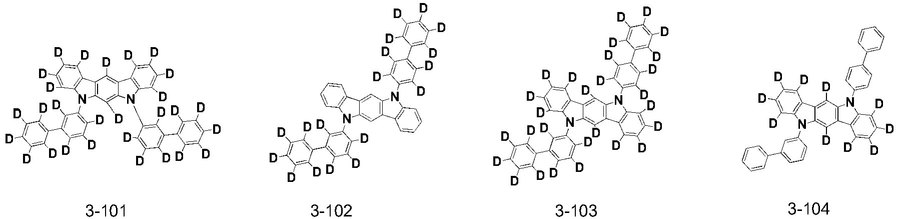

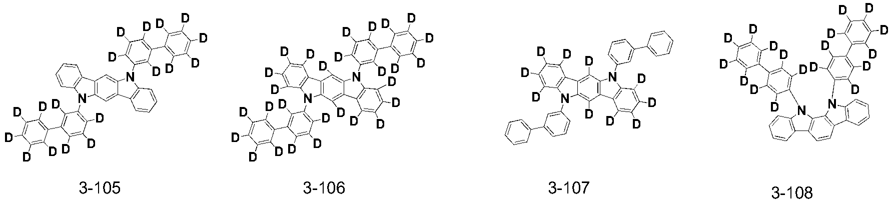

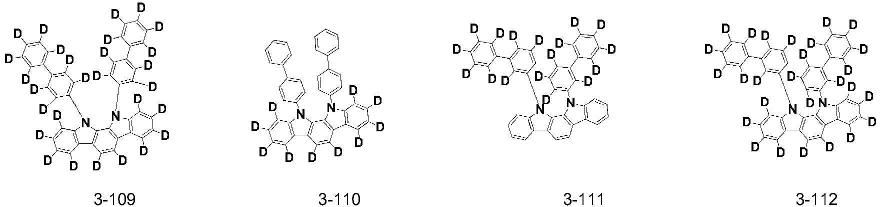

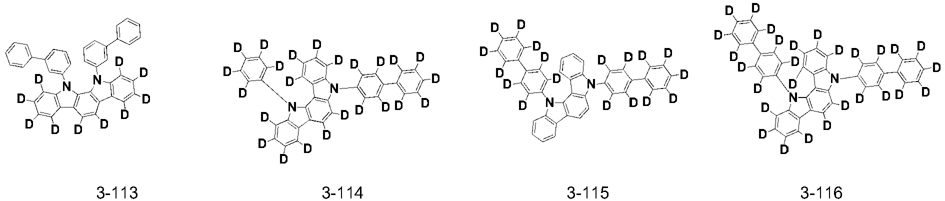

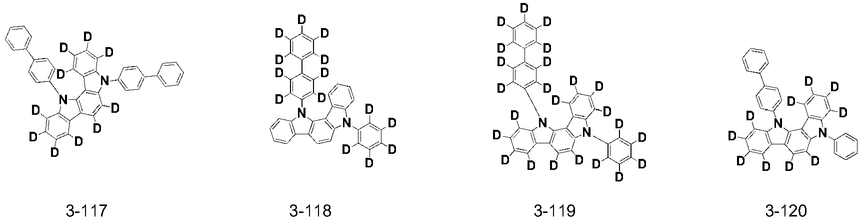

Furthermore, in one embodiment of the present invention, there is provided a composition for an organic material layer comprising the heterocyclic compound represented by Chemical Formula 1 and the heterocyclic compound represented by Chemical Formula 2 or the heterocyclic compound represented by Chemical Formula 3.

Specific details regarding the heterocyclic compounds represented by Formulas 1, 2, and 3 are the same as described above.

In one embodiment of the present invention, a weight ratio of the heterocyclic compound represented by Chemical Formula 1 and the heterocyclic compound represented by Chemical Formula 2 or the heterocyclic compound represented by Chemical Formula 3 in the composition for an organic material layer may be from 1:9 to 9:1, from 1:9 to 5:5, or from 2:8 to 5:5, but is not limited thereto.

In one embodiment of the present invention, a weight ratio of the heterocyclic compound represented by Chemical Formula 1 and the heterocyclic compound represented by Chemical Formula 2 in the composition for an organic material layer may be from 1:9 to 9:1, from 1:9 to 5:5, or from 2:8 to 5:5, but is not limited thereto.

In one embodiment of the present invention, a weight ratio of the heterocyclic compound represented by Chemical Formula 1 and the heterocyclic compound represented by Chemical Formula 3 in the composition for an organic material layer may be from 1:9 to 9:1, from 1:9 to 5:5, or from 2:8 to 5:5, but is not limited thereto.

The composition for an organic material layer may be used in forming an organic material layer of an organic light emitting device, and more preferably, it may be used in forming a host of a emission layer.

In one embodiment of the present invention, the organic material layer comprises the heterocyclic compound represented by Chemical Formula 1 and the heterocyclic compound represented by Chemical Formula 2, and may be used together with a phosphorescent dopant.

In one embodiment of the present invention, the organic material layer comprises the heterocyclic compound represented by Chemical Formula 1 and the heterocyclic compound represented by Chemical Formula 3, and may be used together with a phosphorescent dopant.

As the phosphorescent dopant material, those known in the art may be used. For example, phosphorescent dopant materials represented by LL'MX', LL'L"M, LMX'X", L₂MX', and L₃M may be used, but the scope of the present invention is not limited by these examples.

The M may be iridium, platinum, osmium, or the like.

The L is an anionic bidentate ligand coordinated to the M by an sp² carbon and a heteroatom, and X may perform a function of trapping electrons or holes. Non-limiting examples of L include 2-(1-naphthyl)benzoxazole, 2-phenylbenzoxazole, 2-phenylbenzothiazole, 7,8-benzoquinoline, phenylpyridine, benzothiophenylpyridine, 3-methoxy-2-phenylpyridine, thiophenylpyridine, tolylpyridine, and the like. Non-limiting examples of X' and X" include acetylacetonate (acac), hexafluoroacetylacetonate, salicylidene, picolinate, 8-hydroxyquinolinate, and the like.

Specific examples of the phosphorescent dopant are shown below, but are not limited to these examples.

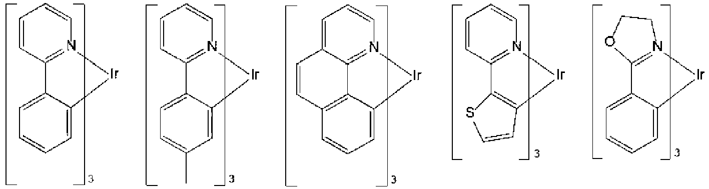

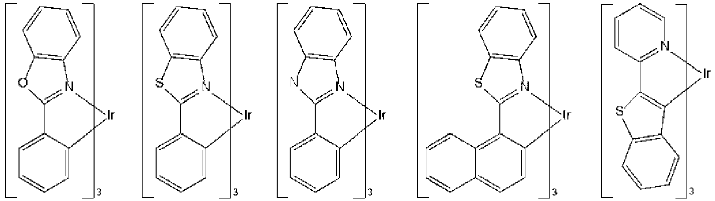

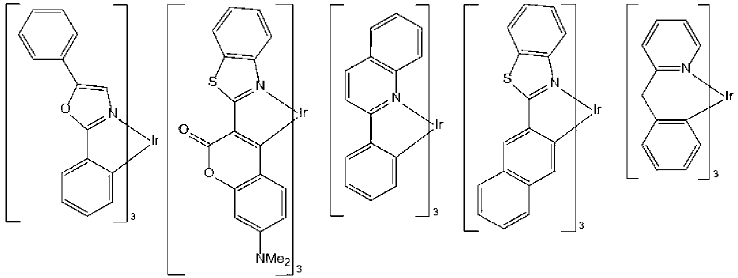

In one embodiment of the present invention, the organic material layer comprises the heterocyclic compound represented by Chemical Formula 1 and the heterocyclic compound represented by Chemical Formula 2, and may be used together with an iridium-based dopant.

In another embodiment of the present invention, the organic material layer comprises the heterocyclic compound represented by Chemical Formula 1 and the heterocyclic compound represented by Chemical Formula 3, and may be used together with an iridium-based dopant.

In one embodiment of the present invention, (piq)₂(Ir) (acac) may be used as a red phosphorescent dopant, or Ir(ppy)₃ may be used as a green phosphorescent dopant, as the iridium-based dopant.

In one embodiment of the present invention, the content of the dopant may range from 1% to 15%, preferably from 2% to 10%, and more preferably from 3% to 7%, based on the total weight of the emission layer.

In an organic light-emitting device according to one embodiment of the present invention, the organic material layer may include an electron injection layer or an electron transport layer, and the electron injection layer or the electron transport layer may include the heterocyclic compound represented by Chemical Formula 1.

In an organic light-emitting device according to another embodiment of the present invention, the organic material layer may include an electron blocking layer or a hole blocking layer, and the electron blocking layer or the hole blocking layer may include the heterocyclic compound represented by Chemical Formula 1.

In an organic light-emitting device according to another embodiment of the present invention, the organic material layer may include an electron transport layer, a emission layer, or a hole blocking layer, and the electron transport layer, the emission layer, or the hole blocking layer may include the heterocyclic compound represented by Chemical Formula 1.

In an organic light-emitting device according to another embodiment of the present invention, the organic material layer may include a emission layer, and the emission layer may include the heterocyclic compound represented by Chemical Formula 1.

In an organic light-emitting device according to another embodiment of the present invention, the organic material layer may include a emission layer, and the emission layer may include the heterocyclic compound represented by Chemical Formula 1 and the heterocyclic compound represented by Chemical Formula 2 or the heterocyclic compound represented by Chemical Formula 3.

In an organic light-emitting device according to another embodiment of the present invention, the organic material layer may include a emission layer, and the emission layer may include a host material, wherein the host material may include the heterocyclic compound represented by Chemical Formula 1.

In an organic light-emitting device according to another embodiment of the present invention, the emission layer may include two or more host materials, wherein at least one of the host materials may include the heterocyclic compound represented by Chemical Formula 1, and another may include the heterocyclic compound represented by Chemical Formula 2.

In an organic light-emitting device according to another embodiment of the present invention, the emission layer may include two or more host materials, wherein at least one of the host materials may include the heterocyclic compound represented by Chemical Formula 1, and another may include the heterocyclic compound represented by Chemical Formula 3.

In an organic light-emitting device according to another embodiment of the present invention, the emission layer may include two or more host materials in a pre-mixed form, wherein at least one of the host materials may include the heterocyclic compound represented by Chemical Formula 1, and another may include the heterocyclic compound represented by Chemical Formula 2.

In an organic light-emitting device according to another embodiment of the present invention, the emission layer may include two or more host materials in a pre-mixed form, wherein at least one of the host materials may include the heterocyclic compound represented by Chemical Formula 1, and another may include the heterocyclic compound represented by Chemical Formula 3.

The term "pre-mixed" means that two or more host materials are mixed in advance and placed in a single source before being deposited to form the organic material layer.

In an organic light-emitting device according to one embodiment of the present invention, the device may further include one or more layers selected from the group consisting of a emission layer, a hole injection layer, a hole transport layer, an electron injection layer, an electron transport layer, an electron blocking layer, and a hole blocking layer.

Figures 1 to 3 illustrate examples of the stacking order of electrodes and organic material layers in the organic light-emitting device according to an embodiment of the present invention. However, it is not intended that the scope of the present application be limited by these drawings, and structures of organic light-emitting devices known in the art may also be applied to the present application.

According to Figure 1, an organic light-emitting device is shown in which an anode 200, an organic material layer 300, and a cathode 400 are sequentially stacked on a substrate 100. However, the structure is not limited thereto, and as shown in Figure 2, an organic light-emitting device may also be implemented in which a cathode, an organic material layer, and an anode are sequentially stacked on a substrate.

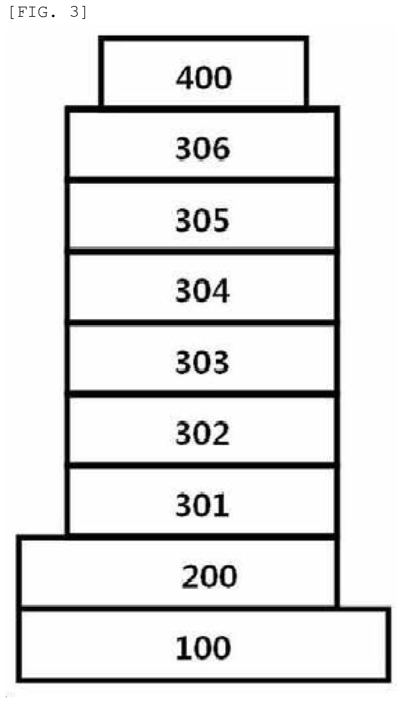

Figure 3 illustrates a case in which the organic material layer has a multi-layer structure. The organic light-emitting device according to Figure 3 includes a hole injection layer 301, a hole transport layer 302, a emission layer 303, a hole blocking layer 304, an electron transport layer 305, and an electron injection layer 306. However, the scope of the present application is not limited by such a layered structure, and, if necessary, layers other than the emission layer may be omitted, or additional functional layers may be further included.

In one embodiment of the present invention, there is provided a method for manufacturing an organic light-emitting device, comprising:
preparing a substrate;
forming a first electrode on the substrate;
forming one or more organic material layers on the first electrode; and
forming a second electrode on the one or more organic material layers, wherein the forming of the one or more organic material layers includes forming the one or more organic material layers using the composition for an organic material layer according to an embodiment of the present invention.

In one embodiment of the present invention, the forming of the organic material layer may include pre-mixing the heterocyclic compound represented by Chemical Formula 1 and the heterocyclic compound represented by Chemical Formula 2, or pre-mixing the heterocyclic compound represented by Chemical Formula 1 and the heterocyclic compound represented by Chemical Formula 3, and forming the layer using a thermal vacuum deposition method.

The term "pre-mixed" means that the heterocyclic compound represented by Chemical Formula 1 and the heterocyclic compound represented by Chemical Formula 2, or the heterocyclic compound represented by Chemical Formula 1 and the heterocyclic compound represented by Chemical Formula 3, are mixed in advance and placed in a single source before being deposited to form the organic material layer.

The pre-mixed material may be referred to as a composition for an organic material layer according to an embodiment of the present application.

The organic material layer including the heterocyclic compound represented by Chemical Formula 1 may further include other materials, if necessary.

The organic material layer including both the heterocyclic compound represented by Chemical Formula 1 and the heterocyclic compound represented by Chemical Formula 2 may further include other materials, if necessary.

The organic material layer including both the heterocyclic compound represented by Chemical Formula 1 and the heterocyclic compound represented by Chemical Formula 3 may further include other materials, if necessary.

In the organic light-emitting device according to one embodiment of the present invention, materials other than the heterocyclic compound represented by Chemical Formula 1, the heterocyclic compound represented by Chemical Formula 2, or the heterocyclic compound represented by Chemical Formula 3 are exemplified below; however, these are provided for illustrative purposes only and are not intended to limit the scope of the present application, and may be replaced with materials known in the art.

As the anode material, materials having a relatively high work function may be used, and transparent conductive oxides, metals, or conductive polymers may be employed. Specific examples of the anode material include metals such as vanadium, chromium, copper, zinc, and gold, or alloys thereof; metal oxides such as zinc oxide, indium oxide, indium tin oxide (ITO), and indium zinc oxide (IZO); combinations of metals and oxides such as ZnO:Al or SnO₂:Sb; and conductive polymers such as poly(3-methylthiophene), poly[3,4-(ethylene-1,2-dioxy)thiophene] (PEDOT), polypyrrole, and polyaniline, but are not limited thereto.

As the cathode material, materials having a relatively low work function may be used, and metals, metal oxides, or conductive polymers may be employed. Specific examples of the cathode material include metals such as magnesium, calcium, sodium, potassium, titanium, indium, yttrium, lithium, gadolinium, aluminum, silver, tin, and lead, or alloys thereof; and multilayer structures such as LiF/Al or LiO₂/Al, but are not limited thereto.

As the hole injection layer material, known hole injection materials may be used. For example, phthalocyanine compounds such as copper phthalocyanine disclosed in U.S. Patent No. 4,356,429, or starburst-type amine derivatives described in the literature [Advanced Materials, 6, p. 677 (1994)] may be used. Specific examples include tris(4-carbazoyl-9-ylphenyl) amine (TCTA), 4,4',4"-tris[phenyl(m-tolyl)amino]triphenylamine (m-MTDATA), and 1,3,5-tris[4-(3-methylphenylphenylamino)phenyl]benzene (m-MTDAPB). In addition, soluble conductive polymers such as polyaniline/dodecylbenzenesulfonic acid, poly(3,4-ethylenedioxythiophene)/poly(4-styrenesulfonate), polyaniline/camphorsulfonic acid, or polyaniline/poly(4-styrenesulfonate) may also be used.

As the hole transport layer material, pyrazoline derivatives, arylamine-based derivatives, stilbene derivatives, and triphenyldiamine derivatives may be used, and either low-molecular-weight or polymer materials may be employed.

As the electron transport layer material, oxadiazole derivatives; anthraquinodimethane and derivatives thereof; benzoquinone and derivatives thereof; naphthoquinone and derivatives thereof; anthraquinone and derivatives thereof; tetracyanoanthraquinodimethane and derivatives thereof; fluorenone derivatives; diphenyldicyanoethylene and derivatives thereof; diphenoquinone derivatives; and metal complexes of 8-hydroxyquinoline and derivatives thereof may be used, and not only low-molecular-weight materials but also polymer materials may be employed.

As the electron injection layer material, for example, LiF is commonly used in the art, but the present application is not limited thereto.

As the emission layer material, red, green, or blue light-emitting materials may be used, and if necessary, two or more light-emitting materials may be mixed and used. In this case, the two or more light-emitting materials may be deposited from separate sources, or may be pre-mixed and deposited from a single source. In addition, a fluorescent material or a phosphorescent material may be used as the emission layer material. The emission layer material may be a material that emits light by combining holes and electrons injected from the anode and cathode, respectively, or may be a material in which both a host material and a dopant material are involved in light emission.

When host materials of the emission layer are used in combination, hosts of the same class may be mixed, or hosts of different classes may be mixed. For example, two or more materials selected from n-type host materials or p-type host materials may be used as host materials of the emission layer.

The organic light-emitting device according to an embodiment of the present invention may be a top-emission type, a bottom-emission type, or a dual-emission type depending on the materials used.

The heterocyclic compound according to an embodiment of the present invention may also operate based on a principle similar to that applied in organic light-emitting devices in other organic electronic devices, including organic solar cells, organic photoconductors, and organic transistors.

### [Mode for Carrying Out the Invention]

Hereinafter, preferred Examples are provided to facilitate understanding of the present invention; however, the following Examples are provided for illustrative purposes only and are not intended to limit the present invention.

### <Preparation Examples>

### Preparation Example 1. Preparation of Compound 1-1

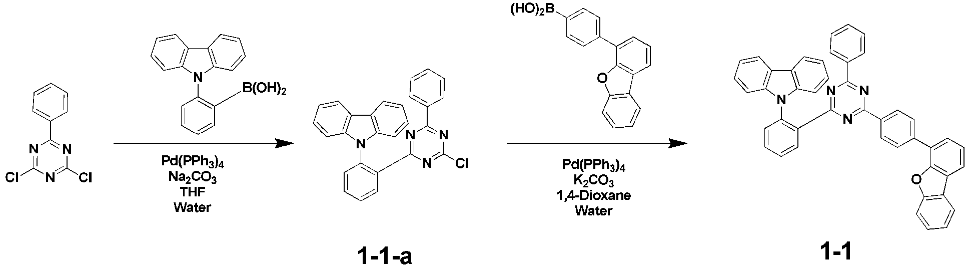

### Preparation Example 1-1. Preparation of Compound 1-1-a

30.0 g (132.7 mmol) of 2,6-dichloro-6-phenyl-1,3,5-triazine, 38.1 g (132.7 mmol) of (2-(9H-carbazol-9-yl)phenyl)boronic acid, 7.7 g (6.6 mmol) of Pd₂(PPh₃)₄, 28.1 g (265.4 mmol) of Na₂CO₃, 300 mL of tetrahydrofuran (THF), and 90 mL of distilled water were added and refluxed with stirring for 6 hours.

After completion of the reaction, distilled water and dichloromethane were added at room temperature, followed by extraction. The organic layer was dried over MgSO₄, and the solvent was removed using a rotary evaporator. The product was purified by column chromatography (n-hexane/dichloromethane = 4:1 (v/v)) to obtain Compound 1-1-a (41.5 g, yield: 72%).

### Preparation Example 1-2. Preparation of Compound 1-1

20.0 g (46.2 mmol) of Compound 1-1-a, 13.3 g (46.2 mmol) of (4-(dibenzo[b,d]furan-4-yl)phenyl)boronic acid, 2.7 g (2.3 mmol) of Pd₂(PPh₃)₄, 12.8 g (92.4 mmol) of K₂CO₃, 200 mL of 1,4-dioxane, and 60 mL of distilled water were added and refluxed with stirring for 6 hours.

After completion of the reaction, distilled water and dichloromethane were added at room temperature, followed by extraction. The organic layer was dried over MgSO₄ , and the solvent was removed using a rotary evaporator. The product was purified by column chromatography (n-hexane/dichloromethane = 3:1 (v/v)) to obtain Compound 1-1 (25.0 g, yield: 84%).

In Preparation Example 1, target compounds shown in Table 1 were synthesized in the same manner as in Preparation Example 1, except that Compound A of Table 1 was used instead of 2,6-dichloro-6-phenyl-1,3,5-triazine, Compound B of Table 1 was used instead of (2-(9H-carbazol-9-yl)phenyl)boronic acid, and Compound C of Table 1 was used instead of (4-(dibenzo[b,d]furan-4-yl)phenyl)boronic acid. The yields refer to those of the final reaction.

**[Table 1]**

| Compound No. | Compound A | Compound B | Compound C | Target Compound | Yield |
|---|---|---|---|---|---|
| 1-2 | 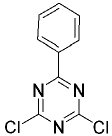 | 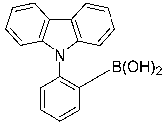 | 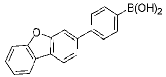 | 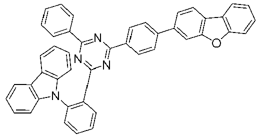 | 86% |
| 1-5 | 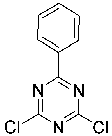 | 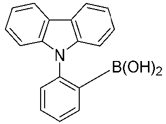 | 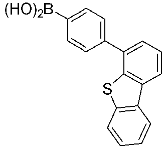 | 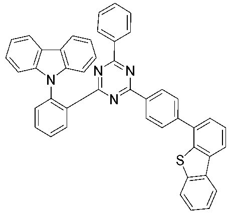 | 83% |
| 1-7 | 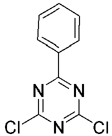 | 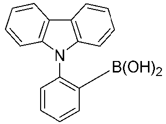 | 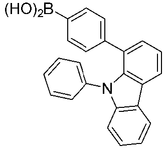 | 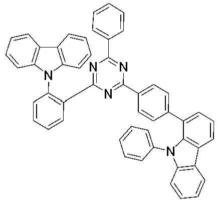 | 78% |
| 1-25 | 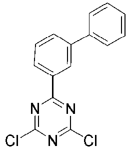 | 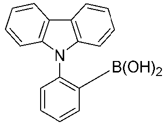 | 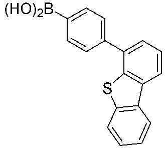 | 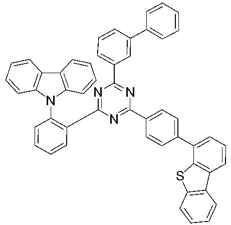 | 89% |
| 1-26 | 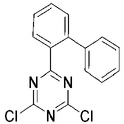 | 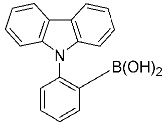 | 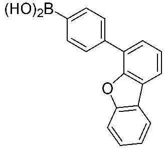 | 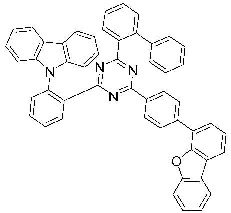 | 83% |
| 1-31 | 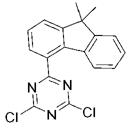 | 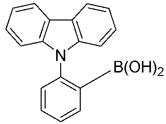 | 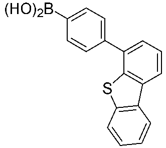 | 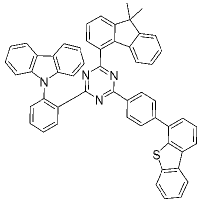 | 82% |
| 1-35 | 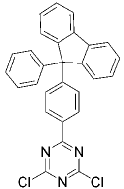 | 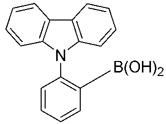 | 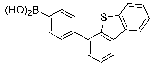 | 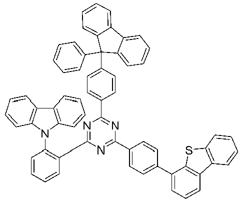 | 83% |
| 1-53 | 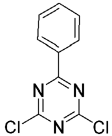 | 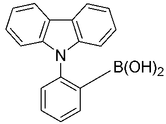 | 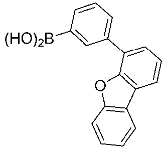 | 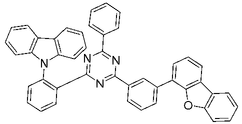 | 84% |
| 1-54 | 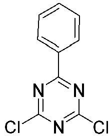 | 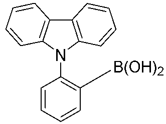 | 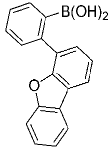 | 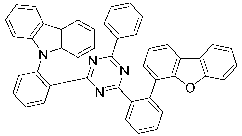 | 79% |
| 1-57 | 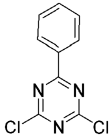 | 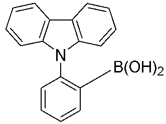 | 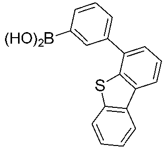 | 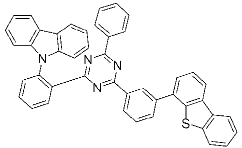 | 85% |
| 1-60 | 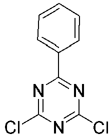 | 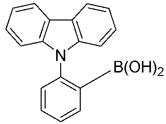 | 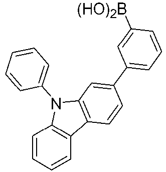 | 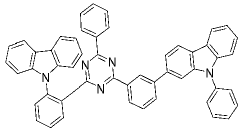 | 83% |
| 1-107 | 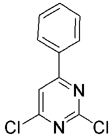 | 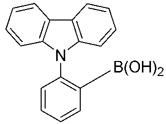 | 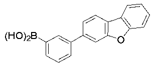 | 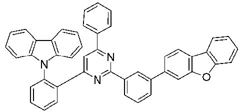 | 87% |
| 1-109 | 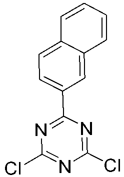 | 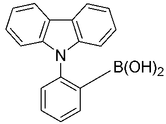 | 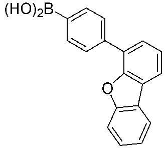 | 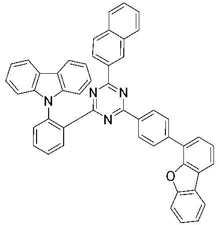 | 89% |
| 1-111 | 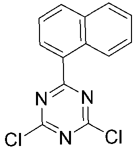 | 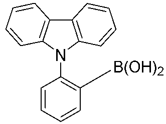 | 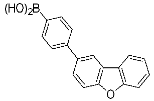 | 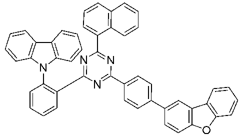 | 87% |
| 1-140 | 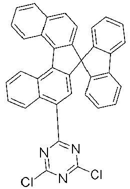 | 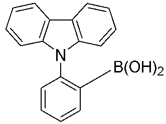 | 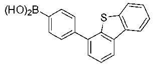 | 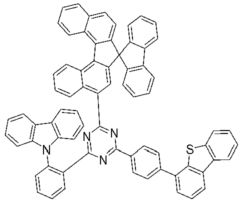 | 79% |
| 1-161 | 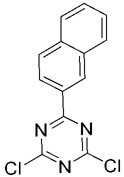 | 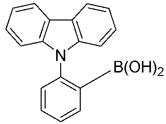 | 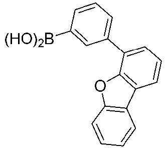 | 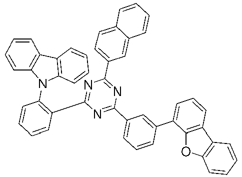 | 79% |
| 1-165 | 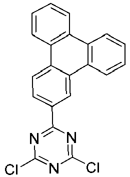 | 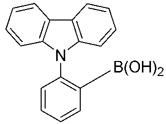 | 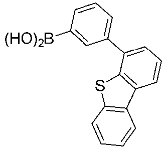 | 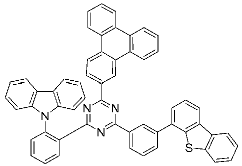 | 91% |
| 1-168 | 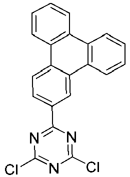 | 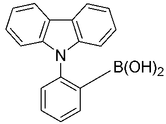 | 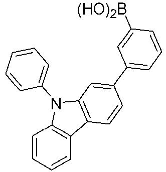 | 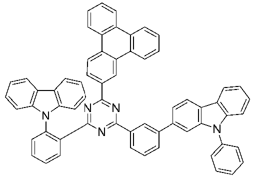 | 90% |
| 1-219 | 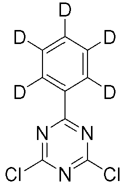 | 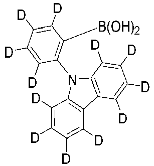 | 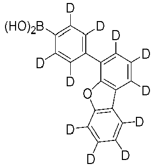 | 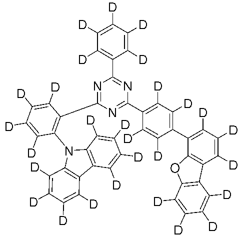 | 84% |
| 1-220 | 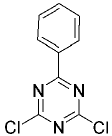 | 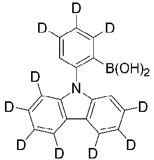 | 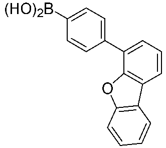 | 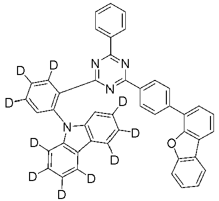 | 87% |
| 1-221 | 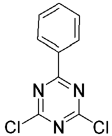 | 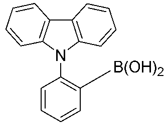 | 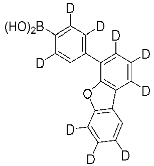 | 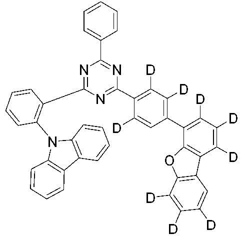 | 86% |
| 1-227 | 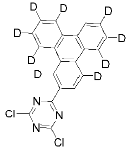 | 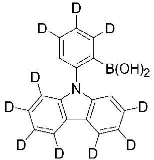 | 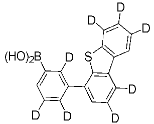 | 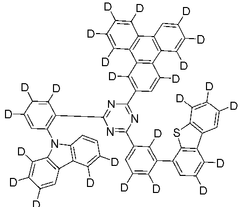 | 82% |

### Preparation Example 2. Preparation of Compound 1-82

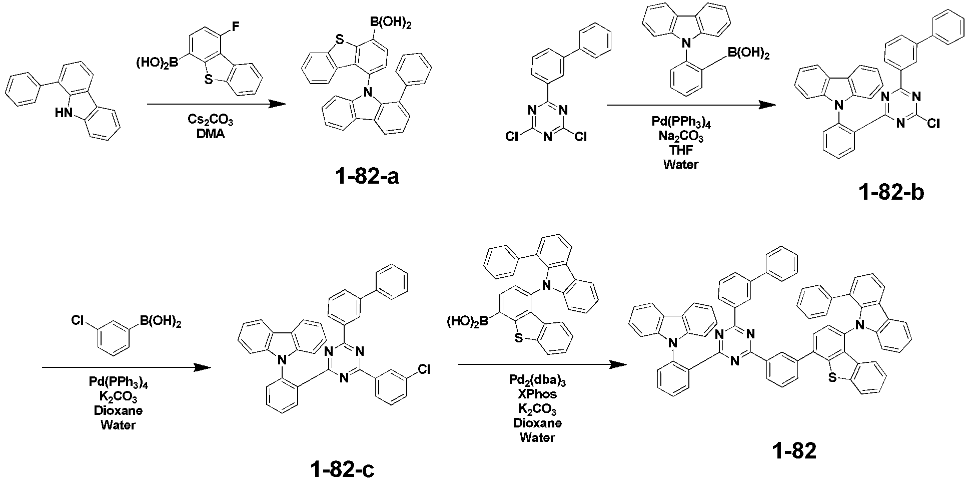

### Preparation Example 2-1. Preparation of Compound 1-82-a

30.0 g (123.3 mmol) of 1-phenyl-9H-carbazole, 30.3 g (123.3 mmol) of (1-fluorodibenzo[b,d]thiophen-4-yl)boronic acid, 80.3 g (246.6 mmol) of Cs₂CO₃, and 300 mL of dimethylacetamide (DMA) were added and refluxed with stirring for 16 hours.

After completion of the reaction, distilled water and ethyl acetate were added at room temperature, followed by extraction. The organic layer was dried over MgSO₄, and the solvent was removed using a rotary evaporator. The product was recrystallized using ethyl acetate and methanol to obtain Compound 1-82-a (51.5 g, yield: 89%).

### Preparation Example 2-2. Preparation of Compound 1-82-b

20.0 g (66.2 mmol) of 2-([1,1'-biphenyl]-3-yl)-4,6-dichloro-1,3,5-triazine, 19.0 g (66.2 mmol) of (2-(9H-carbazol-9-yl)phenyl)boronic acid, 3.8 g (3.3 mmol) of Pd(PPh₃)₄, 14.0 g (132.4 mmol) of Na₂CO₃, 200 mL of tetrahydrofuran (THF), and 60 mL of distilled water were added and refluxed with stirring for 6 hours.

After completion of the reaction, distilled water and dichloromethane were added at room temperature, followed by extraction. The organic layer was dried over MgSO₄, and the solvent was removed using a rotary evaporator. The product was purified by column chromatography (n-hexane/dichloromethane = 4:1 (v/v)) to obtain Compound 1-82-b (25.3 g, yield: 75%).

### Preparation Example 2-3. Preparation of Compound 1-82-c

22.0 g (43.2 mmol) of Compound 1-82-b, 6.8 g (43.2 mmol) of (3-chlorophenyl)boronic acid, 2.5 g (2.2 mmol) of Pd(PPh₃)₄, 11.9 g (8.6 mmol) of K₂CO₃, 220 mL of 1,4-dioxane, and 66 mL of distilled water were added and refluxed with stirring for 6 hours.

After completion of the reaction, distilled water and dichloromethane were added at room temperature, followed by extraction. The organic layer was dried over MgSO₄, and the solvent was removed using a rotary evaporator. The product was purified by column chromatography (n-hexane/dichloromethane = 3:1 (v/v)) to obtain Compound 1-82-c (21.8 g, yield: 86%).

### Preparation Example 2-4. Preparation of Compound 1-82

15.0 g (25.6 mmol) of Compound 1-82-c, 12.0 g (25.6 mmol) of Compound 1-82-a, 1.2 g (1.3 mmol) of Pd₂(dba)₃, 2.4 g (5.2 mmol) of XPhos, 7.1 g (51.2 mmol) of K₂CO₃, 120 mL of 1,4-dioxane, and 63 mL of distilled water were added and refluxed with stirring for 6 hours.

After completion of the reaction, distilled water and dichloromethane were added at room temperature, followed by extraction. The organic layer was dried over MgSO₄, and the solvent was removed using a rotary evaporator. The product was purified by column chromatography (n-hexane/dichloromethane = 2:1 (v/v)) to obtain Compound 1-82 (18.3 g, yield: 73%).

In Preparation Example 2, target compounds shown in Table 2 were synthesized in the same manner as in Preparation Example 2, except that Compound D of Table 2 was used instead of 2-([1,1'-biphenyl]-3-yl)-2,6-dichloro-1,3,5-triazine, Compound E of Table 2 was used instead of (3-chlorophenyl)boronic acid, and Compound F of Table 2 was used instead of Compound 1-82-a. The yields refer to those of the final reaction.

**[Table 2]**

| Compound No. | Compound D | Compound E | Compound F | Target Compound | Yield |
|---|---|---|---|---|---|
| 1-14 | 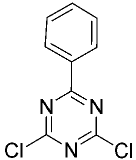 | 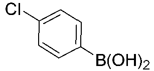 | 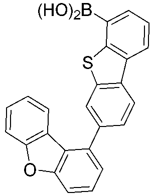 | 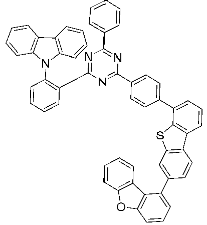 | 74% |
| 1-16 | 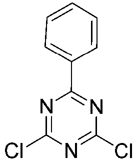 | 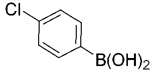 | 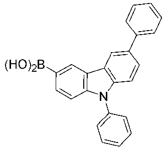 | 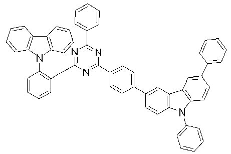 | 76% |
| 1-18 | 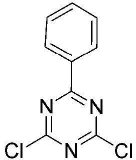 | 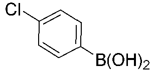 | 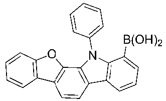 | 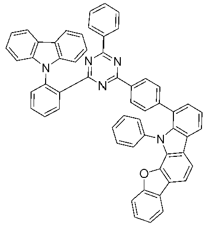 | 69% |
| 1-64 | 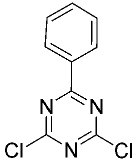 | 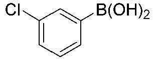 | 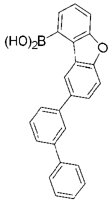 | 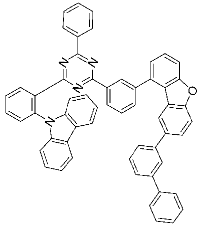 | 72% |
| 1-66 | 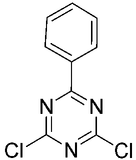 | 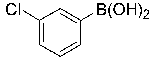 | 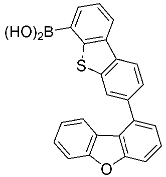 | 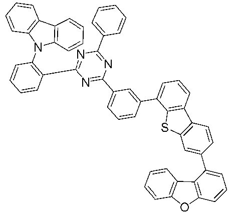 | 72% |
| 1-72 | 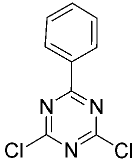 | 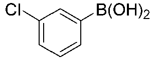 | 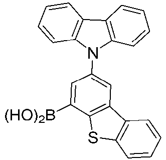 | 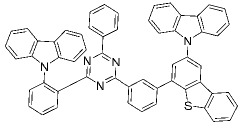 | 73% |
| 1-74 | 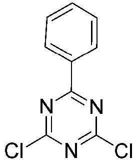 | 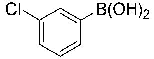 | 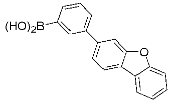 | 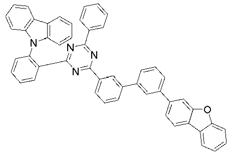 | 75% |
| 1-108 | 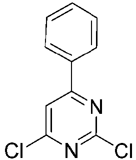 | 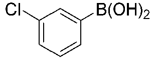 | 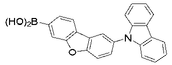 | 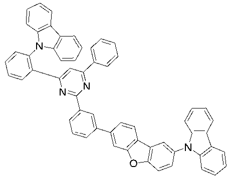 | 74% |
| 1-129 | 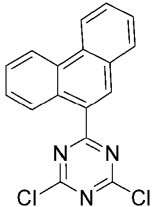 | 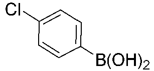 | 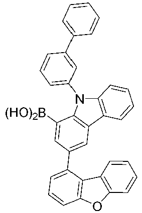 | 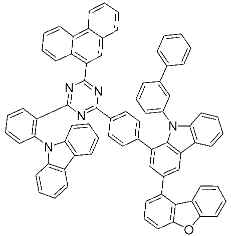 | 72% |
| 1-169 | 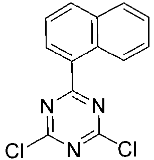 | 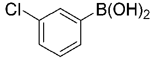 | 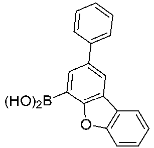 | 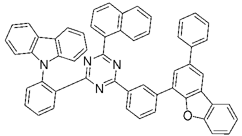 | 75% |
| 1-179 | 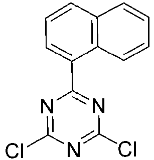 | 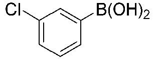 | 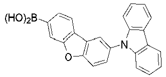 | 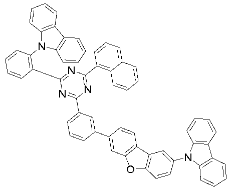 | 73% |

### Preparation Example 3. Preparation of Compound 1-208

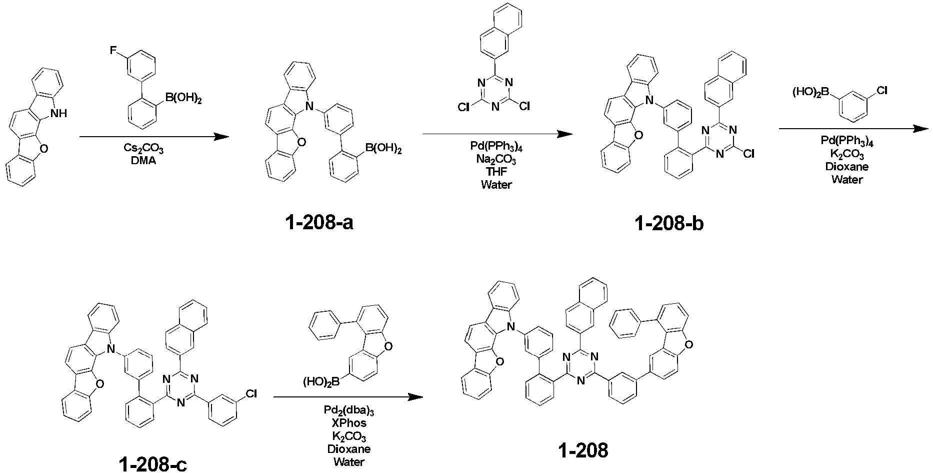

### Preparation Example 3-1. Preparation of Compound 1-208-a

20.0 g (77.8 mmol) of 12H-benzofuro[2,3-a]carbazole, 16.8 g (77.8 mmol) of (3'-fluoro-[1,1'-biphenyl]-2-yl)boronic acid, 50.7 g (155.4 mmol) of Cs₂CO₃, and 200 mL of dimethylacetamide (DMA) were added and refluxed with stirring for 16 hours.

After completion of the reaction, distilled water and ethyl acetate were added at room temperature, followed by extraction. The organic layer was dried over MgSO₄, and the solvent was removed using a rotary evaporator. The product was recrystallized using ethyl acetate and methanol to obtain Compound 1-208-a (32.0 g, yield: 91%).

### Preparation Example 3-2. Preparation of Compound 1-208-b

20 g (44.1 mmol) of Compound 1-208-a, 12.2 g (44.1 mmol) of 2,4-dichloro-6-(naphthalen-2-yl)-1,3,5-triazine, 2.5 g (2.2 mmol) of Pd(PPh₃)₄, 9.4 g (88.2 mmol) of Na₂CO₃, 200 mL of tetrahydrofuran (THF), and 60 mL of distilled water were added and refluxed with stirring for 6 hours.

After completion of the reaction, distilled water and dichloromethane were added at room temperature, followed by extraction. The organic layer was dried over MgSO₄, and the solvent was removed using a rotary evaporator. The product was purified by column chromatography (n-hexane/dichloromethane = 4:1 (v/v)) to obtain Compound 1-208-b (21.0 g, yield: 73%).

### Preparation Example 3-3. Preparation of Compound 1-208-c

20.0 g (30.8 mmol) of Compound 1-208-b, 4.8 g (30.8 mmol) of (3-chlorophenyl)boronic acid, 1.8 g (1.5 mmol) of Pd(PPh₃)₄, 8.5 g (61.6 mmol) of K₂CO₃, 200 mL of 1,4-dioxane, and 60 mL of distilled water were added and refluxed with stirring for 6 hours.

After completion of the reaction, distilled water and dichloromethane were added at room temperature, followed by extraction. The organic layer was dried over MgSO₄, and the solvent was removed using a rotary evaporator. The product was purified by column chromatography (n-hexane/dichloromethane = 3:1 (v/v)) to obtain Compound 1-208-c (19.4 g, yield: 87%).

### Preparation Example 3-4. Preparation of Compound 1-208

20.0 g (27.6 mmol) of Compound 1-208-c, 7.9 g (27.6 mmol) of (9-phenyldibenzo[b,d]furan-2-yl)boronic acid, 1.3 g (1.4 mmol) of Pd₂(dba)₃, 2.6 g (5.5 mmol) of XPhos, 7.6 g (55.2 mmol) of K₂CO₃, 200 mL of 1,4-dioxane, and 60 mL of distilled water were added and refluxed with stirring for 6 hours.

After completion of the reaction, distilled water and dichloromethane were added at room temperature, followed by extraction. The organic layer was dried over MgSO₄, and the solvent was removed using a rotary evaporator. The product was purified by column chromatography (n-hexane/dichloromethane = 2:1 (v/v)) to obtain Compound 1-208 (18.8 g, yield: 73%).

In Preparation Example 3, target compounds shown in Table 3 were synthesized in the same manner as in Preparation Example 3, except that Compound G of Table 3 was used instead of Compound 1-208-a, Compound H of Table 3 was used instead of (3-chlorophenyl)boronic acid, and Compound I of Table 3 was used instead of (9-phenyldibenzo[b,d]furan-2-yl)boronic acid. The yields refer to those of the final reaction.

**[Table 3]**

| Comp ound No. | Compound G | Compound H | Compound I | Target Compound | Yield |
|---|---|---|---|---|---|
| 1-39 | 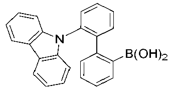 | 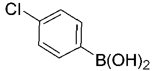 | 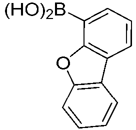 | 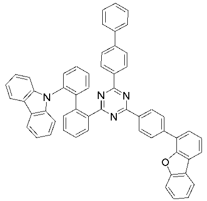 | 76% |
| 1-47 | 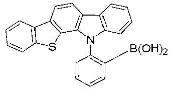 | 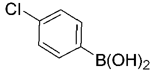 | 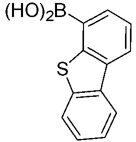 | 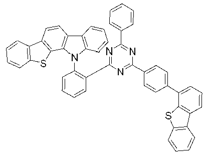 | 77% |
| 1-94 | 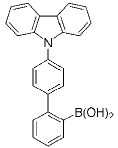 | 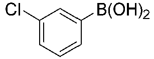 | 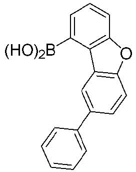 | 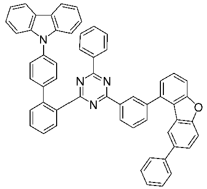 | 74% |
| 1-97 | 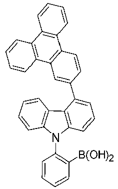 | 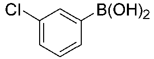 | 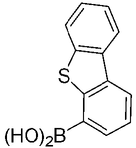 | 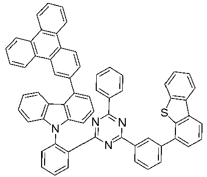 | 78% |
| 1-104 | 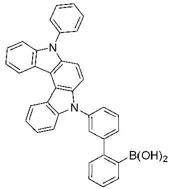 | 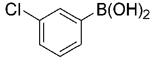 | 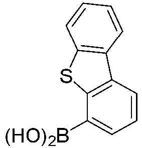 | 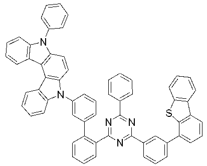 | 78% |
| 1-152 | 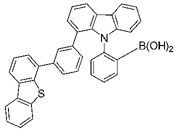 | 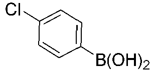 | 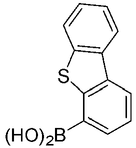 | 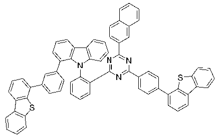 | 76% |
| 1-226 | 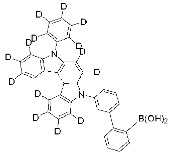 | 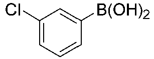 | 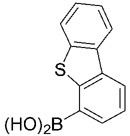 | 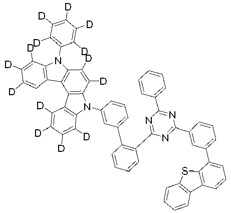 | 76% |
| 1-230 | 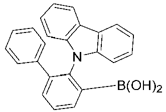 | 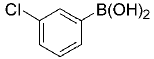 | 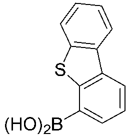 | 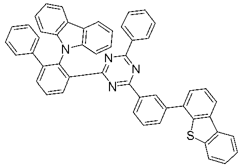 | 77% |
| 1-231 | 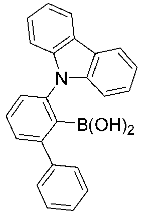 | 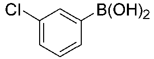 | 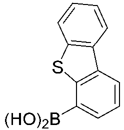 | 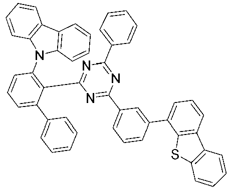 | 78% |

### Preparation Example 4. Preparation of Compound 2-1

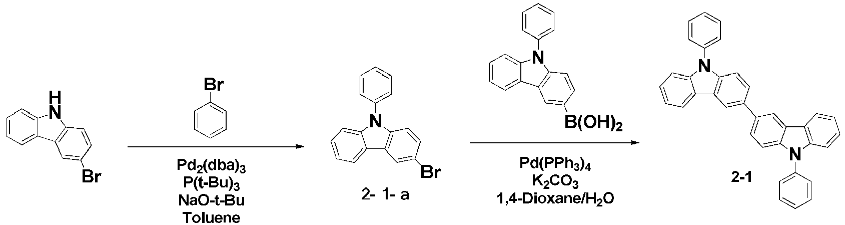

### Preparation Example 4-1. Preparation of Compound 2-1-a

10.0 g (49.6 mmol) of 3-bromo-9H-carbazole, 24.2 g (148.8 mmol) of bromobenzene, 2.3 g (2.5 mmol) of Pd₂(dba)₃, 2.4 mL (9.9 mmol) of P(t-Bu)₃, 9.53 g (99.2 mmol) of NaO-t-Bu, and 100 mL of toluene were added and heated at 135°C for 15 hours.

After completion of the reaction, distilled water and dichloromethane were added, followed by extraction. The product was purified by column chromatography to obtain Compound 2-1-a (14.0 g, yield: 98%).

### Preparation Example 4-2. Preparation of Compound 2-1

14.0 g (43.4 mmol) of Compound 2-1-a, 14.9 g (52.0 mmol) of ((9-phenyl-9H-carbazol-3-yl)boronic acid), 2.5 g (2.2 mmol) of Pd₂(pph₃)₄, 17.9 g (130.0 mmol) of K₂CO₃, 140 mL of 1,4-dioxane, and 35 mL of distilled water were added and refluxed with stirring at 120°C for 6 hours.

Thereafter, the temperature was lowered to room temperature, and the resulting solid was washed with distilled water and methanol to obtain Compound 2-1 (17.0 g, yield: 80%).

In Preparation Example 4, except that Compound a of Table 4 was used instead of bromobenzene and Compound b of Table 4 was used instead of (9-phenyl-9H-carbazol-3-yl)boronic acid, the target compounds of Table 4 were synthesized in the same manner as in Preparation Example 4. The yields represent those of the final reaction.

**[Table 4]**

| Comp ound No. | Compound a | Compound b | Target Compound | Yie ld |
|---|---|---|---|---|
| 2-2 | 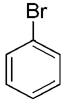 | 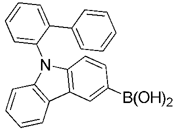 | 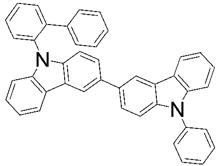 | 82% |
| 2-3 | 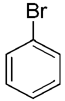 | 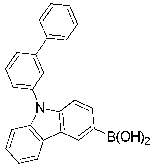 | 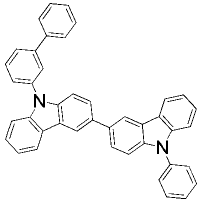 | 80% |
| 2-11 | 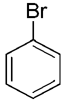 | 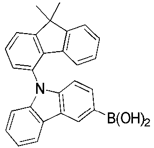 | 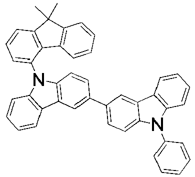 | 81% |
| 2-16 | 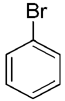 | 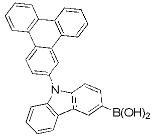 | 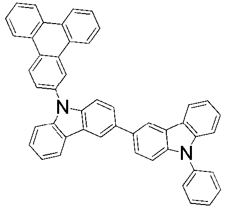 | 80% |
| 2-26 | 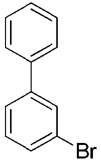 | 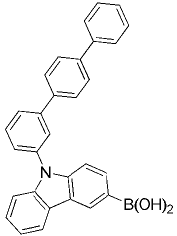 | 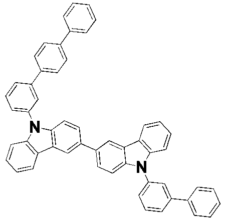 | 81% |
| 2-27 | 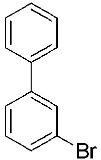 | 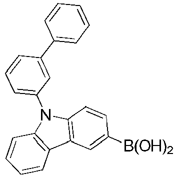 | 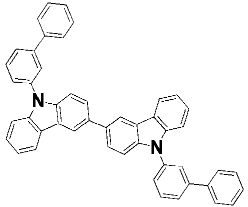 | 85% |
| 2-28 | 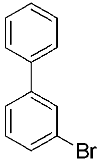 | 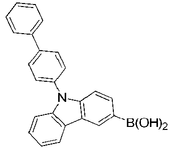 | 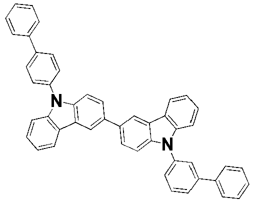 | 81% |
| 2-32 | 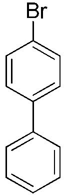 | 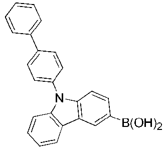 | 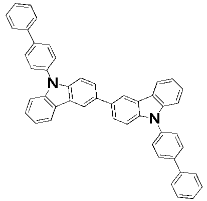 | 81% |
| 2-41 | 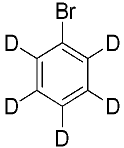 | 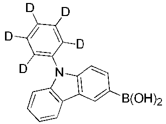 | 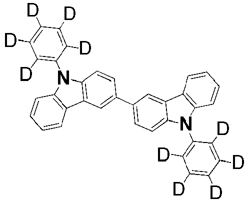 | 80% |
| 2-48 | 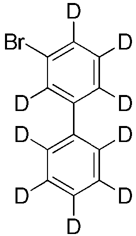 | 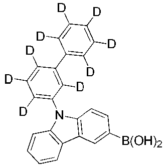 | 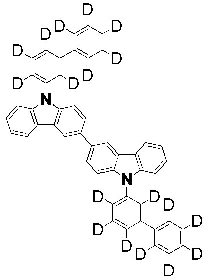 | 81% |
| 2-49 | 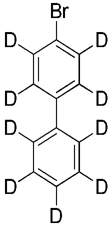 | 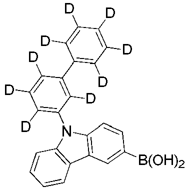 | 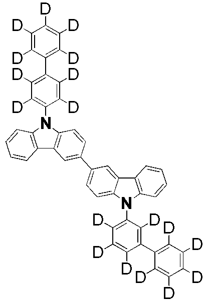 | 83% |
| 2-50 | 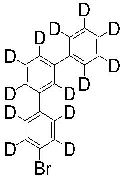 | 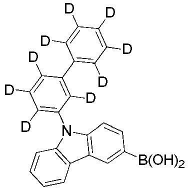 | 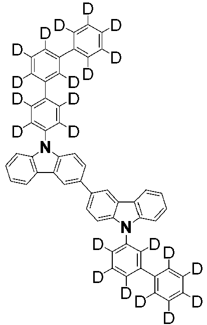 | 82% |
| 2-60 | 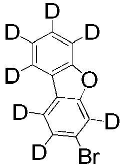 | 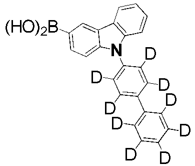 | 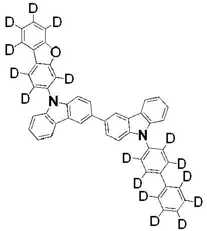 | 82% |

### Preparation Example 5. Preparation of Compound 2-61

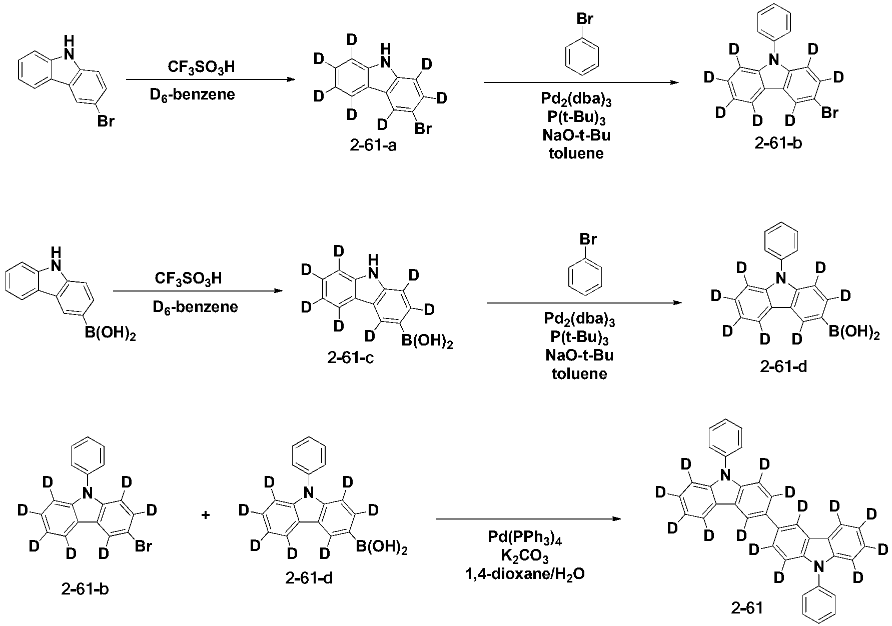

### Preparation Example 5-1. Preparation of Compound 2-61-a

10.0 g(40.2 mmol) of 3-Bromo-9H-carbazole, 1,000 mL of D₆-benzene, and 170.0 g(1.1 mmol) of triflic acid (CF₃SO₃H,) were added and stirred at 50°C.

Upon completion of the reaction, the mixture was neutralized with D₂O, and then extracted with an aqueous Na₂CO₃ solution and dichloromethane at room temperature. The organic layer was dried over MgSO₄, and the solvent was removed using a rotary evaporator.

The residue was purified by column chromatography (n-hexane/dichloromethane = 2:1 (v/v)) and recrystallized from methanol to obtain Compound 2-61-a (10.0 g, yield: 98%).

### Preparation Example 5-2. Preparation of Compound 2-61-b

10.0 g(39.5 mmol) of Compound 2-61-a, 12.4 g(79.0 mmol) of bromobenzene, 1.8 g(2.0 mmol) of Pd₂(dba)₃, 1.9 mL(7.9 mmol) of P(t-Bu)₃, 11.4 g(118.5 mmol) of NaO-t-Bu, and 100 mL of toluene were added and heated at 135°C for 15 hours.

After completion of the reaction, the mixture was extracted with distilled water and dichloromethane, and purified by column chromatography to obtain Compound 2-61-b (11.0 g, yield: 84%).

### Preparation Example 5-3. Preparation of Compound 2-61-c

10.0 g(47.3 mmol) of 9H-Carbazol-3-ylboronic acid, 1,000 mL of D₆-benzene, and 170.0 g(1.1 mmol) of triflic acid (CF₃SO₃H) were added and stirred at 50°C.

Upon completion of the reaction, the mixture was neutralized with D₂O, and then extracted with an aqueous Na₂CO₃ solution and dichloromethane at room temperature. The organic layer was dried over MgSO₄, and the solvent was removed using a rotary evaporator.

The residue was purified by column chromatography (n-hexane/dichloromethane = 2:1 (v/v)) and recrystallized from methanol to obtain Compound 2-61-c (9.0 g, yield: 87%).

### Preparation Example 5-4. Preparation of Compound 2-61-d

9.0 g(41.3 mmol) of Compound 2-61-c, 12.9 g(82.5 mmol) of bromobenzene, 1.9 g(2.1 mmol) of Pd₂(dba)₃, 2.0 mL(8.3 mmol) of P(t-Bu)₃, and 7.9 g(82.6 mmol) of NaO-t-Bu were added, followed by the addition of toluene (100 mL), and the mixture was heated at 135°C for 15 hours.

After completion of the reaction, the mixture was extracted with distilled water and dichloromethane, and purified by column chromatography to obtain Compound 2-61-d (10.0 g, yield: 82%).

### Preparation Example 5-5. Preparation of Compound 2-61

10.0 g(30.4 mmol) of Compound 2-61-b, 17.9 g(60.8 mmol) of Compound 2-61-d, 1.4 g(1.5 mmol) of Pd(pph₃)₄, and 12.6 g(91.1 mmol) of K₂CO₃ were added, followed by the addition of 1,4-dioxane (140 mL) and distilled water (35 mL), and the mixture was stirred at 120°C for 4 hours.

Thereafter, the temperature was lowered to room temperature, and the resulting solid was washed with distilled water and methanol to obtain Compound 2-61 (13.0 g, yield: 85%).

In Preparation Example 5, except that Compound c of Table 5 was used instead of bromobenzene in Preparation Example 5-2 and Compound d of Table 5 was used instead of bromobenzene in Preparation Example 5-4, the target compounds of Table 5 were synthesized in the same manner as in Preparation Example 5. The yields represent those of the final reaction.

**[Table 5]**

| Compou nd No. | Compound c | Compound d | Target Compound | Yield |
|---|---|---|---|---|
| 2-65 | 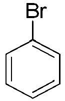 | 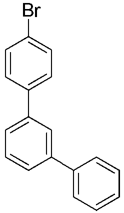 | 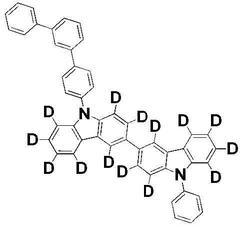 | 81% |
| 2-68 | 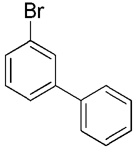 | 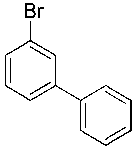 | 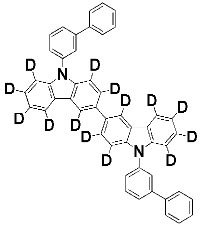 | 84% |
| 2-88 | 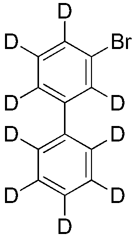 | 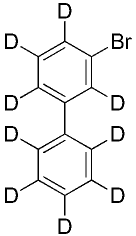 | 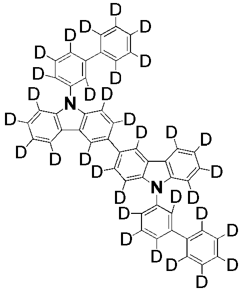 | 82% |
| 2-89 | 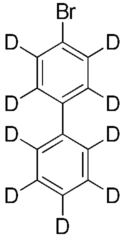 | 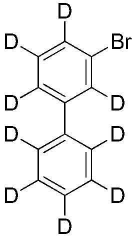 | 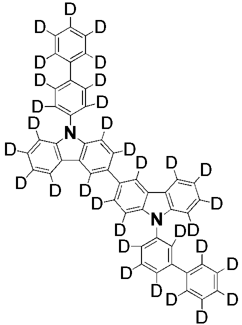 | 82% |
| 2-90 | 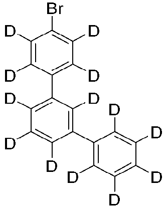 | 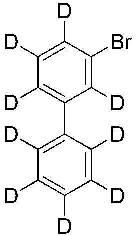 | 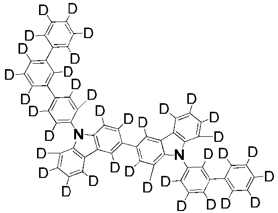 | 84% |
| 2-100 | 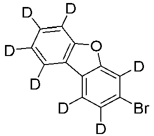 | 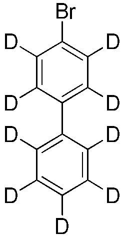 | 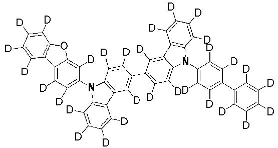 | 84% |
| 2-102 | 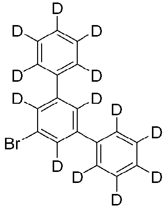 | 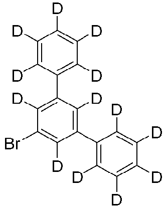 | 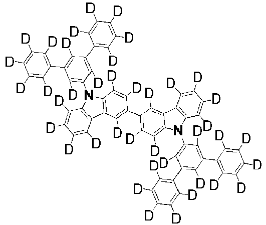 | 81% |

### Preparation Example 6. Preparation of Compound 2-82

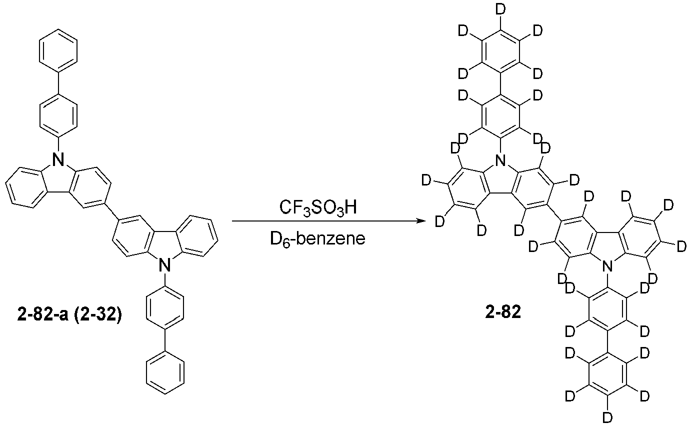

10.0 g(15.7 mmol) of Compound 2-82-a (Compound 2-32), 1,000 mL of D₆-benzene, and 170.0 g(1.1 mmol) of triflic acid (CF₃SO₃H) were added and stirred at 50°C.

Upon completion of the reaction, the mixture was neutralized with D₂O, and then extracted with an aqueous Na₂CO₃ solution and dichloromethane at room temperature. The organic layer was dried over MgSO₄, and the solvent was removed using a rotary evaporator.

The residue was purified by column chromatography (n-hexane/dichloromethane = 2:1 (v/v)) and recrystallized from methanol to obtain the target Compound 2-82 (10.0 g, yield: 95%).

### Preparation Example 7. Preparation of Compound 3-1

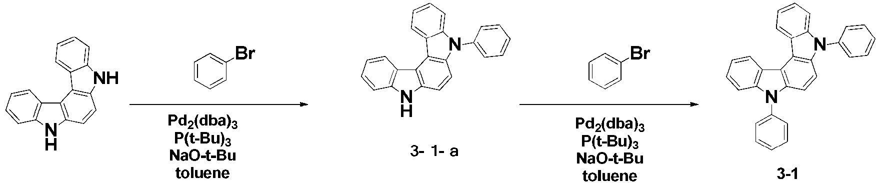

### Preparation Example 7-1. Preparation of Compound 3-1-a

10.0 g(39.0 mmol) of 5,8-Dihydroindolo[2,3-c]carbazole, 6.1 g(39.0 mmol) of bromobenzene, 1.8 g(2.0 mmol) of Pd₂(dba)₃, 0.9 mL(3.9 mmol) of P(t-Bu)₃, 7.5 g(78.0 mmol) of NaO-t-Bu, and toluene (100 mL) were added and heated at 135°C for 15 hours.

After completion of the reaction, the mixture was extracted with distilled water and dichloromethane, and purified by column chromatography to obtain Compound 3-1-a (7.3 g, yield: 56%).

### Preparation Example 7-2. Preparation of Compound 3-1

7.3 g(22.0 mmol) of Compound 3-1-a, 3.8 g(24.2 mmol) of bromobenzene, 1.0 g(1.1 mmol) of Pd₂(dba)₃, 0.5 mL(3.9 mmol) of P(t-Bu)₃, 4.2 g(44.0 mmol) of NaO-t-Bu, and toluene (70 mL) were added and heated at 135°C for 15 hours.

After completion of the reaction, the mixture was extracted with distilled water and dichloromethane, and purified by column chromatography to obtain Compound 3-1 (8.3 g, yield: 93%).

In Preparation Example 7, except that Compound c of Table 6 was used instead of 5,8-dihydroindolo[2,3-c]carbazole, Compound d of Table 6 was used instead of bromobenzene in Preparation Example 7-1, and Compound e of Table 6 was used instead of bromobenzene in Preparation Example 7-2, the target compounds of Table 6 were synthesized in the same manner as in Preparation Example 7. The yields represent those of the final reaction.

**[Table 6]**

| Compo und No. | Compound c | Compound d | Compound e | Target Compound | Yiel d |
|---|---|---|---|---|---|
| 3-4 | 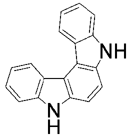 | 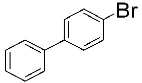 | 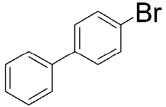 | 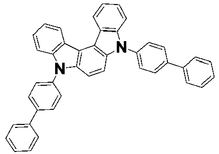 | 93% |
| 3-5 | 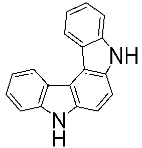 | 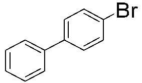 | 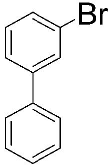 | 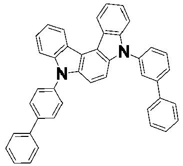 | 92% |
| 3-22 | 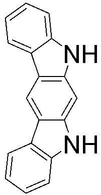 | 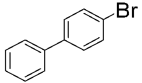 | 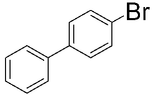 | 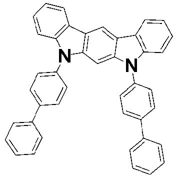 | 89% |
| 3-23 | 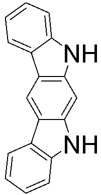 | 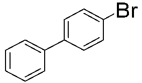 | 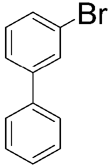 | 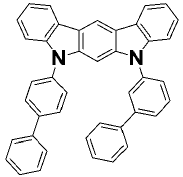 | 89% |
| 3-32 | 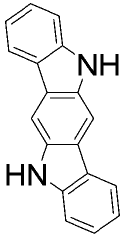 | 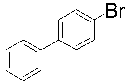 | 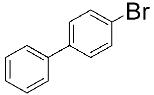 | 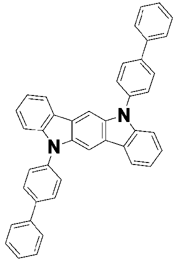 | 87% |
| 3-35 | 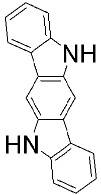 | 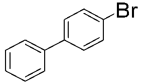 | 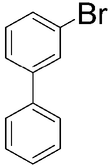 | 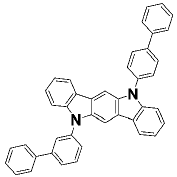 | 91% |
| 3-41 | 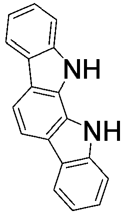 | 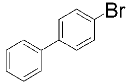 | 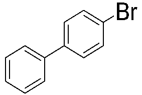 | 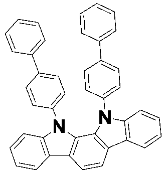 | 86% |
| 3-61 | 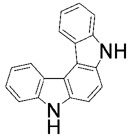 | 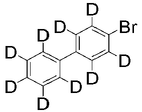 | 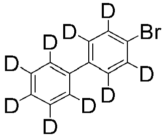 | 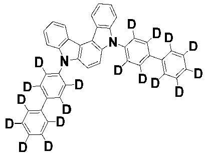 | 92% |
| 3-69 | 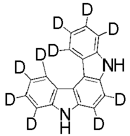 | 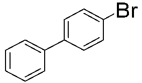 | 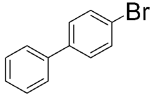 | 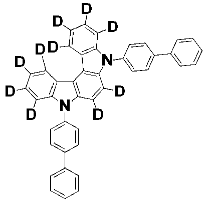 | 91% |
| 3-75 | 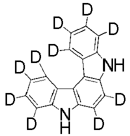 | 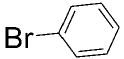 | 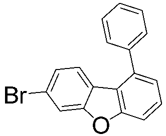 | 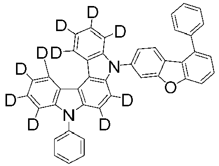 | 90% |
| 3-77 | 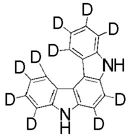 | 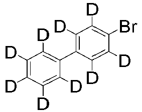 | 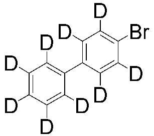 | 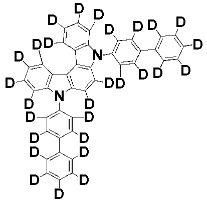 | 90% |
| 3-78 | 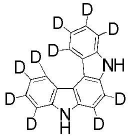 | 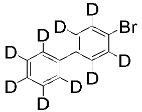 | 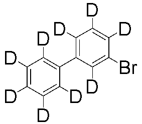 | 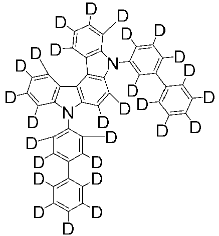 | 91% |
| 3-91 | 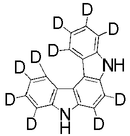 | 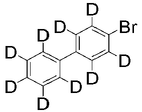 | 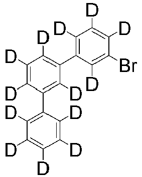 | 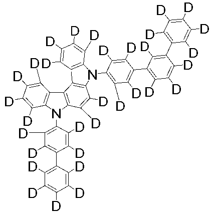 | 92% |

The synthesis results of the compounds described in Preparation Examples 1 to 7 and Tables 1 to 6 are shown in Tables 7 and 8 below.

Table 7 shows the measured values of ¹H NMR (CDCl₃, 400 MHz), and Table 8 shows the measured values of FD mass spectrometry (FD-MS: Field Desorption Mass Spectrometry).

**[Table 7]**

| Compound No. | ¹H NMR(CDCl₃, 400MHz) |
|---|---|
| 1-1 | δ= 8.55 (1H, d), 8.36 (2H, d), 8.25~8.19 (4H, m), 8.08~7.92 (4H, m), 7.80 (1H, t), 7.52~7.18 (16H, m) |
| 1-2 | δ= 8.55 (1H, d), 8.36 (2H, m), 8.25~8.19 (4H, m), 7.98~7.92 (2H, m), 7.83~7.80 (2H, m), 7.69~7.63 (2H, m), 7.52~7.18 (14H, m) |
| 1-5 | δ= 8.55 (1H, d), 8.36 (2H, m), 8.25~8.19 (4H, m), 8.08~7.92 (4H, m), 7.80 (1H, t), 7.52~7.18 (16H, m) |
| 1-7 | δ= 8.55 (1H, d), 8.36~8.16 (7H, m), 8.06 (1H, d), 7.92 (1H, d), 7.80 (1H, t), 7.62~7.50 (11H, m), 7.40 (1H, d), 7.29~7.11 (9H, m) |
| 1-14 | δ= 8.55 (1H, d), 8.36~8.17 (9H, m), 7.98~7.92 (3H, m), 7.80 (1H, t), 7.62~7.50 (19H, m) |
| 1-16 | δ= 8.55 (1H, d), 8.36 (2H, d), 8.25~8.19 (4H, m), 7.92~7.71 (8H, m), 7.62~7.41 (16H, m), 7.25~7.11 (6H, m) |
| 1-18 | δ= 8.65 (1H, d), 8.55 (1H, d), 8.42~8.36 (3H, m), 8.25~8.19 (4H, m), 7.98~7.92 (2H, m), 7.80 (1H, t), 7.62~7.11 (23H, m), |
| 1-25 | δ= 8.55 (2H, m), 8.45~8.17 (7H, m), 7.94~7.75 (7H, m), 7.52~7.25 (16H, m) |
| 1-26 | δ= 8.55 (1H, d), 8.45~8.17 (5H, m), 8.02~7.92 (4H, m), 7.89~7.79 (3H, m), 7.63 (2H, m), 7.52~7.20 (16H, m) |
| 1-31 | δ= 8.55 (2H, m), 8.45 (1H, d), 8.32~8.19 (5H, m), 7.92~7.80 (5H, m), 7.65 (1H, d), 7.52~7.20 (16H, m) |
| 1-35 | δ= 8.55 (4H, m), 8.45 (1H, d), 8.32~8.19 (5H, m), 7.92~7.80 (5H, m), 7.52~7.20 (16H, m) |
| 1-39 | δ= 8.55 (1H, d), 8.25~8.19 (8H, m), 8.08~7.92 (4H, m), 7.80~7.75 (3H, m), 7.63 (2H, m), 7.54~7.11 (18H, m) |
| 1-47 | δ= 8.55 (2H, m), 8.45 (2H, d), 8.32~8.19 (5H, m), 7.92~7.80 (5H, m), 7.56~7.49 (8H, m), 7.32~7.20 (7H, m) |
| 1-53 | δ= 8.55 (1H, d), 8.38~8.39 (3H, m), 8.20 (2H, m), 8.08~7.92 (4H, m), 7.80~7.71 (2H, m), 7.61~7.31 (11H, m), 7.20~7.11 (4H, m) |
| 1-54 | δ= 8.55 (1H, d), 8.36 (2H, m), 8.25~8.19 (4H, m), 8.08~7.92 (4H, m), 7.80 (1H, t), 7.52~7.39 (10H, m), 7.32~7.18 (16H, m) |
| 1-57 | δ= 8.55 (2H, m), 8.45~8.36 (4H, m), 8.20~8.19 (2H, m), 7.94~7.86 (5H, m), 7.56~7.49 (8H, m), 7.40 (1H, d), 7.31 (1H, t), 7.20~7.18 (4H, m) |
| 1-60 | δ= 8.55 (1H, d), 8.36~8.16 (7H, m), 8.06 (1H, d), 7.92 (1H, d), 7.80 (1H, t), 7.62~7.50 (11H, m), 7.40 (1H, d), 7.29~7.11 (9H, m) |
| 1-64 | δ= 8.55 (1H, d), 8.38~8.36 (3H, m), 8.20~8.19 (2H, m), 7.94~7.92 (3H, m), 7.80~7.41 (23H, m), 7.20~7.11 (4H, m) |
| 1-66 | δ= 8.55 (2H, m), 8.38~8.32 (2H, m), 8.20~8.17 (4H, m), 7.98~7.92 (4H, m), 7.80~7.71 (2H, m), 7.57~7.31 (14H, m), 7.20~7.11 (4H, m) |
| 1-72 | δ= 8.55 (2H, d), 8.46~8.38 (4H, m), 8.20 (3H, m), 8.00 (1H, s), 7.94~7.71 (6H, m), 7.56~7.40 (10H, m), 7.20~7.11 (8H, m) |
| 1-74 | δ= 8.55 (1H, d), 8.38~8.36 (3H, m), 8.20~8.19 (2H, m), 7.98~7.92 (4H, m), 7.80~7.71 (5H, m), 7.61~7.31 (13H, m), 7.20~7.11 (4H, m) |
| 1-94 | δ= 8.55 (1H, d), 8.36 (3H, m), 8.25~8.19 (5H, m), 7.94~7.92 (3H, m), 7.83 (1H, s), 7.71~7.49 (19H, m) |
| 1-97 | δ= 9.60 (1H, d), 9.27 (1H, s), 8.55 (2H, m), 8.45~8.32 (8H, m), 8.20 (1H, d), 7.92~7.31 (24H, m), 7.16~7.11 (2H, m) |
| 1-104 | δ= 8.55 (3H, m), 8.45~8.21 (8H, m), 7.94 (1H, s), 7.86 (1H, d), 7.71~7.50 (20H, m), 7.31 (1H, t), 7.16~7.08 (5H, m) |
| 1-107 | δ= 8.55 (1H, d), 8.38 (1H, d), 8.23~8.19 (3H, m), 7.98~7.92 (5H, m), 7.80~7.71 (4H, m), 7.55~7.31 (11H, m), 7.20~7.11 (4H, m) |
| 1-108 | δ= 8.55 (2H, m), 8.38 (1H, d), 8.23~8.19 (4H, m), 7.94~7.92 (4H, m), 7.80~7.40 (16H, m), 7.31 (1H, d), 7.20~7.11 (8H, m) |
| 1-109 | δ= 9.09 (1H, s), 8.55~8.50 (2H, m), 8.20~8.00 (11H, m), 7.80 (1H, t), 7.60~7.20 (15H, m) |
| 1-111 | δ= 8.97 (1H, d), 8.55 (1H, d), 8.25~8.20 (4H, m), 8.00~7.80 (7H, m), 7.69~7.63 (2H, m), 7.54~7.20 (14H, m) |
| 1-129 | δ= 9.08 (1H, d), 8.84 (1H, d), 8.55 (2H, d), 8.25~8.17 (6H, m), 8.05~7.90 (5H, m), 7.80~7.11 (32H, m) |
| 1-140 | δ= 8.90~8.89 (3H, m), 8.55 (2H, m), 8.32~8.19 (6H, m). 7.92~7.80 (6H, m), 7.70~7.11 (24H, m) |
| 1-152 | δ= 9.09 (1H, s), 8.55~8.45 (6H, m), 8.32~7.49 (26H, m), 7.31~7.11 (7H, m) |
| 1-161 | δ= 9.09 (1H, s), 8.55 (2H, m), 8.38 (1H, d), 8.20~7.92 (10H, m), 7.80~7.31 (12H, m), 7.20~7.18 (4H, m) |
| 1-165 | δ= 9.27 (1H, s), 8.79 (1H, d), 8.55 (2H, m), 8.45~8.30 (6H, m), 8.20~8.15 (3H, m), 7.70~7.49 (17H, m), 7.25~7.15 (4H, m) |
| 1-168 | δ= 9.27 (1H, s), 8.79 (1H, d), 8.55 (2H, m), 8.45~8.30 (4H, m), 8.20~8.16 (3H, m), 7.94~7.92 (2H, m), 7.82~7.40 (20H, m), 7.20~7.11 (6H, m) |
| 1-169 | δ= 8.97 (1H, d), 8.55 (1H, d), 8.38 (1H, d), 8.20 (2H, m), 8.00~7.80 (13H, m), 7.92~7.80 (12H, m), 7.52~7.31 (12H, m), 7.16~7.11 (4H, m) |
| 1-179 | δ= 8.97 (1H, d), 8.55 (2H, m), 8.38 (1H, d), 8.20~8.19 (3H, m), 8.00~7.40 (22H, m), 7.20~7.11 (8H, m) |
| 1-219 | δ= None (Deuteration 100%) |
| 1-220 | δ= 8.36 (2H, d), 8.25 (2H, d), 8.02~7.98 (3H, m), 7.64 (1H, s), 7.50~7.25 (10H, m) |
| 1-221 | δ= 8.55 (1H, d), 8.36 (2H, d), 8.20~8.19 (2H, m), 7.92~7.80 (3H, m), 7.58~7.40 (7H, m), 7.20~7.11 (4H, m) |
| 1-226 | δ= 8.55 (1H, d), 8.45~8.21 (8H, m), 7.94 (1H, s), 7.86 (1H, d), 7.71~7.47 (13H, m), 7.31 (1H, t) |
| 1-227 | δ= 8.76 (1H, s), 7.99 (1H, s), 7.78~7.77 (2H, s), 7.65~7.63 (3H, m), 7.52 (1H, d), 7.16 (1H, d) |
| 1-230 | δ= 8.55 (2H, m), 8.45~8.30 (7H, m), 8.19 (1H, d), 7.94 (1H, s), 7.86 (1H, d), 7.71~7.40 (12H, m), 7.19~7.10 (8H, m) |
| 1-231 | δ= 8.55 (2H, m), 8.45~8.36 (5H, m), 8.21~8.19 (2H, m), 8.02 (1H, d), 7.94 (1H, s), 7.86~7.76 (5H, m), 7.56~7.41 (12H, m), 7.20~7.11 (4H, m) |
| 2-1 | δ= 8.55 (1H, d), 8.30 (1H, d), 8.19~8.13 (2H, m), 7.99~7.89 (4H, m), 7.77 (1H, d), 7.62~7.50 (12H, m), 7.35(1H, t), 7.20~7.16 (2H, m) |
| 2-16 | δ= 9.05 (1H, s), 8.55 (1H, d), 8.33~8.13 (7H, m), 7.99~7.89 (5H, m), 7.77~7.50(13H, m), 7.35 (1H, t), 7.20~7.16 (2H, m) |
| 2-27 | δ= 8.55 (1H, m), 8.30 (1H, d), 8.21~8.13 (4H, m), 7.99~7.89 (4H, m), 7.77~7.35 (20H, m), 7.20~7.16 (2H, m) |
| 2-28 | δ= 8.55(1H, m), 8.18~8.09 (3H, m), 8.00~8.79 (2H, m), 7.87 (1H, m), 7.79~7.77 (4H, m), 7.69~7.63 (4H, m), 7.52~7.25 (12H, m) |
| 2-32 | δ= 8.55 (1H, m), 8.18~8.12 (2H, m), 8.00~7.87 (3H, m), 7.79~7.77 (6H, m), 7.69~7.63 (6H, m), 7.52~7.25 (14H, m) |
| 2-41 | δ= 8.55 (1H, m), 8.30 (1H, d), 8.19~8.13 (2H, m), 7.99~7.89(4H, m), 7.77(1H, d), 7.58~7.50(2H, m), 7.35 (1H, t), 7.20~7.16 (2H, m) |
| 2-61 | δ= 7.62~7.50 (10H, m) |
| 2-65 | δ= 7.79 (2H, m), 7.70~7.68 (3H, m), 7.58~7.41 (13H, m) |
| 2-68 | δ= 8.21 (2H, s), 7.75~7.60 (8H, m), 7.49~7.41 (8H, m) |
| 2-82 | δ= None (Deuteration 100%) |
| 2-88 | δ= None (Deuteration 100%) |
| 2-100 | δ= None (Deuteration 100%) |
| 3-1 | δ= 8.55 (2H, d), 7.62~7.50 (12H, m), 7.24 (2H, m), 7.16~7.11 (4H, m) |
| 3-4 | δ= 8.55 (2H, d), 7.94~7.91 (10H, m), 7.75 (4H, d), 7.49~7.35 (10H, m), 7.16 (2H, t) |
| 3-5 | δ= 8.55 (2H, d), 8.21 (1H, s), 7.94~7.91 (6H, m), 7.75~7.35 (16H, m), 7.26 (1H, d), 7.16 (2H, t) |
| 3-22 | δ= 8.55 (1H, d), 8.19 (1H, d), 7.94~7.91 (9H, m), 7.75 (4H, d), 7.58~7.35 (11H, m), 7.20~7.16 (2H, m) |
| 3-23 | δ= 8.55 (1H, d), 8.21~8.19 (2H, m), 7.94 7.91 (5H, m), 7.75~7.35 (18H, m), 7.20~7.16 (2H, m) |
| 3-32 | δ= 8.55 (1H, d), 8.19 (1H, d), 7.94~7.91 (9H, m), 7.75 (4H, d), 7.58~7.35 (11H, m), 7.20~7.16 (2H, m) |
| 3-35 | δ= 8.55 (1H, d), 8.21~8.19 (2H, m), 7.94~7.91 (5H, m), 7.68~7.35 (18H, m), 7.20~7.16 (2H, m) |
| 3-41 | δ= 8.55(2H, d), 7.94~7.91 (10H, m), 7.84 (2H, d), 7.75 (4H, d), 7.49~7.35 (8H, m), 7.16 (2H, t) |
| 3-61 | δ= 8.55 (2H, d), 7.94 (2H, d), 7.42~7.35 (4H, m), 7.16 (2H, t) |
| 3-69 | δ= 7.92~7.91 (8H, m), 7.75 (4H, d), 7.49~7.41 (6H, m) |
| 3-75 | δ= 7.79~7.73 (3H, m), 7.60~7.41 (12H, m), 7.25 (1H, d) |
| 3-77 | δ= None (Deuteration 100%) |
| 3-78 | δ= None (Deuteration 100%) |
| 3-91 | δ= None (Deuteration 100%) |

**[Table 8]**

| Compound No. | FD-MS | Compound No. | FD-MS |
|---|---|---|---|
| 1-1 | m/z= 640.23 (C₄₅H₂₈N₄₀= 640.75) | 1-2 | m/z= 640.23 (C₄₅H₂₈N₄O= 640.75) |
| 1-5 | m/z= 656.20 (C₄₅H₂₈N₄S= 656.81) | 1-7 | m/z= 715.27 (C₅₁H₃₃N₅= 715.86) |
| 1-14 | m/z= 822.25 (C₅₇H₃₄N₄OS= 822.99) | 1-16 | m/z= 791.30 (C₅₇H₃₇N₅= 791.96) |
| 1-18 | m/z= 805.28 (C₅₇H₃₅N₅O= 805.94) | 1-25 | m/z= 732.23 (C₅₁H₃₂N₄S= 732.91) |
| 1-26 | m/z= 732.23 (C₅₁H₃₂N₄S= 732.91) | 1-31 | m/z= 772.27 (C₅₄H₃₆N₄S= 772.97) |
| 1-35 | m/z= 896.30 (C₆₄H₄₀N₄S= 897.11) | 1-39 | m/z= 792.29 (C₅₇H₃₆N₄O= 792.94) |
| 1-47 | m/z= 762.19 (C₅₁H₃₀N₄S₂= 762.95) | 1-53 | m/z= 640.23 (C₄₅H₂₈N₄O = 640.75) |
| 1-54 | m/z= 640.23 (C₄₅H₂₈N₄O = 640.75) | 1-57 | m/z= 656.20 (C₄₅H₂₈N₄S= 656.81) |
| 1-60 | m/z= 715.27 (C₅₁H₃₃N₅= 715.86) | 1-64 | m/z= 792.29 (C₅₇H₃₆N₄O= 792.94) |
| 1-66 | m/z= 822.25 (C₅₇H₃₄N₄OS= 822.99) | 1-72 | m/z= 821.26 (C₅₇H₃₅N₅S= 822.00) |
| 1-74 | m/z= 716.26 (C₅₁H₃₂N₄O= 716.84) | 1-94 | m/z= 792.29 (C₅₇H₃₆N₄O= 792.94) |
| 1-97 | m/z= 882.28 (C₆₃H₃₈N₄S= 883.09) | 1-104 | m/z= 897.29 (C₆₃H₃₉N₅S= 898.10) |
| 1-107 | m/z= 639.23 (C₄₆H₂₉N₃₀= 639.76) | 1-108 | m/z= 804.29 (C₅₈H₃₆N₄O= 804.95) |
| 1-109 | m/z= 690.24 (C49H₃₀N₄O= 690.81) | 1-111 | m/z= 690.24 (C49H₃₀N₄O= 690.81) |
| 1-129 | m/z= 1057.38 (C₇₇H₄₇N₅₀= 1058.26) | 1-140 | m/z= 994.31 (C₇₂H₄₂N₄S= 995.22) |
| 1-152 | m/z= 964.27 (C₆₇H₄0N₄S₂= 965.21) | 1-161 | m/z= 690.24 (C49H₃₀N₄O= 690.81) |
| 1-165 | m/z= 806.25 (C₅₇H₃₄N₄S= 806.99) | 1-168 | m/z= 865.32 (C₆₃H₃₉N₅= 866.04) |
| 1-169 | m/z= 690.24 (C₄₉H₃₀N₄O= 690.81) | 1-179 | m/z= 855.30 (C₆₁H₃₇N₅O= 856.00) |
| 1-219 | m/z= 668.40 (C₄₅D₂₈N₄O= 668.92) | 1-220 | m/z= 650.29 (C₄₅H₁₈D₁₀N₄O= 650.81) |
| 1-221 | m/z= 649.28 (C₄₅H₁₉D₉N₄₀= 649.80) | 1-226 | m/z= 911.38 (C₆₃H₂₅D₁₄N₅S= 912.19) |
| 1-227 | m/z= 831.41 (C₅₇H₉D₂₅N₄S= 832.14) | 1-230 | m/z= 732.23 (C₅₁H₃₂N₄S= 732.91) |
| 1-231 | m/z= 732.23 (C₅₁H₃₂N₄S= 732.91) | | |
| 2-1 | m/z= 484.59 (C₃₆H₂₄N₂= 484.19) | 2-16 | m/z= 634.77 (C₄₈H₃₀N₂= 634.24) |
| 2-27 | m/z= 712.88 (C₅₄H₃₆N₂= 712.29) | 2-28 | m/z= 712.88 (C₅₄H₃₆N₂= 712.29) |
| 2-32 | m/z= 636.78 (C₄₈H₃₂N₂= 636.26) | 2-41 | m/z= 494.65 (C₃₆H₁₄D₁₀N₂= 494.26) |
| 2-61 | m/z= 498.68 (C₃₆H₁₀D₁₄N₂= 498.28) | 2-65 | m/z= 650.87 (C₄₈H₁₈D₁₄N₂= 650.34) |
| 2-68 | m/z= 650.87 (C₄₈H₁₈D₁₄N₂= 650.34) | 2-82 | m/z= 668.98 (C₄₈D₃₂N₂= 668.46) |
| 2-88 | m/z= 668.98 (C₄₈D₃₂N₂= 668.46) | 2-100 | m/z= 680.95 (C₄₈D₃₀N₂₀= 680.42) |
| 3-1 | m/z= 408.16 (C₃₀H₂₀N₂= 408.50) | 3-4 | m/z= 560.23 (C₄₂H₂₈N₂= 560.70) |
| 3-5 | m/z= 560.23 (C₄₂H₂₈N₂= 560.70) | 3-22 | m/z= 560.23 (C₄₂H₂₈N₂= 560.70) |
| 3-23 | m/z= 560.23 (C₄₂H₂₈N₂= 560.70) | 3-32 | m/z= 560.23 (C₄₂H₂₈N₂= 560.70) |
| 3-35 | m/z= 560.23 (C₄₂H₂₈N₂= 560.70) | 3-41 | m/z= 560.23 (C₄₂H₂₈N₂= 560.70) |
| 3-61 | m/z= 578.34 (C₄₂H₁₀D₁₈N₂= 578.81) | 3-69 | m/z= 570.29 (C₄₂H₁₈D₁₀N₂= 570.76) |
| 3-75 | m/z= 584.27 (C₄₂H₁₆D₁₀N₂₀= 584.74) | 3-77 | m/z= 588.40 (C₄₂D₂₈N₂= 588.87) |
| 3-78 | m/z= 588.40 (C₄₂D₂₈N₂= 588.87) | 3-91 | m/z= 668.46 (C₄₈D₃₂N₂= 668.99) |

### Experimental Example 1

### Experimental Example 1-1. Fabrication of Organic Light-Emitting Device

A glass substrate on which ITO had been thin-film coated to a thickness of 1,500 Å was ultrasonically cleaned with distilled water. After the distilled water cleaning, the substrate was ultrasonically cleaned with solvents such as acetone, methanol, and isopropyl alcohol, dried, and then subjected to UVO (ultraviolet ozone) treatment for 5 minutes using UV in a UV cleaner. Thereafter, the substrate was transferred to a plasma cleaner (PT), and plasma treatment was performed under vacuum to increase the work function of ITO and remove residual contaminants, followed by transfer to a thermal evaporation apparatus for organic deposition.

On the ITO transparent electrode (anode), a hole injection layer of 4,4',4"-Tris[2-naphthyl(phenyl)amino]triphenylamine (2-TNATA) having a thickness of 600 Å and a hole transport layer of N,N'-bis(α-naphthyl)-N,N'-diphenyl-4,4'-diamine (NPB) having a thickness of 300 Å were deposited.

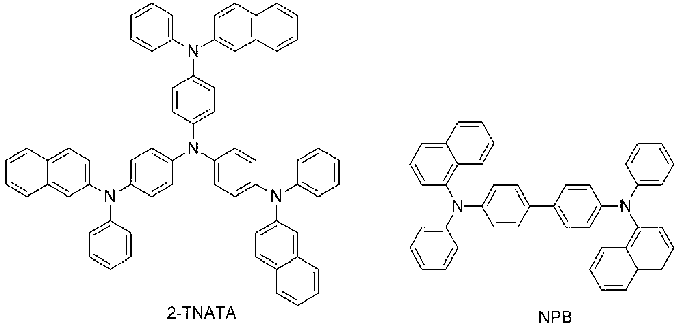

An light emmitting layer was then formed thereon by thermal vacuum deposition as follows. The light emmitting layer used the compounds listed in Table 9 as a host, and Ir(ppy)₃ (tris(2-phenylpyridine)iridium) as a green phosphorescent dopant. Ir(ppy)₃ was doped at 7% in the host, and the layer was deposited to a thickness of 360 Å.

Thereafter, BCP was deposited as a hole blocking layer to a thickness of 60 Å, and Alq₃ was deposited thereon as an electron transport layer to a thickness of 200 Å. Finally, lithium fluoride (LiF) was deposited on the electron transport layer to a thickness of 10 Å to form an electron injection layer, and aluminum (Al) was deposited on the electron injection layer to a thickness of 1,200 Å to form a cathode, thereby fabricating an organic electroluminescent device.

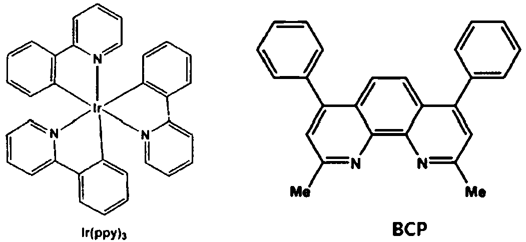

Meanwhile, all organic compounds required for fabricating the OLED device were purified by vacuum sublimation for each material under a pressure of 10⁻⁶ to 10⁻⁸ torr and then used for OLED (Organic Light Emitting Device) fabrication.

### Experimental Example 1-2. Driving Voltage and Luminous Efficiency of Organic Light-Emitting Device

For the organic light-emitting device fabricated as described above, electroluminescence (EL) characteristics were measured using an M7000 (manufactured by McScience). Based on the measurement results, T90 was measured using a lifetime measurement system (M6000, manufactured by McScience) at a reference luminance of 6,000 cd/m². The driving voltage, luminous efficiency, color coordinates (CIE), and lifetime of the organic light-emitting device according to the present invention are shown in Table 9.

The T90 refers to the lifetime (unit: hours) at which the luminance decreases to 90% of the initial luminance.

**[Table 9]**

| | Emission layer compound | Driving voltage (V) | Efficiency (cd/A) | Lifetime (T₉₀) |
|---|---|---|---|---|
| Example 1 | 1-1 | 4.40 | 67.9 | 145 |
| Example 2 | 1-2 | 4.33 | 66.8 | 138 |
| Example 3 | 1-5 | 4.42 | 67.7 | 148 |
| Example 4 | 1-7 | 4.44 | 67.6 | 140 |
| Example 5 | 1-14 | 4.45 | 67.3 | 142 |
| Example 6 | 1-16 | 4.29 | 65.7 | 135 |
| Example 7 | 1-18 | 4.42 | 67.4 | 137 |
| Example 8 | 1-25 | 4.44 | 67.9 | 150 |
| Example 9 | 1-26 | 4.42 | 68.4 | 138 |
| Example 10 | 1-31 | 4.49 | 68.2 | 147 |
| Example 11 | 1-35 | 4.40 | 67.2 | 149 |
| Example 12 | 1-39 | 4.37 | 66.9 | 142 |
| Example 13 | 1-47 | 4.32 | 65.1 | 151 |
| Example 14 | 1-53 | 4.50 | 69.2 | 175 |
| Example 15 | 1-54 | 4.44 | 68.6 | 150 |
| Example 16 | 1-57 | 4.52 | 69.0 | 179 |
| Example 17 | 1-60 | 4.43 | 68.0 | 170 |
| Example 18 | 1-64 | 4.54 | 69.3 | 183 |
| Example 19 | 1-66 | 4.53 | 69.2 | 178 |
| Example 20 | 1-72 | 4.54 | 69.1 | 182 |
| Example 21 | 1-74 | 4.52 | 69.4 | 168 |
| Example 22 | 1-94 | 4.49 | 68.8 | 185 |
| Example 23 | 1-97 | 4.59 | 69.3 | 178 |
| Example 24 | 1-104 | 4.50 | 68.0 | 177 |
| Example 25 | 1-107 | 4.62 | 67.2 | 172 |
| Example 26 | 1-108 | 4.65 | 67.7 | 173 |
| Example 27 | 1-109 | 4.33 | 65.0 | 143 |
| Example 28 | 1-111 | 4.32 | 64.8 | 142 |
| Example 29 | 1-129 | 4.54 | 63.2 | 140 |
| Example 30 | 1-140 | 4.47 | 64.4 | 144 |
| Example 31 | 1-152 | 4.37 | 65.4 | 146 |
| Example 32 | 1-161 | 4.48 | 65.2 | 177 |
| Example 33 | 1-165 | 4.49 | 65.7 | 180 |
| Example 34 | 1-168 | 4.44 | 65.3 | 176 |
| Example 35 | 1-169 | 4.52 | 65.6 | 182 |
| Example 36 | 1-179 | 4.47 | 64.8 | 170 |
| Example 37 | 1-219 | 4.39 | 68.0 | 163 |
| Example 38 | 1-220 | 4.41 | 68.1 | 160 |
| Example 39 | 1-221 | 4.37 | 67.6 | 142 |
| Example 40 | 1-226 | 4.51 | 68.3 | 184 |
| Example 41 | 1-227 | 4.51 | 65.9 | 192 |
| Example 42 | 1-230 | 4.62 | 69.8 | 165 |
| Example 43 | 1-231 | 4.59 | 69.5 | 162 |
| Comparative Example 1 | A | 4.66 | 53.2 | 50 |
| Comparative Example 2 | B | 4.73 | 58.1 | 56 |
| Comparative Example 3 | C | 4.68 | 53.6 | 68 |
| Comparative Example 4 | D | 5.20 | 56.0 | 85 |
| Comparative Example 5 | E | 4.73 | 59.2 | 57 |
| Comparative Example 6 | F | 5.19 | 56.2 | 72 |
| Comparative Example 7 | G | 5.04 | 55.2 | 62 |

### [Comparative Example Compound]

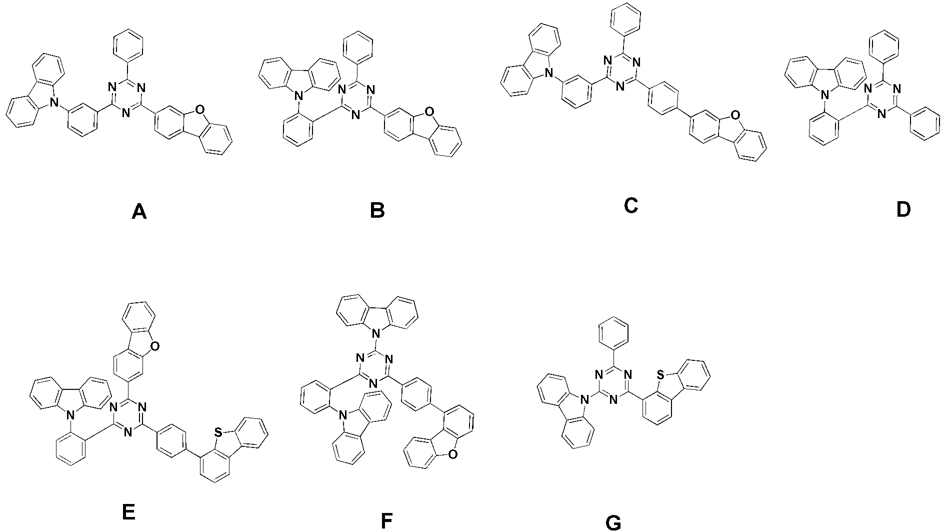

As OLED panels are being applied in various fields, the structures and configurations of devices are becoming increasingly diverse. Accordingly, the concept of host materials is also evolving. The heterocyclic compound represented by Chemical Formula 1 of the present invention is characterized as an n-type host having weak electron transport (ET) properties. Therefore, in the organic light-emitting device, when the LUMO is reduced, a significant improvement in efficiency and lifetime can be observed. Furthermore, such a host exhibits a characteristic that makes it very easy to achieve charge balance within the emission layer when combined with a p-type host having weak hole transport (HT) properties. This is because the variation in ratio between the p-type and n-type hosts is not large. In particular, in a multi-stack structure rather than a single-stack structure, it is more difficult to achieve charge balance within the device due to the nature of the OLED process, which requires precise thickness control and stacking of multiple layers by vacuum deposition. Therefore, the structure of the heterocyclic compound represented by Chemical Formula 1 of the present invention can provide a significant advantage.

In Comparative Example 1, Compound A has a structure in which triazine and carbazole are connected via a meta-phenylene linkage, and dibenzofuran is directly bonded to the triazine. Unlike Compound 1-2 of Example 2, which is connected via an ortho-phenylene linkage, Comparative Example 1 with a meta-phenylene linkage shows reduced efficiency and lifetime compared to Example 2. The LUMO is primarily formed in triazine, which has strong electron transport (ET) properties. When connected in an ortho configuration, as in the heterocyclic compound represented by Chemical Formula 1 of the present invention, the LUMO is relatively more reduced compared to para or meta linkage forms, and the separation from the HOMO formed on the carbazole moiety becomes the largest. Accordingly, in the ortho-linked structure of the heterocyclic compound represented by Chemical Formula 1 of the present invention, the HOMO and LUMO have the shallowest energy levels, and the energy gap (Eg) also has the largest value compared to meta- or para-linked structures. In addition, the dihedral angle is the largest, and accordingly, the T1 value is also the highest, resulting in the most excellent exciton confinement capability. This is because, in order to prevent triplet excitons transferred from the host to the dopant from leaking back to the host, the host must have a higher T1 value than the dopant, and the higher this value, the better the exciton confinement capability. In particular, for phosphorescent green or blue dopants having high T1 values, this characteristic becomes even more important. As a result, compared to meta- or para-phenylene linkages, the ortho-phenylene linkage provides advantages of improved efficiency and lifetime.

This can also be confirmed through the Gaussian calculations shown in Table 10 below. Even when the substituent directly connected to the triazine is changed, it can be seen that the T1 value is significantly higher in the case of an ortho linkage than in para or meta linkages.

**[Table 10]**

| Linkage position | Compound | HOMO | LUMO | T1 |
|---|---|---|---|---|
| Met a | 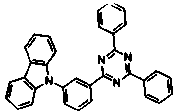 | -5.71 | -2.36 | 2.65 |
| Ort ho | 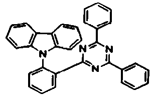 | -5.66 | -2.27 | 2.69 |
| Par a | 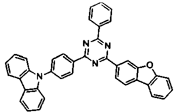 | -5.75 | -2.42 | 2.62 |
| Met a | 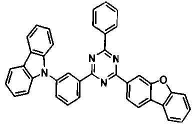 | -5.70 | -2.44 | 2.66 |
| Ort ho | 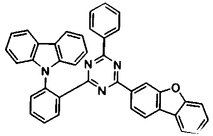 | -5.66 | -2.33 | 2.69 |
| Par a | 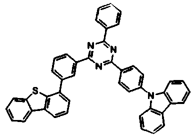 | -5.76 | -2.34 | 2.66 |
| Met a | 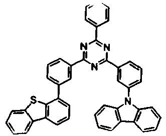 | -5.72 | -2.37 | 2.69 |
| Ort ho | 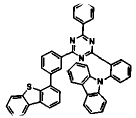 | -5.67 | -2.20 | 2.80 |

Compound B of Comparative Example 2 has a structure in which triazine is connected via an ortho-phenylene and dibenzofuran is directly bonded. Compared with Compound 1-2 of Example 2, in which triazine and dibenzofuran are connected through a phenylene linker, Compound B lacks a phenylene spacer between triazine and dibenzofuran, resulting in increased charge overlap between the triazine and dibenzofuran moieties. When the HOMO and LUMO are strongly overlapped, charge transfer (CT) within the molecule is less efficient than when they are spatially separated to an appropriate extent. The optimal degree of separation varies depending on the molecular structure, and when the intramolecular charge transfer distance is properly controlled, improvements in efficiency and lifetime can be achieved. Due to these differences, it is considered that Compound B of Comparative Example 2 exhibits reduced efficiency and shorter lifetime.

Compound C of Comparative Example 3 is similar to the heterocyclic compound represented by Chemical Formula 1 of the present invention in that triazine and dibenzofuran are connected via a phenylene linker; however, it differs in that the linkage is meta-phenylene. Due to the meta-phenylene linkage between triazine and carbazole, Compound C is considered to have a lower T₁ value and a deeper LUMO level, resulting in decreased efficiency and reduced device lifetime.

Compound D of Comparative Example 4 does not include a heteroaryl group, unlike the heterocyclic compound represented by Chemical Formula 1 of the present invention, and instead contains only aryl groups. Comparative Example 4 exhibited a higher driving voltage and lower efficiency than the Example. This is considered to be due to the absence of the heteroaryl group, which weakens intermolecular π-π interactions and significantly slows down charge transport between molecules. As a result, an imbalance between holes and electrons occurs within the emission layer, leading to reduced exciton generation efficiency.

Meanwhile, when comparing Compound 1-5 of Example 3 with Compound E of Comparative Example 5, Compound E of Comparative Example 5 is connected at the 3-position of dibenzofuran as Ar1 instead of an aryl group, resulting in an expansion of the LUMO. Consequently, it was confirmed that the efficiency and lifetime are reduced.

Compound F of Comparative Example 6 has a structure in which Ar1 is a heteroaryl group, specifically carbazole, directly bonded. In Compound F, the nitrogen atom of carbazole is directly connected to triazine, resulting in an increased dihedral angle and a higher T₁ value. However, since the carbazole nitrogen (an sp³-like nitrogen contributing to hole transport (HT) characteristics) is directly bonded to triazine, which has strong electron transport (ET) characteristics, the hole transport property is significantly weakened. As a result, the HOMO level becomes excessively deep, leading to a relatively higher driving voltage. In addition, because a C-N bond, which is relatively weaker than a C-C bond, directly connects carbazole and triazine, the bond may be cleaved under continuous electrical stress during device operation due to its lower bond dissociation energy. Consequently, this can lead to an increase in driving voltage and a decrease in efficiency and device lifetime.

Compound G of Comparative Example 7 is considered to exhibit reduced efficiency and shortened lifetime due to excessively strong charge overlap between triazine and dibenzothiophene, which results in an overly short intramolecular charge transfer distance.

### Experimental Example 2

The organic light-emitting device was fabricated in the same manner as in Experimental Example 1-1, except that the emission layer was formed by pre-mixing one first host (a compound of Chemical Formula 1) and one second host (a compound of Chemical Formula 2 or Chemical Formula 3) listed in Table 11, followed by co-deposition from a single source.

### Experimental Example 2-2. Driving Voltage and Luminous Efficiency of Organic Light-Emitting Device

The electroluminescence (EL) characteristics of the organic light-emitting device fabricated as described above were measured using an M7000 (manufactured by McScience). Based on the measurement results, T₉₀ was measured using a lifetime measurement system (M6000, manufactured by McScience) at a reference luminance of 6,000 cd/m². The driving voltage, luminous efficiency, color coordinates (CIE), and lifetime of the organic light-emitting device according to the present invention are shown in Table 11.

T₉₀ refers to the lifetime (unit: hours) at which the luminance decreases to 90% of the initial luminance.

**[Table 11]**

| | Emission layer compound | ratio | Driving Voltage (V) | Efficiency (cd/A) | Lifetime (T₉₀) |
|---|---|---|---|---|---|
| Example 44 | 1-1:2-1 | 5:1 | 4.07 | 70.1 | 151 |
| Example 45 | 1-1:2-1 | 2:1 | 4.13 | 70.7 | 166 |
| Example 46 | 1-1:2-1 | 1:1 | 4.18 | 71.3 | 203 |
| Example 47 | 1-1:2-1 | 1:2 | 4.31 | 75.1 | 254 |
| Example 48 | 1-1:2-1 | 1:5 | 4.39 | 67.1 | 183 |
| Example 49 | 1-5:2-16 | 2:1 | 4.25 | 70.9 | 170 |
| Example 50 | 1-5:2-16 | 1:1 | 4.30 | 71.5 | 207 |
| Example 51 | 1-5:2-16 | 1:2 | 4.43 | 75.3 | 259 |
| Example 52 | 1-7:2-65 | 2:1 | 4.26 | 70.9 | 207 |
| Example 53 | 1-7:2-65 | 1:1 | 4.31 | 71.5 | 252 |
| Example 54 | 1-7:2-65 | 1:2 | 4.44 | 75.3 | 315 |
| Example 55 | 1-25:2-82 | 2:1 | 4.13 | 70.7 | 216 |
| Example 56 | 1-25:2-82 | 1:1 | 4.18 | 71.3 | 263 |
| Example 57 | 1-25:2-82 | 1:2 | 4.31 | 75.1 | 329 |
| Example 58 | 1-47:2-88 | 2:1 | 4.16 | 68.6 | 222 |
| Example 59 | 1-47:2-88 | 1:1 | 4.21 | 69.2 | 271 |
| Example 60 | 1-47:2-88 | 1:2 | 4.34 | 72.9 | 339 |
| Example 61 | 1-57:2-100 | 2:1 | 4.13 | 73.3 | 230 |
| Example 62 | 1-57:2-100 | 1:1 | 4.18 | 73.9 | 281 |
| Example 63 | 1-57:2-100 | 1:2 | 4.31 | 77.8 | 351 |
| Example 64 | 1-94:3-1 | 2:1 | 4.13 | 70.9 | 217 |
| Example 65 | 1-94:3-1 | 1:1 | 4.18 | 71.5 | 265 |
| Example 66 | 1-94:3-1 | 1:2 | 4.31 | 75.3 | 331 |
| Example 67 | 1-109:3-5 | 2:1 | 3.99 | 68.9 | 189 |
| Example 68 | 1-109:3-5 | 1:1 | 4.04 | 69.5 | 230 |
| Example 69 | 1-109:3-5 | 1:2 | 4.16 | 73.2 | 288 |
| Example 70 | 1-165:3-22 | 2:1 | 4.10 | 68.6 | 214 |
| Example 71 | 1-165:3-22 | 1:1 | 4.15 | 69.2 | 261 |
| Example 72 | 1-165:3-22 | 1:2 | 4.27 | 72.9 | 326 |
| Example 73 | 1-219:3-75 | 2:1 | 4.04 | 71.9 | 190 |
| Example 74 | 1-219:3-75 | 1:1 | 4.09 | 72.5 | 232 |
| Example 75 | 1-219:3-75 | 1:2 | 4.21 | 76.3 | 290 |
| Example 76 | 1-226:3- | 2:1 | 4.06 | 71.9 | 220 |
| | 78 | | | | |
| Example 77 | 1-226:3-78 | 1:1 | 4.11 | 72.5 | 268 |
| Example 78 | 1-226:3-78 | 1:2 | 4.23 | 76.3 | 335 |
| Example 79 | 1-230:3-91 | 2:1 | 4.30 | 72.0 | 198 |
| Example 80 | 1-230:3-91 | 1:1 | 4.35 | 72.6 | 241 |
| Example 81 | 1-230:3-91 | 1:2 | 4.48 | 76.4 | 301 |
| Comparative Example 8 | A: 2-1 | 2:1 | 4.55 | 62.5 | 75 |
| Comparative Example 9 | A: 2-1 | 1:1 | 4.61 | 63.8 | 91 |
| Comparative Example 10 | A: 2-1 | 1:2 | 4.67 | 64.3 | 114 |
| Comparative Example 11 | B:3-35 | 2:1 | 4.66 | 64.5 | 89 |
| Comparative Example 12 | B:3-35 | 1:1 | 4.73 | 65.9 | 102 |
| Comparative Example 13 | B:3-35 | 1:2 | 4.79 | 66.4 | 121 |
| Comparative Example 14 | D:3-1 | 2:1 | 5.10 | 62.2 | 102 |
| Comparative Example 15 | D:3-1 | 1:1 | 5.16 | 63.1 | 125 |
| Comparative Example 16 | D:3-1 | 1:2 | 5.21 | 63.8 | 146 |

### [Comparative Example Compound]

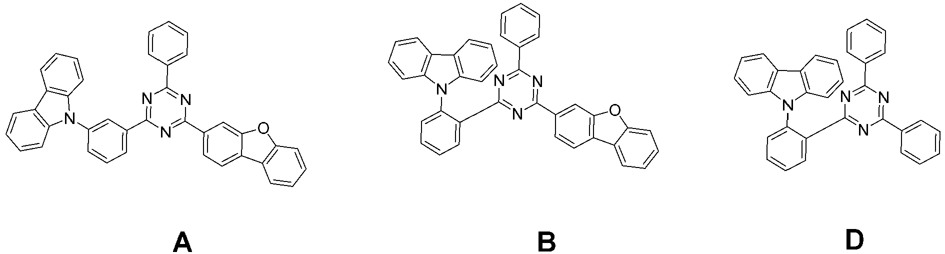

From the results shown in Table 11, it was confirmed that when the heterocyclic compound represented by Chemical Formula 1 of the present invention is used as an n-type host, and the heterocyclic compound represented by Chemical Formula 2 or Chemical Formula 3 is used as a p-type host, and these two hosts are mixed and deposited, the driving voltage, luminous efficiency, and lifetime of the organic light-emitting device are improved.

From this, it can be expected that an exciplex phenomenon occurs when the two compounds are mixed and deposited.

The exciplex phenomenon refers to the emission of energy corresponding to the HOMO energy level of a donor (P-type host) and the LUMO energy level of an acceptor (N-type host) through electron exchange between two molecules. When an exciplex is formed between the two molecules, reverse intersystem crossing (RISC) can occur, thereby allowing the internal quantum efficiency of fluorescence to increase up to 100%.
When a donor (P-type host) having good hole-transporting properties and an acceptor (N-type host) having good electron-transporting properties are used as hosts in the emission layer, holes are injected into the P-type host and electrons are injected into the N-type host. Accordingly, the driving voltage of the organic light-emitting device can be reduced, which in turn contributes to an improvement in device lifetime.

In addition, it can be confirmed that deuterium substitution leads to improved lifetime characteristics. This suggests that even compounds having similar structures may exhibit different properties depending on deuterium substitution. Since the bond dissociation energy of a carbon-deuterium single bond is higher than that of a carbon-hydrogen single bond, the thermal stability of the molecule is increased, resulting in improved device lifetime.

### <Explanation of Reference Numerals and Symbols>

- 100:: Substrate
- 200:: Anode
- 300:: Organic material layer
- 301:: Hole injection layer
- 302:: Hole transport layer
- 303:: Emission layer
- 304:: Hole blocking layer
- 305:: Electron transport layer
- 306:: Electron injection layer
- 400:: Cathode

## Claims

1. A heterocyclic compound represented by the following Chemical Formula 1: 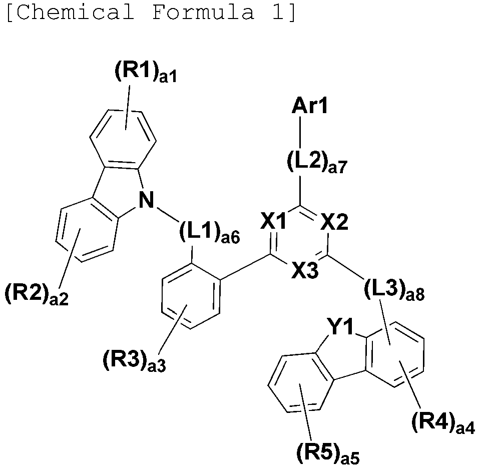 in Chemical Formula 1,
X1 is N; or CRa,
X2 is N; or CRb,
X3 is N; or CRc,
at least two of X1 to X3 are N,
Y1 is O; S; or NRd,
R1 to R5 and Ra to Rd are the same as or different from each other, and are each independently selected from the group consisting of hydrogen; deuterium; halogen; cyano group;
substituted or unsubstituted C1 to C60 alkyl group; substituted or unsubstituted C2 to C60 alkenyl group; substituted or unsubstituted C2 to C60 alkynyl group; substituted or unsubstituted C1 to C60 alkoxy group; substituted or unsubstituted C3 to C60 cycloalkyl group; substituted or unsubstituted C2 to C60 heterocycloalkyl group; substituted or unsubstituted C6 to C60 aryl group; substituted or unsubstituted C2 to C60 heteroaryl group; -P(=O)R101R102; -SiR101R102R103; and
-NR101R102, or two or more adjacent groups may be bonded to each other to form a substituted or unsubstituted C6 to C60 aromatic hydrocarbon ring; or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group.
a1 is an integer of 0 to 4, and when a1 is 2 or more, R1s are the same as or different from each other,
a2 is an integer of 0 to 4, and when a2 is 2 or more, R2s are the same as or different from each other,
a3 is an integer of 0 to 4, and when a3 is 2 or more, R3s are the same as or different from each other,
a4 is an integer of 0 to 3, and when a4 is 2 or more, R4s are the same as or different from each other,
a5 is an integer of 0 to 4, and when a5 is 2 or more, R5s are the same as or different from each other,
Ar1 is a substituted or unsubstituted C6 to C60 aryl group,
L1 and L2 are the same as or different from each other, and each independently a direct bond; a substituted or unsubstituted C6 to C60 arylene group; or a C2 to C60 heteroarylene group,
a6 is an integer of 0 to 5, and when a6 is 2 or more, L1s are the same as or different from each other,
a7 is an integer of 0 to 5, and when a7 is 2 or more, L2s are the same as or different from each other,
L3 is a substituted or unsubstituted C6 to C60 arylene group; or a C2 to C60 heteroarylene group,
a8 is an integer of 1 to 5, and when a8 is 2 or more, L3s are the same as or different from each other.

2. A heterocyclic compound according to claim 1,
wherein the heterocyclic compound represented by the Chemical Formula 1 is represented by the following Chemical Formula 1-1 to 1-4: 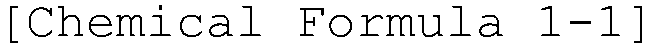 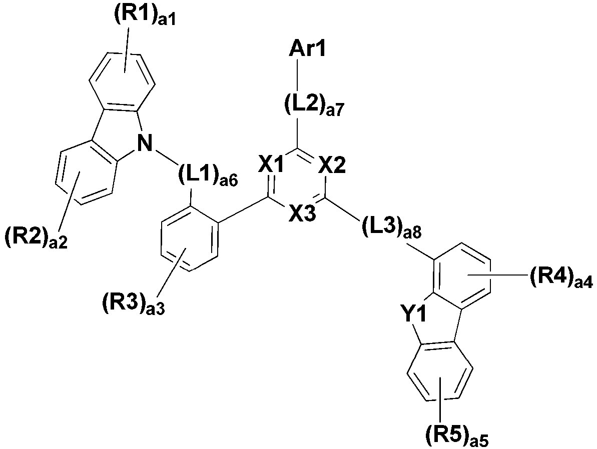 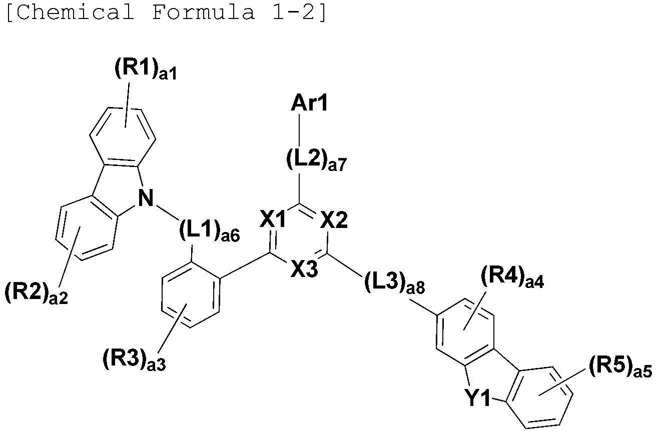 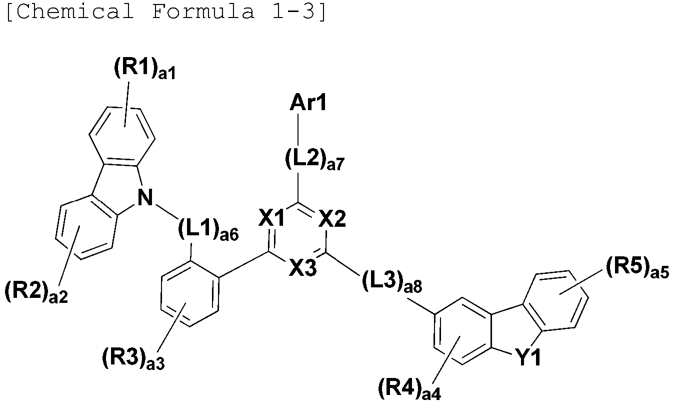 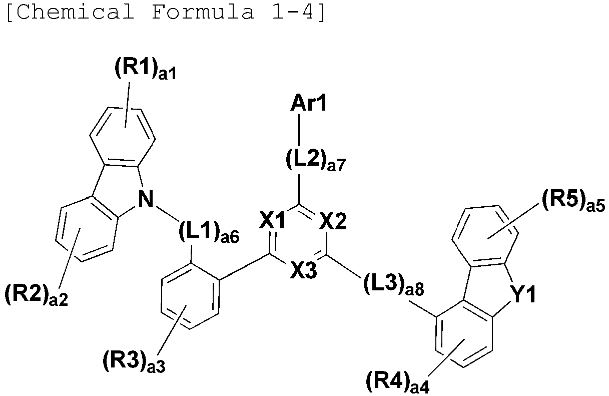
in Chemical Formulas 1-1 to 1-4,
X1 to X3, Y1, R1 to R5, L1 to L3, Ar1, and a1 to a8 are the same as defined in Chemical Formula 1.

3. A heterocyclic compound according to claim 1,
wherein the above 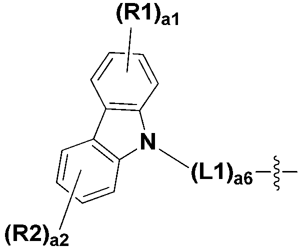 is represented by any one of Formulas A-1 to A-3 below: 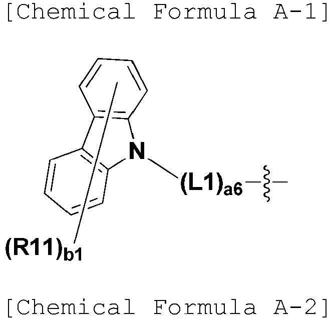 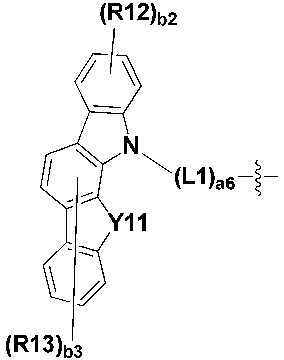 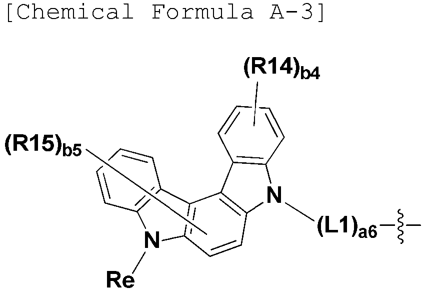
in Formulas A-1 to A-3,
Y11 is O; or S,
R11 to R15 and Re are the same as or different from each other and are each independently selected from the group consisting of hydrogen; deuterium; halogen; cyano; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, wherein R101, R102, and R103 may be the same as or different from each other and are each independently a substituted or unsubstituted C1 to C60 alkyl group; a substituted or unsubstituted C6 to C60 aryl group; or a substituted or unsubstituted C2 to C60 heteroaryl group,
b1 is an integer of 0 to 8, and when b1 is 2 or greater, R11 are the same as or different from each other,
b2 is an integer of 0 to 4, and when b2 is 2 or greater, R12 are the same as or different from each other,
b3 is an integer of 0 to 6, and when b3 is 2 or greater, R13 are the same as or different from each other,
b4 is an integer of 0 to 4, and when b4 is 2 or greater, R14 are the same as or different from each other,
b5 is an integer of 0 to 6, and when b5 is 2 or greater, R15 are the same as or different from each other, and
L1 and a6 are as defined in Chemical Formula 1.

4. A heterocyclic compound according to claim 1,
wherein the above 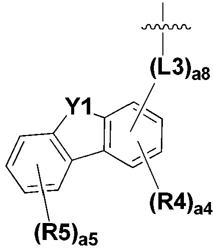 is represented by any one of Formulas B-1 to B-3 below: 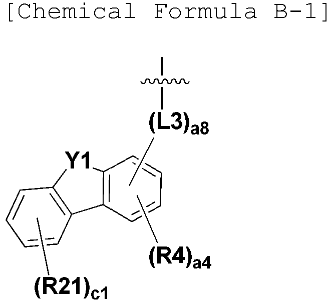 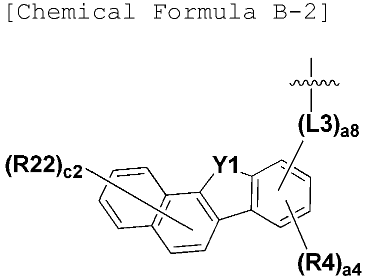 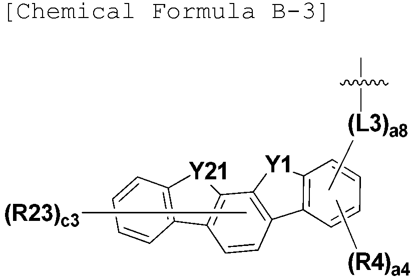
in Formulas B-1 to B-3,
Y21 is O; or NRf,
R21 to R23 and Rf may be the same as or different from each other and are each independently selected from the group consisting of hydrogen; deuterium; halogen; cyano; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, wherein R101, R102, and R103 may be the same as or different from each other and are each independently a substituted or unsubstituted C1 to C60 alkyl group; a substituted or unsubstituted C6 to C60 aryl group; or a substituted or unsubstituted C2 to C60 heteroaryl group,
c1 is an integer of 0 to 4, and when c1 is 2 or greater, R21 are the same as or different from each other,
c2 is an integer of 0 to 6, and when c2 is 2 or greater, R22 are the same as or different from each other,
c3 is an integer of 0 to 6, and when c3 is 2 or greater, R23 are the same as or different from each other, and
Y1, R4, L3, a4, and a8 are as defined in Chemical Formula 1.

5. A heterocyclic compound according to claim 1,
wherein the heterocyclic compound represented by Chemical Formula 1 does not include deuterium as a substituent, or the deuterium content relative to the total number of hydrogen atoms and deuterium atoms is 1% to 100%.

6. A heterocyclic compound according to claim 1,
wherein the heterocyclic compound represented by Chemical Formula 1 is represented by any one of the following compounds: s

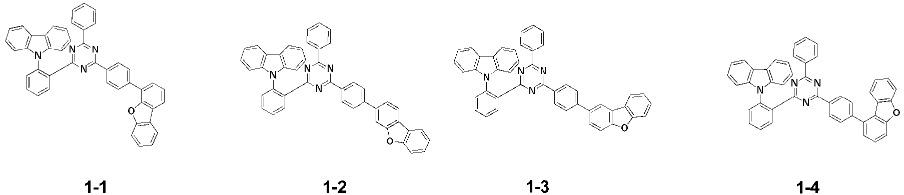

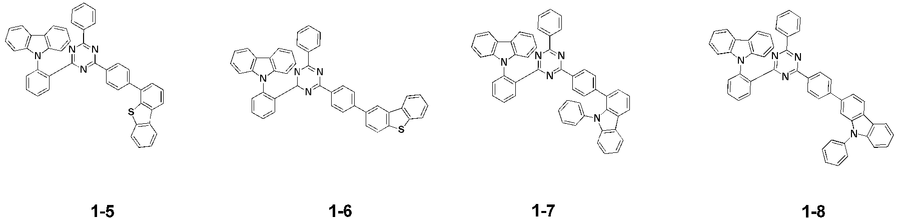

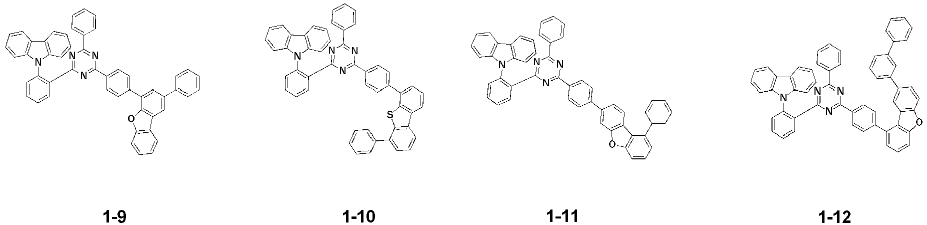

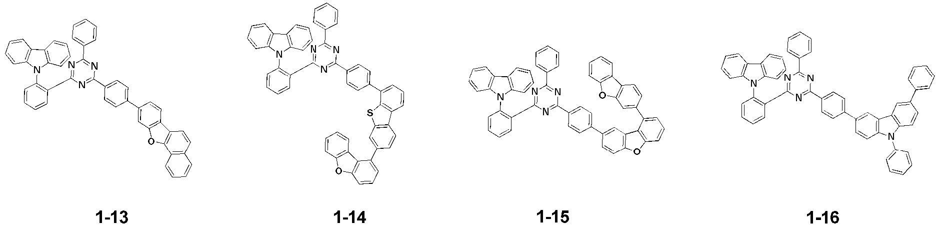

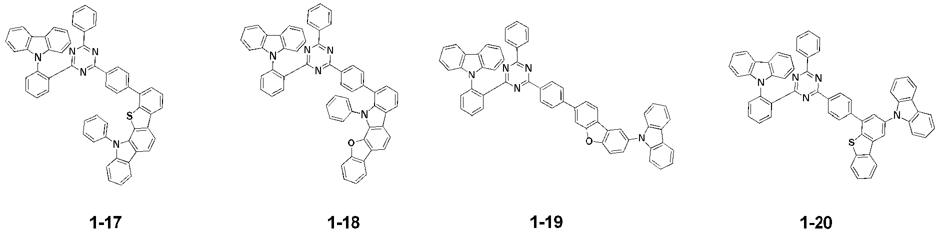

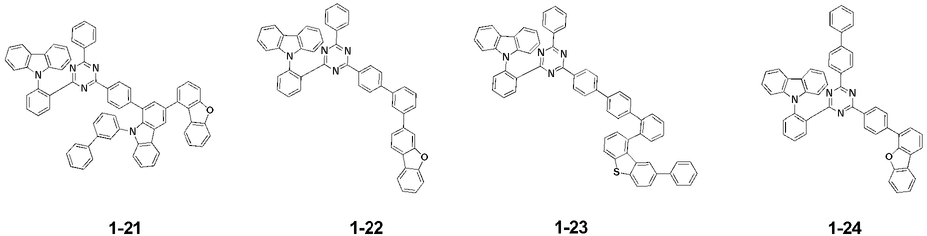

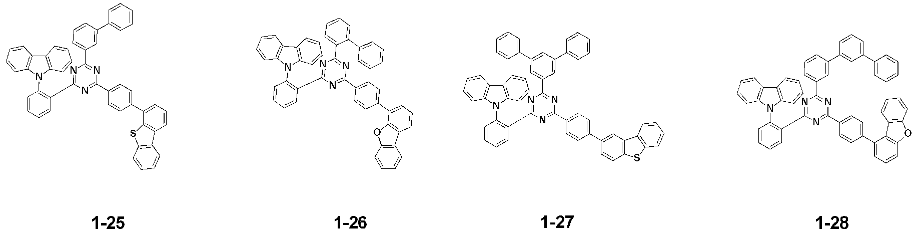

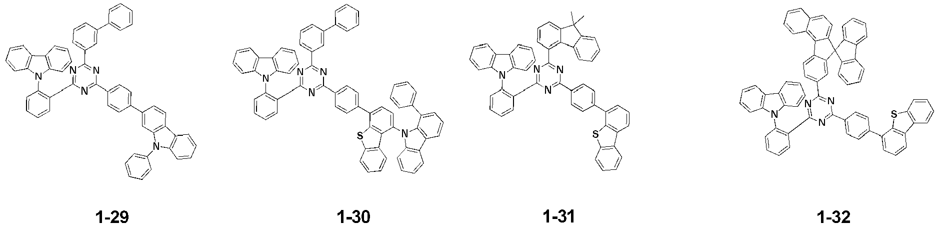

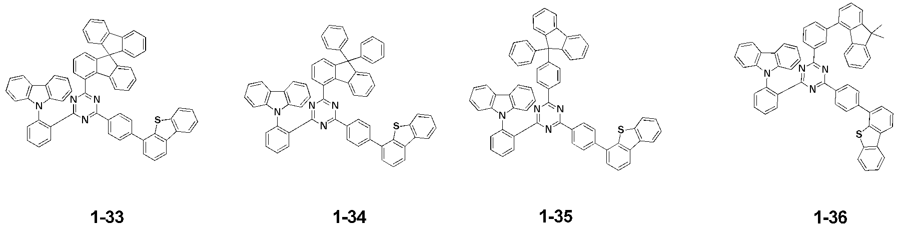

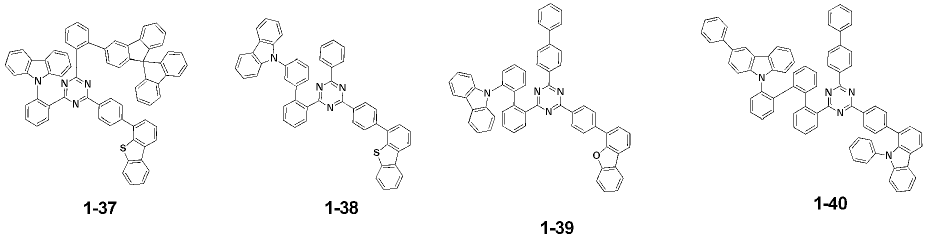

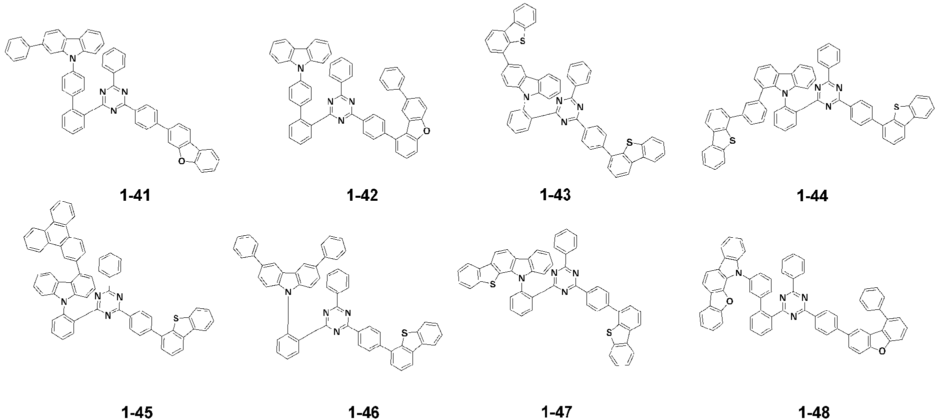

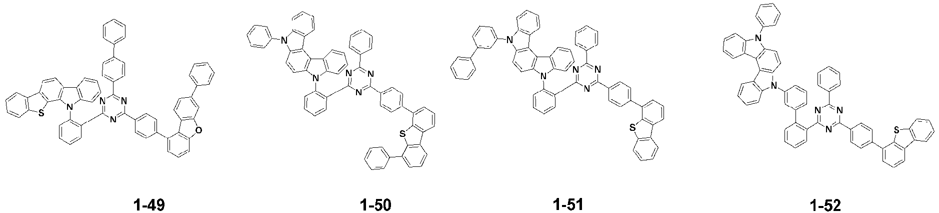

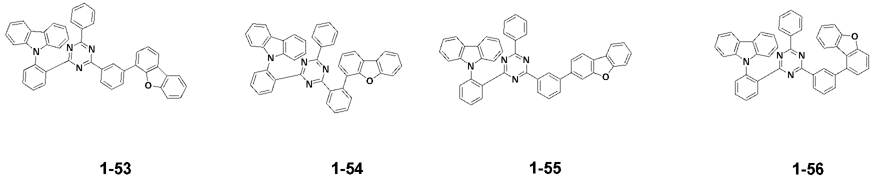

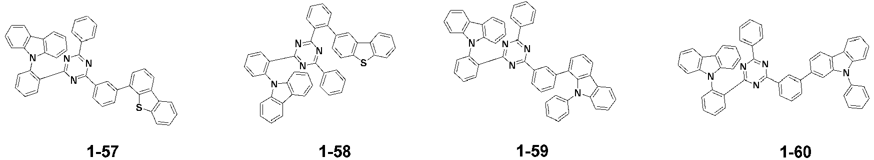

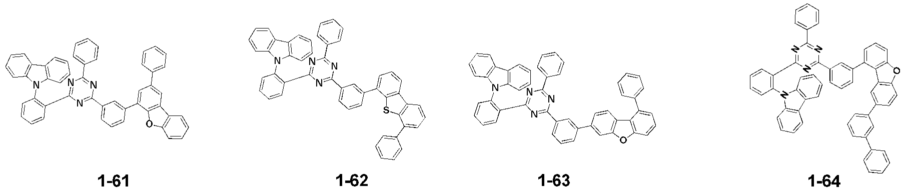

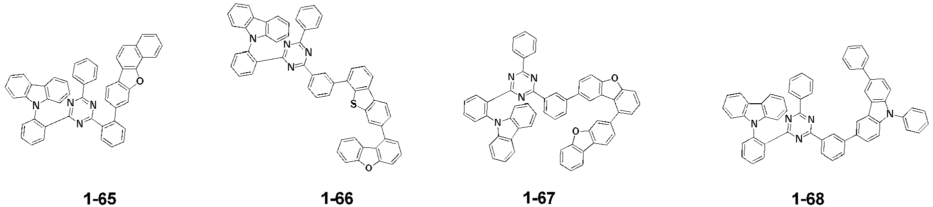

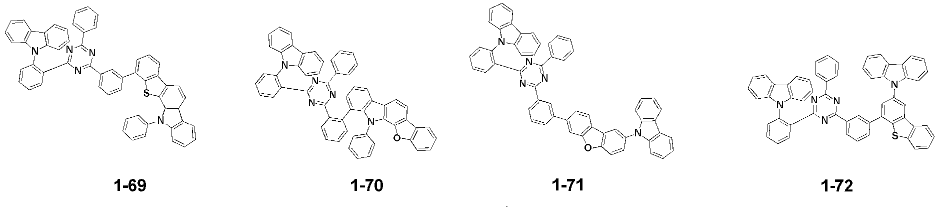

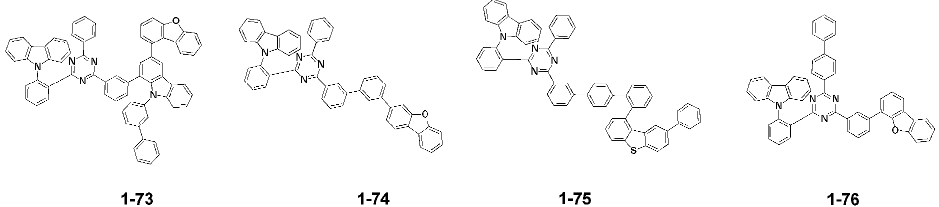

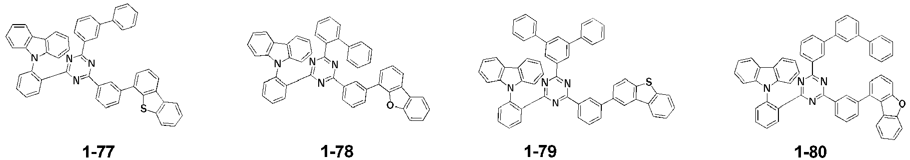

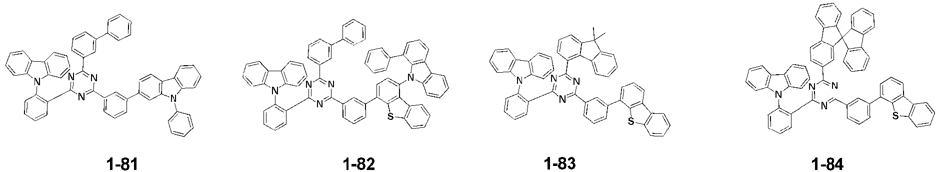

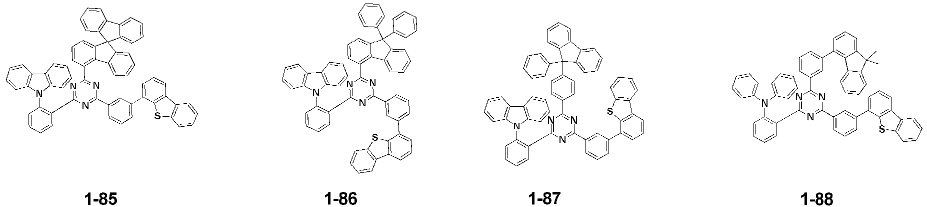

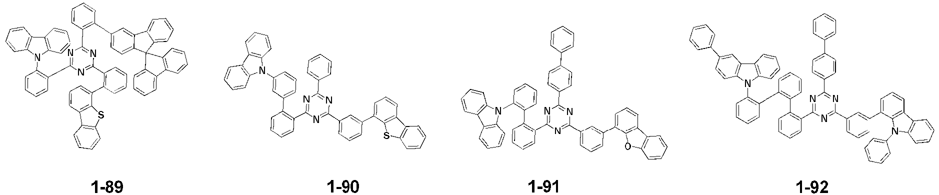

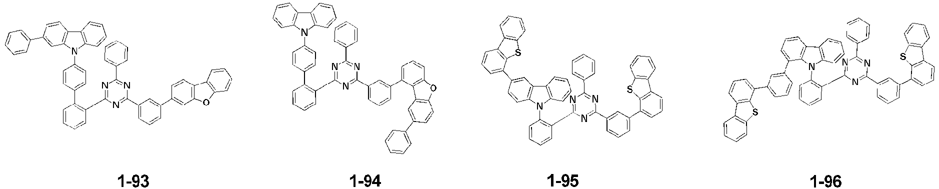

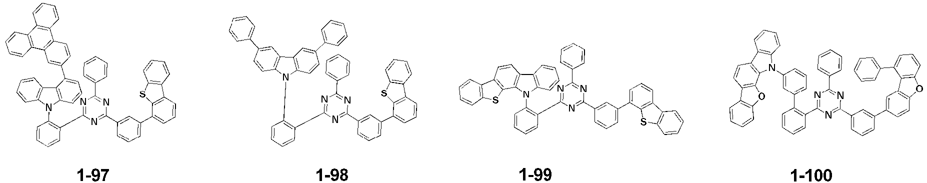

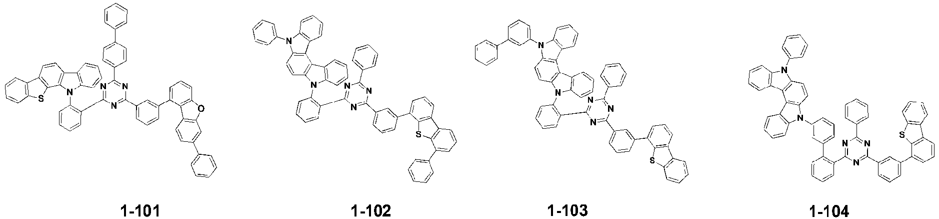

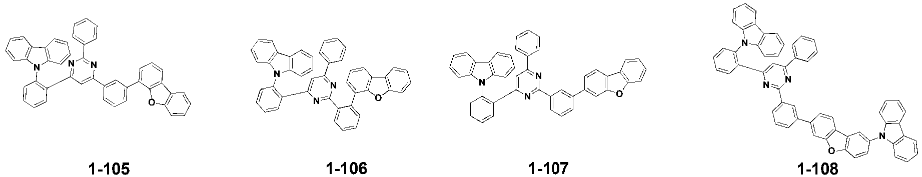

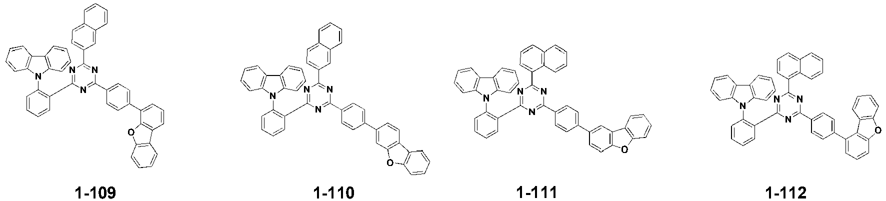

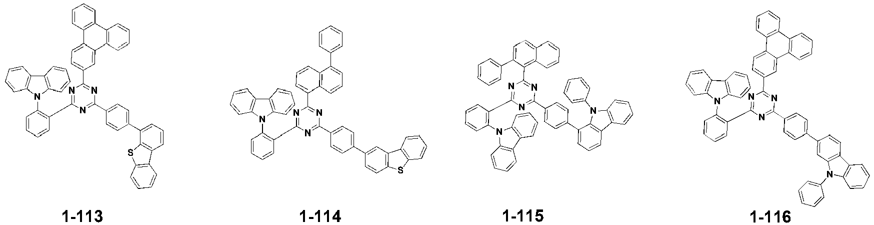

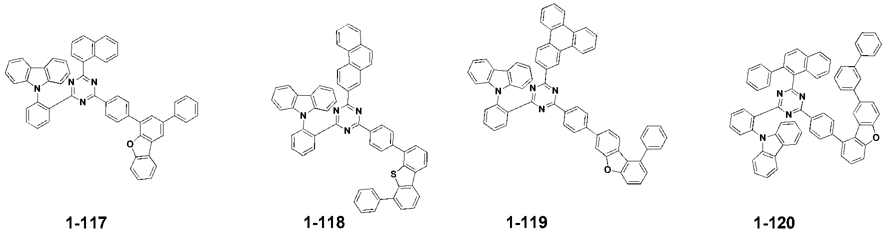

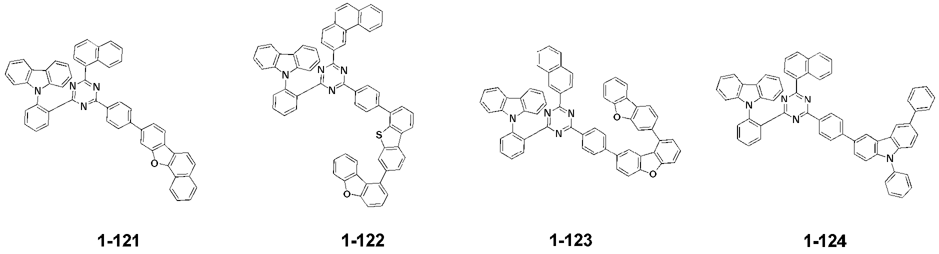

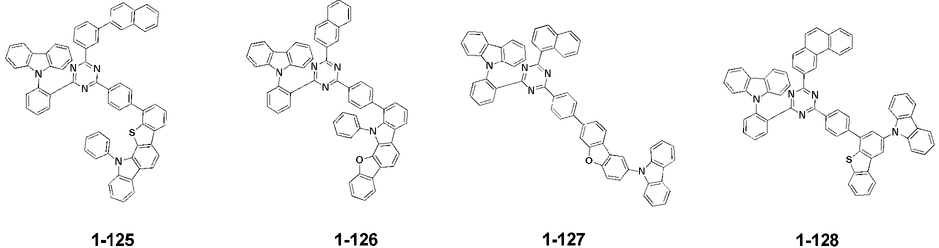

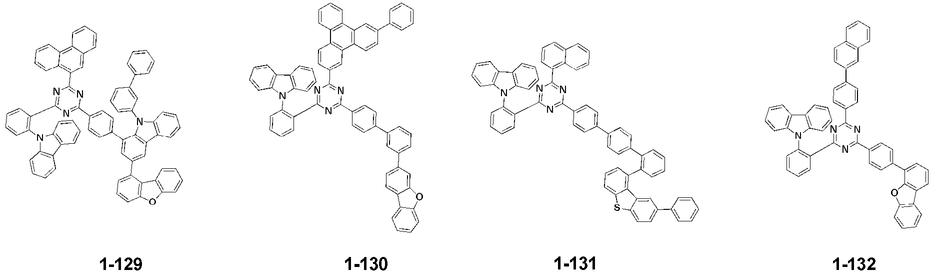

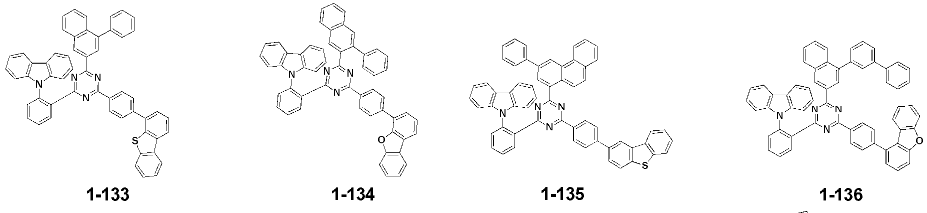

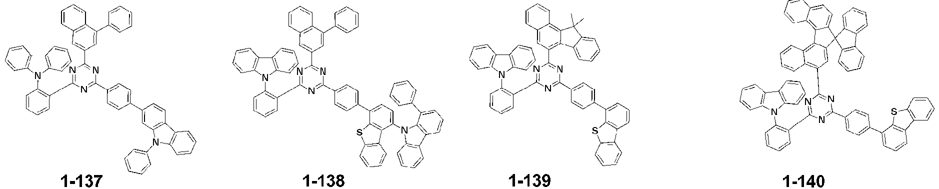

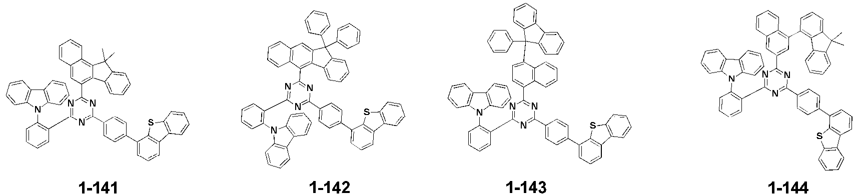

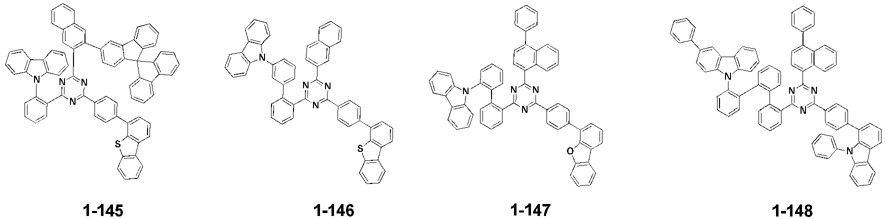

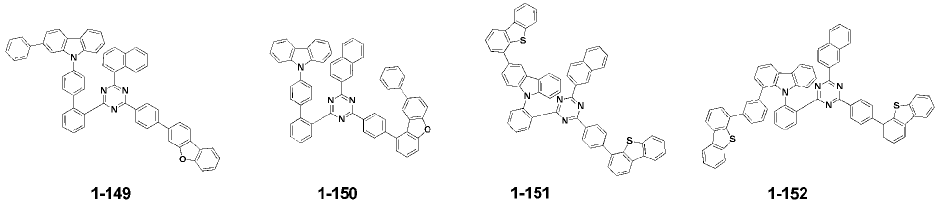

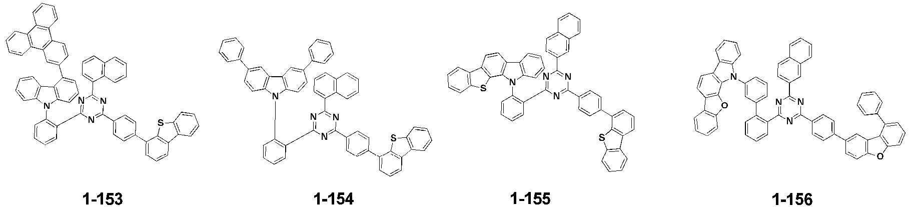

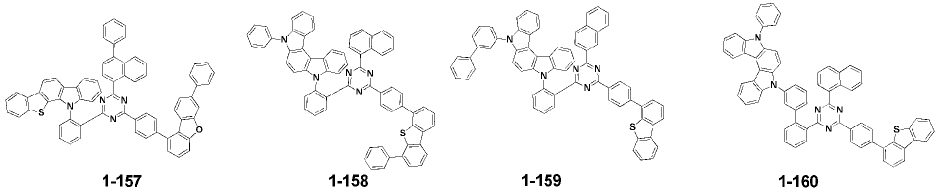

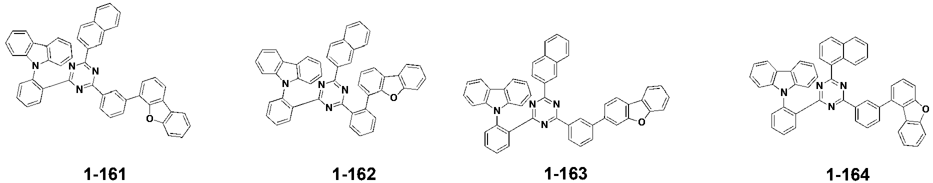

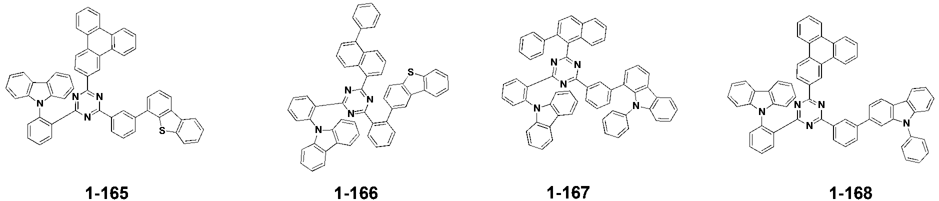

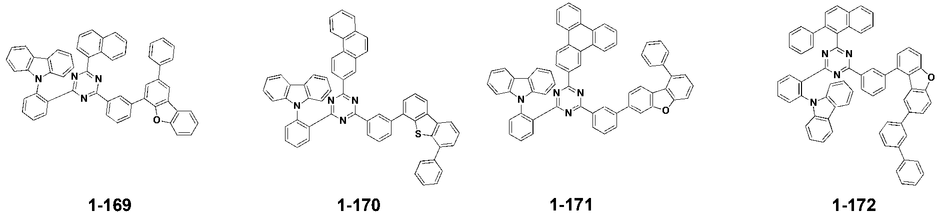

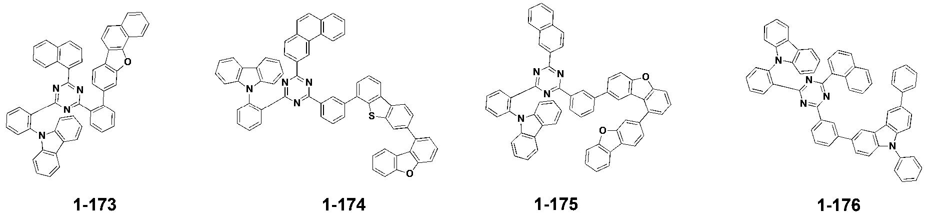

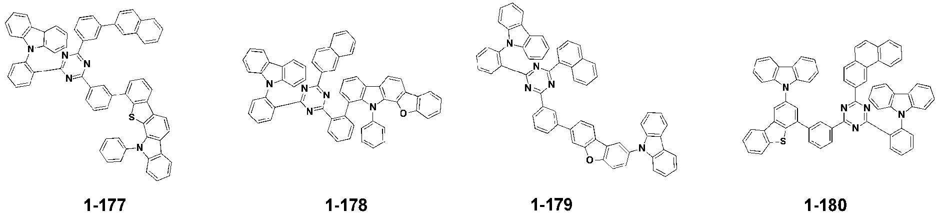

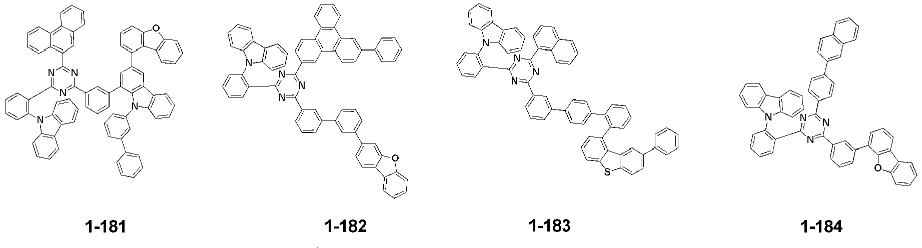

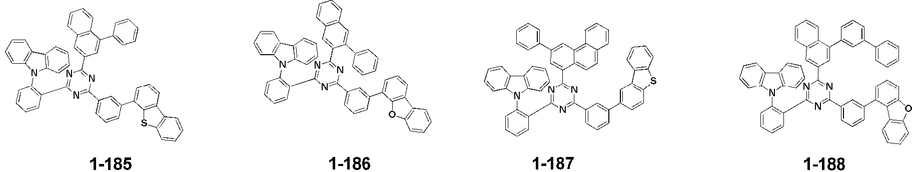

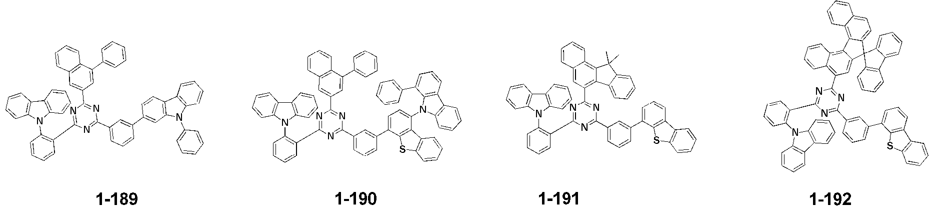

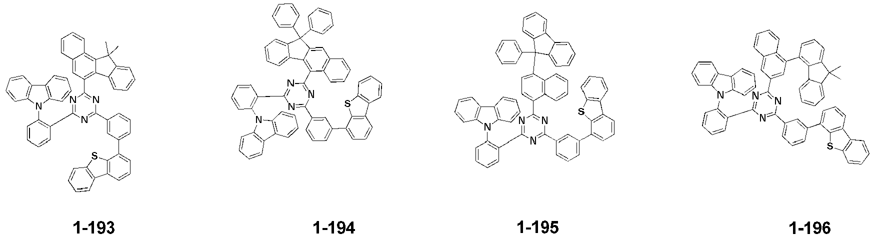

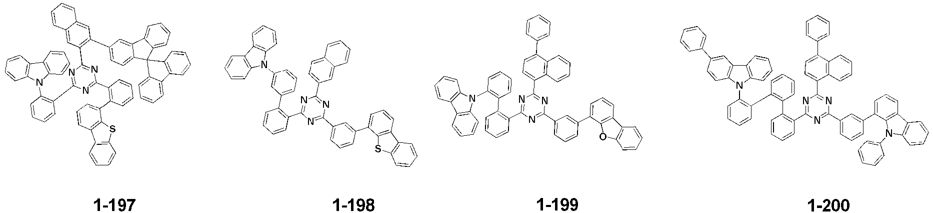

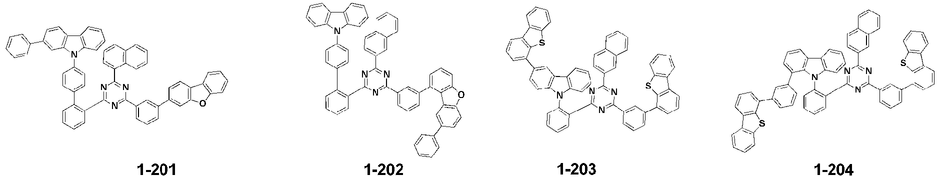

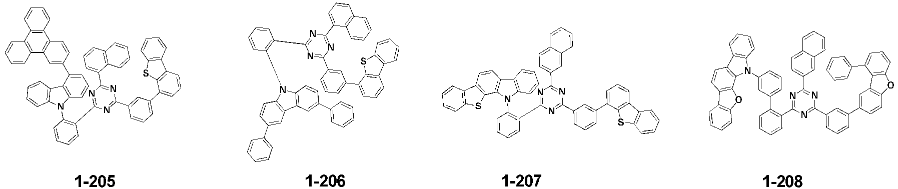

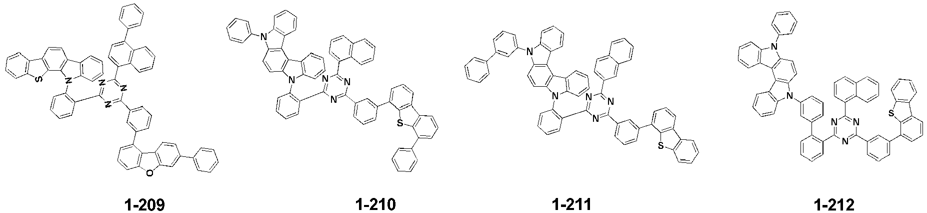

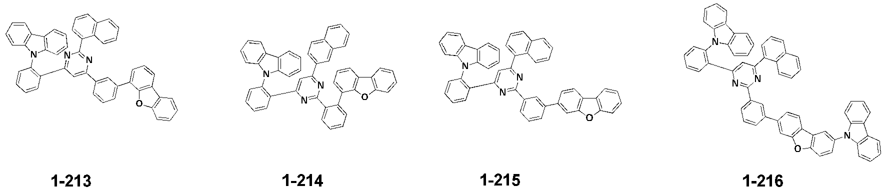

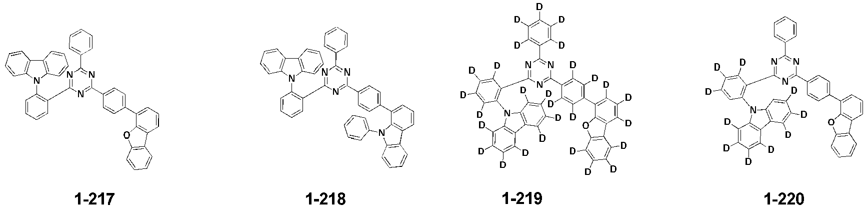

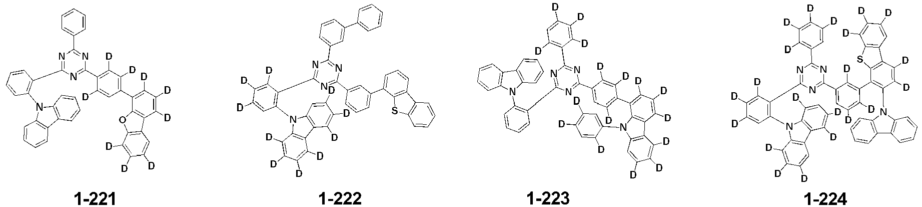

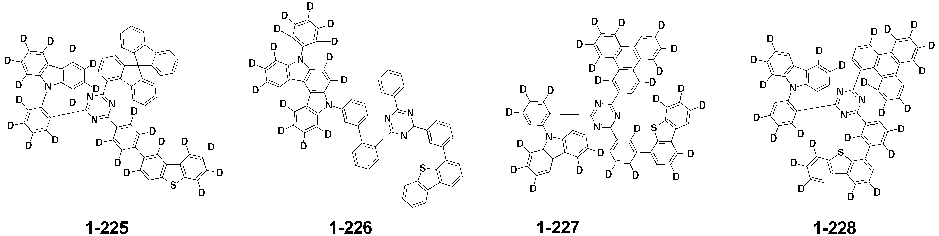

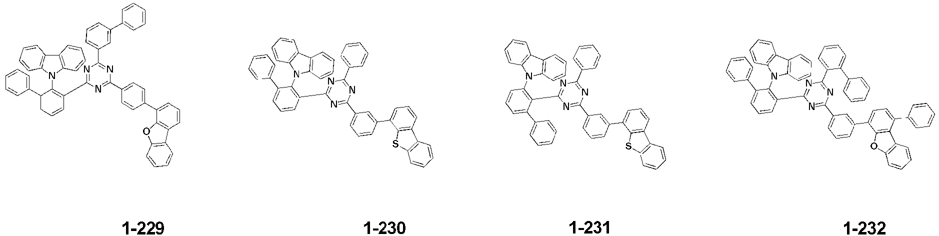

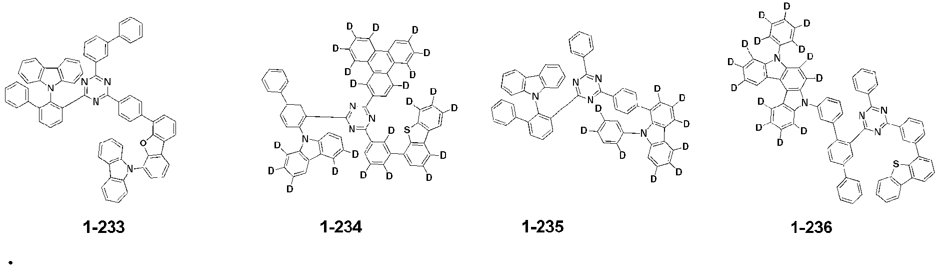

7. An organic light-emitting device comprising:
a first electrode;
a second electrode disposed to face the first electrode; and
one or more organic material layers disposed between the first electrode and the second electrode,
wherein at least one of the organic material layers comprises the heterocyclic compound according to claim 1.

8. An organic light-emitting device according to claim 7,
wherein the organic material layer includes an emission layer, and the emission layer includes the heterocyclic compound according to claim 1.

9. An organic light-emitting device according to claim 7,
wherein the organic material layer includes an emission layer, and the emission layer includes a host material,
wherein the host material includes the heterocyclic compound according to claim 1.

10. An organic light-emitting device according to claim 7,
which the organic material layer further includes the heterocyclic compound represented by Chemical Formula 2 or Chemical Formula 3: 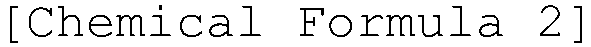 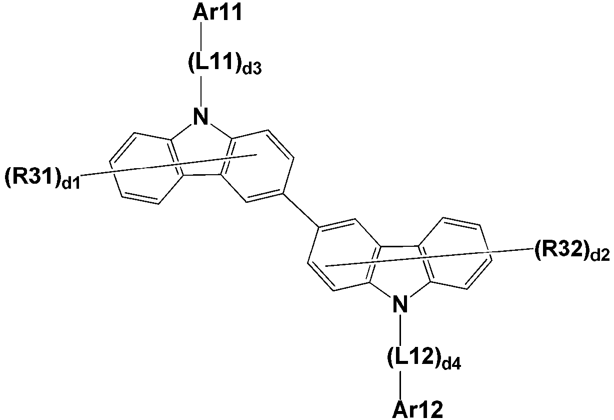 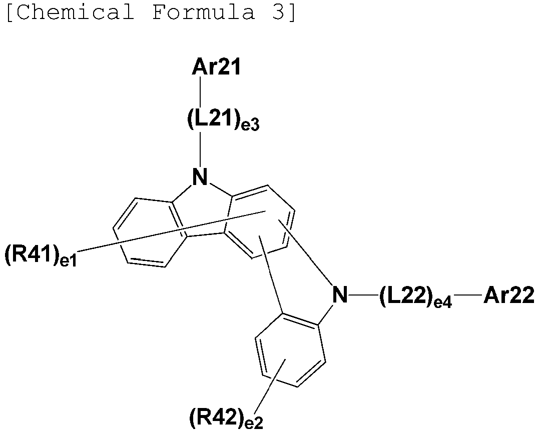
in Chemical Formulas 2 and 3,
R31, R32, R41, and R42 are the same as or different from each other, and each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - P(=O)R201R202; -SiR201R202R203; and -NR201R202; or two or more adjacent groups may be bonded to each other to form a substituted or unsubstituted C6 to C60 aromatic hydrocarbon ring or a substituted or unsubstituted C2 to C60 heterocyclic ring, wherein R201, R202, and R203 are the same as or different from each other, and each independently a substituted or unsubstituted C1 to C60 alkyl group; a substituted or unsubstituted C6 to C60 aryl group; or a substituted or unsubstituted C2 to C60 heteroaryl group,
d1 is an integer of 0 to 7, and when d1 is 2 or more, R31 are the same as or different from each other,
d2 is an integer of 0 to 7, and when d2 is 2 or more, R32 are the same as or different from each other,
e1 is an integer of 0 to 6, and when e1 is 2 or more, R41 are the same as or different from each other,
e2 is an integer of 0 to 4, and when e2 is 2 or more, R42 are the same as or different from each other,
Ar11, Ar12, Ar21, and Ar22 are the same as or different from each other, and each independently a cyano group; - SiR201R202R203; a substituted or unsubstituted C6 to C60 aryl group; or a substituted or unsubstituted C2 to C60 heteroaryl group, wherein R201, R202, and R203 are the same as or different from each other, and each independently a substituted or unsubstituted C1 to C60 alkyl group; a substituted or unsubstituted C6 to C60 aryl group; or a substituted or unsubstituted C2 to C60 heteroaryl group,
L11, L12, L21, and L22 are the same as or different from each other, and each independently a direct bond; a substituted or unsubstituted C6 to C60 arylene group; or a substituted or unsubstituted C2 to C60 heteroarylene group,
d3 is an integer of 0 to 5, and when d3 is 2 or more, L11 are the same as or different from each other,
d4 is an integer of 0 to 5, and when d4 is 2 or more, L12 are the same as or different from each other,
e3 is an integer of 0 to 5, and when e3 is 2 or more, L21 are the same as or different from each other,
e4 is an integer of 0 to 5, and when e4 is 2 or more, L22 are the same as or different from each other.

11. An organic light-emitting device according to claim 10,
wherein the heterocyclic compound represented by Chemical Formula 2 is represented by any one of the following compounds:

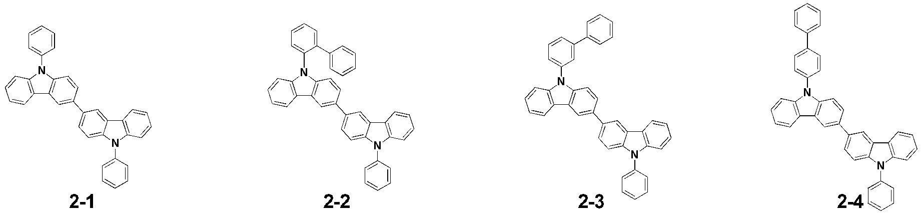

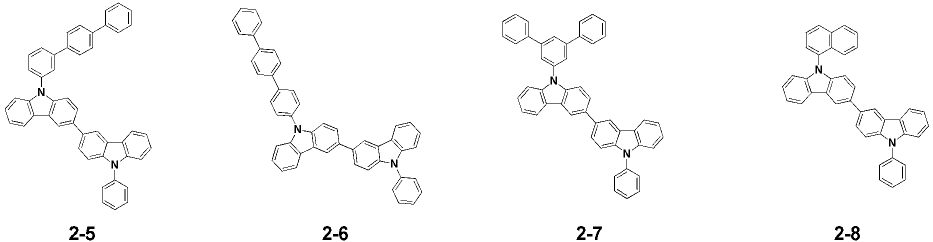

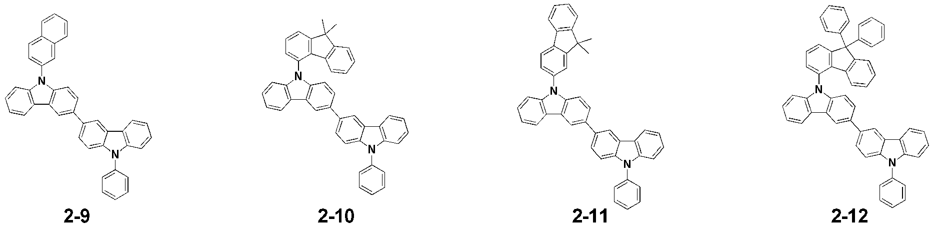

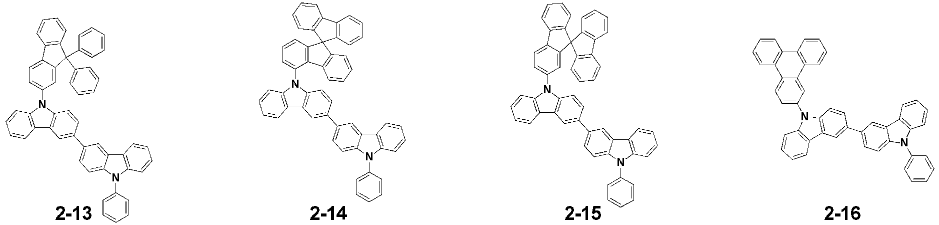

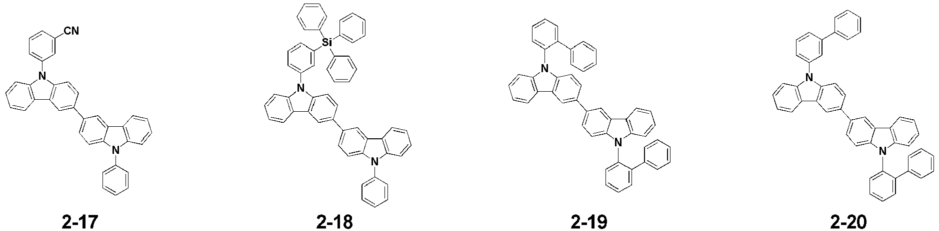

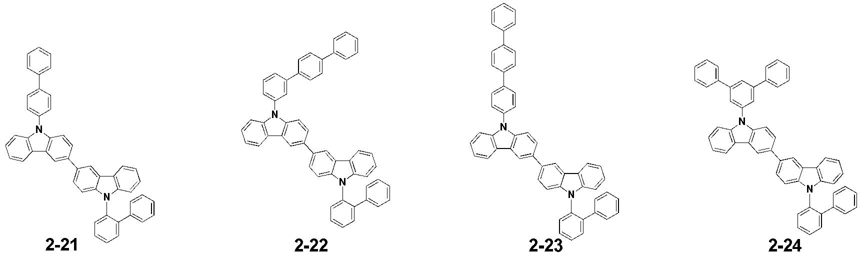

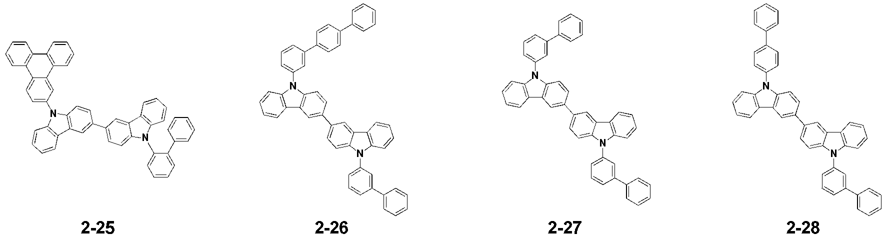

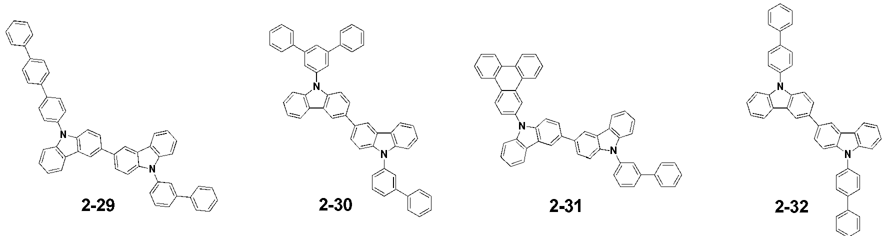

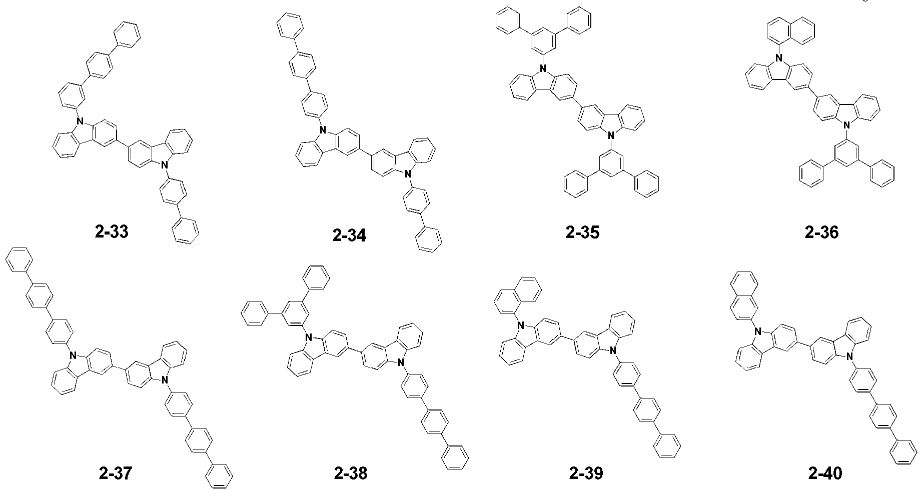

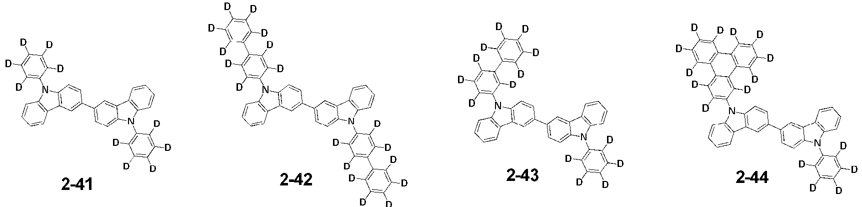

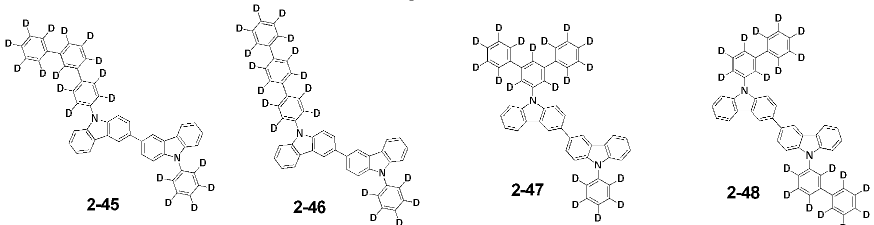

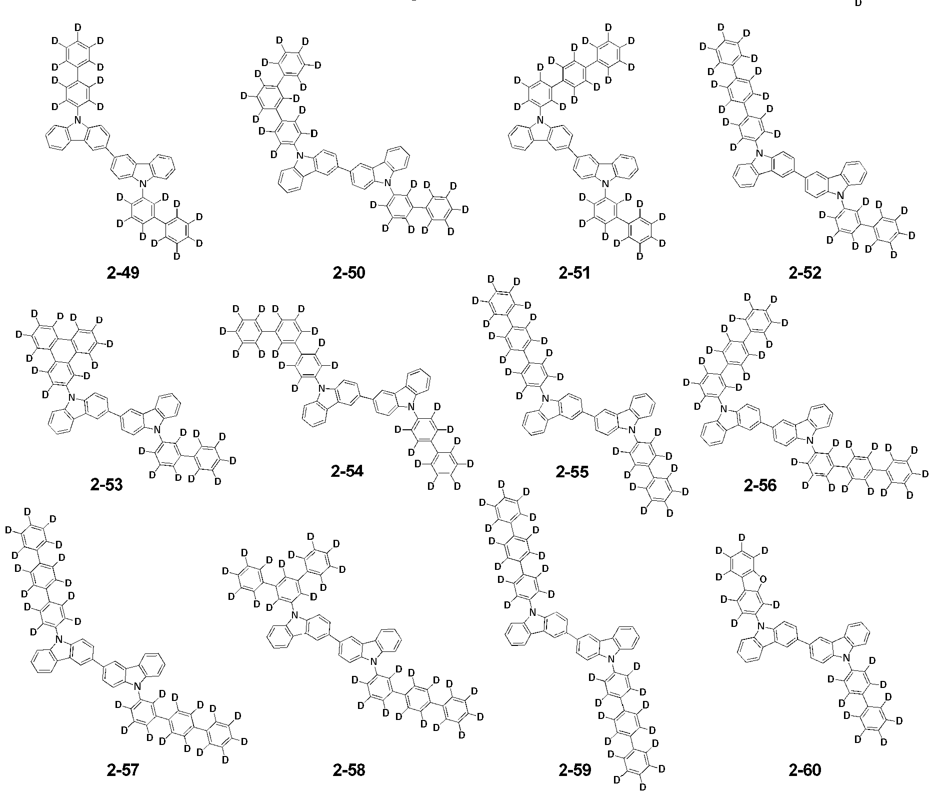

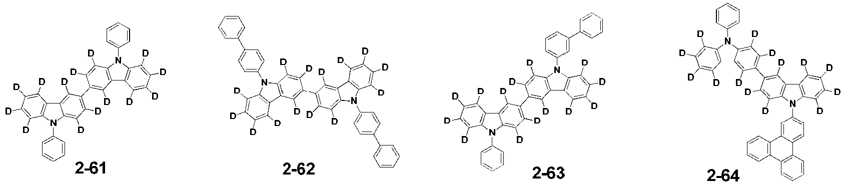

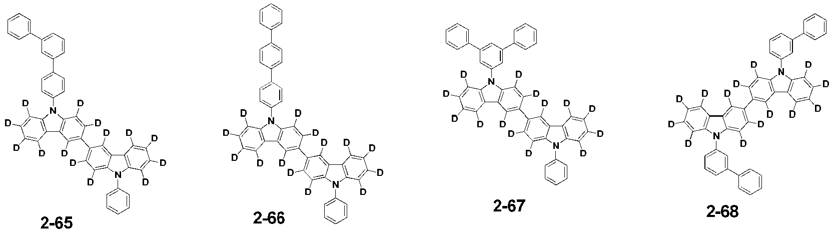

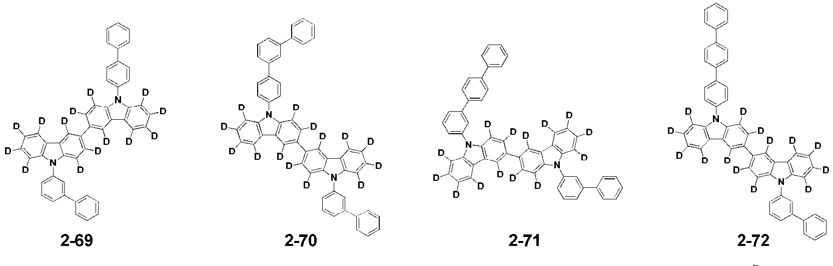

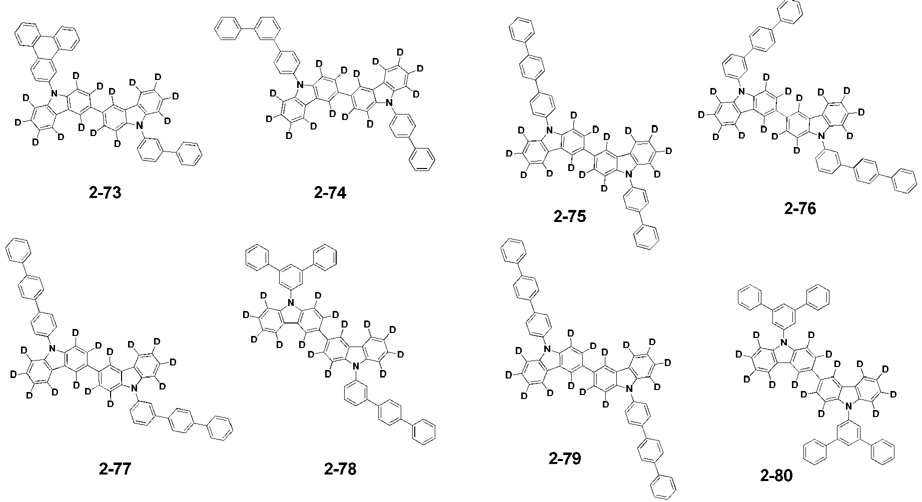

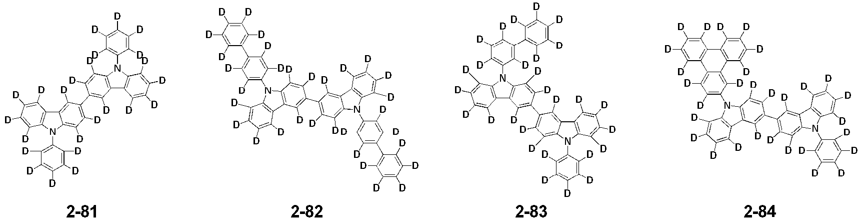

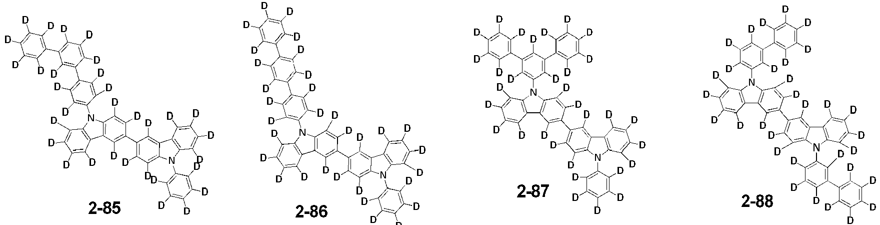

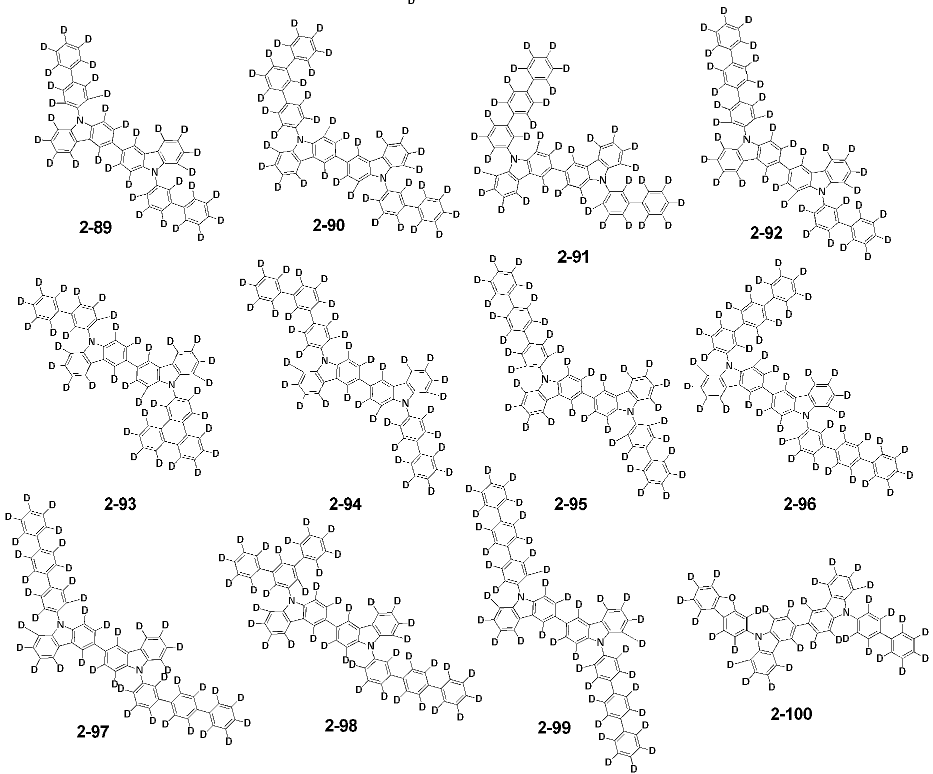

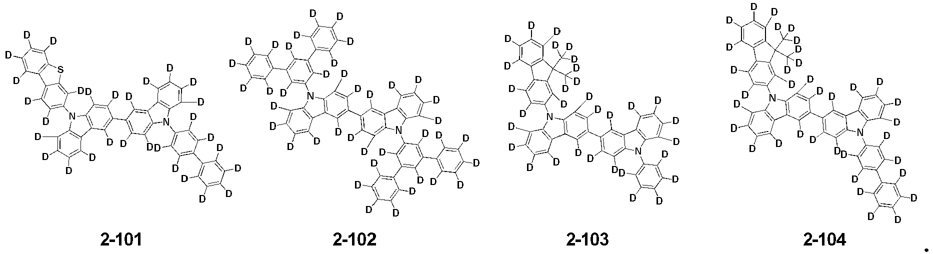

12. An organic light-emitting device according to claim 10,
Wherein the heterocyclic compound represented by Chemical Formula 3 is represented by any one of the following Formulas 3-1 to 3-6: 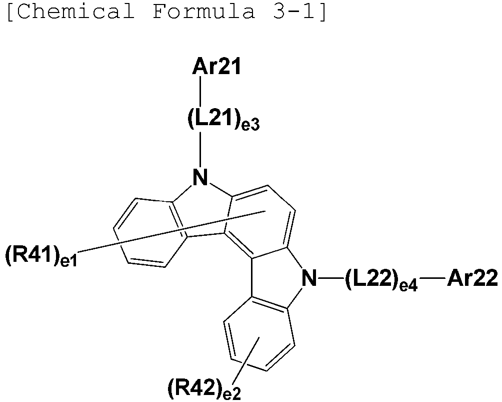 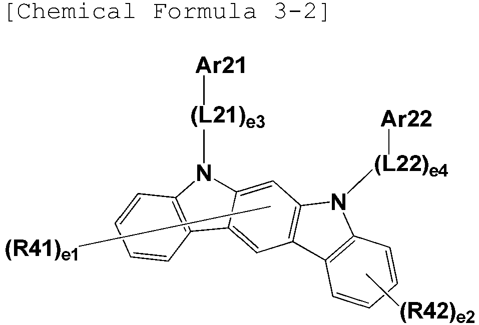 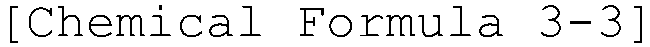 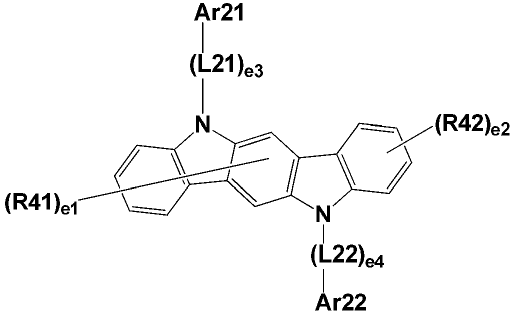 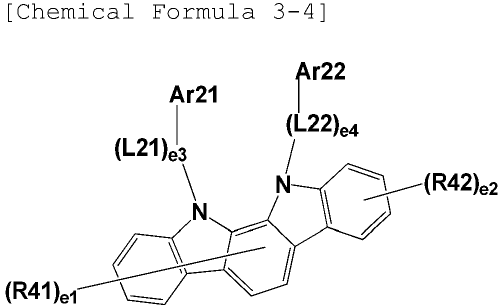 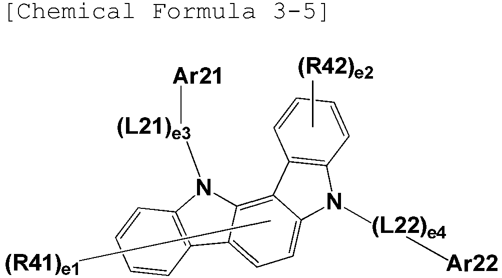 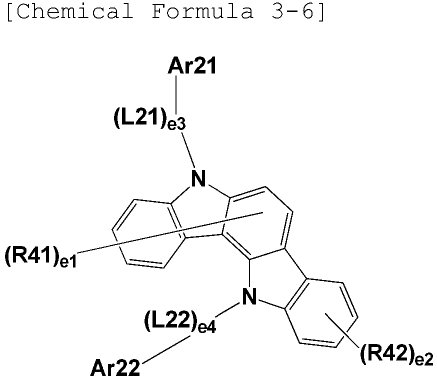
in Formulas 3-1 to 3-6,
R41, R42, Ar21, Ar22, L21, L22, and e1 to e4 are the same as defined in Chemical Formula 3.

13. An organic light-emitting device according to claim 10,
wherein the heterocyclic compound represented by Chemical Formula 3 is represented by any one of the following compounds:

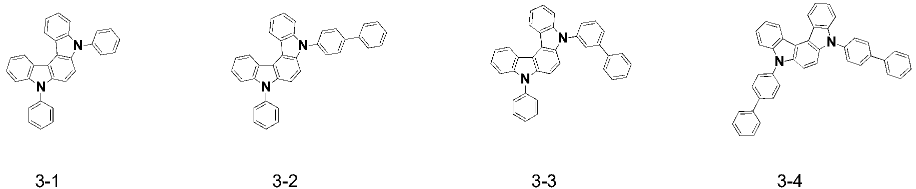

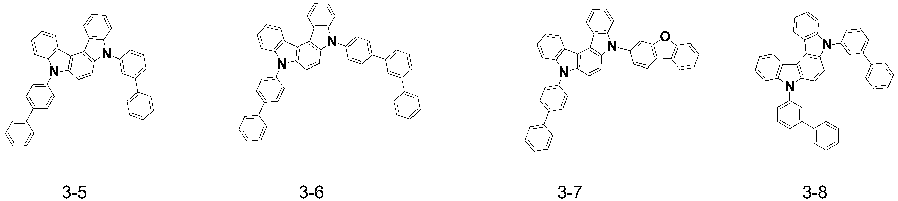

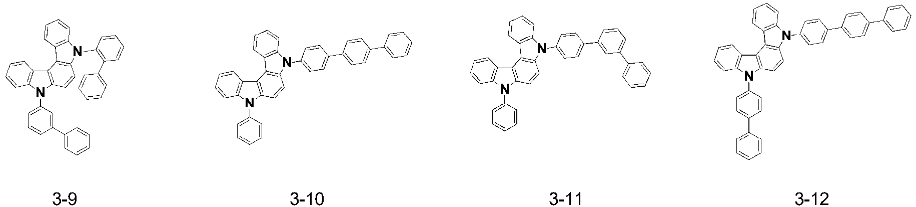

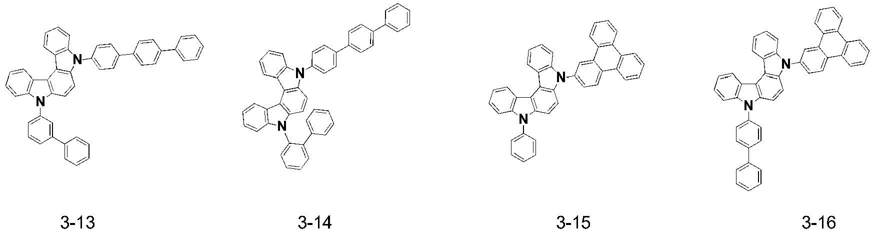

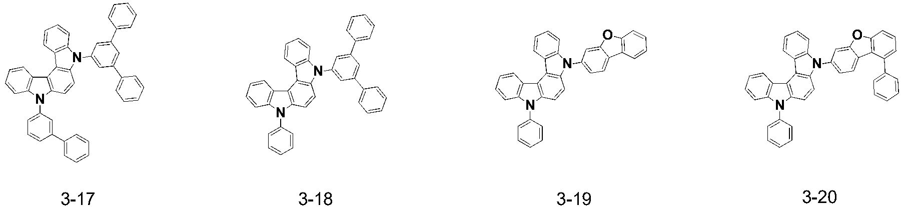

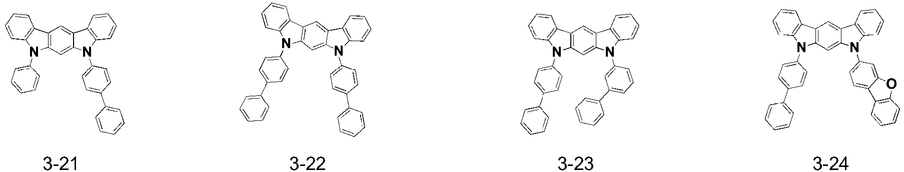

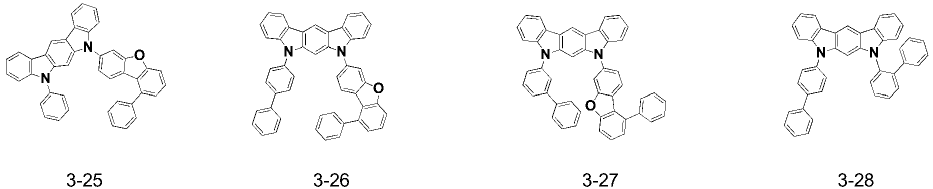

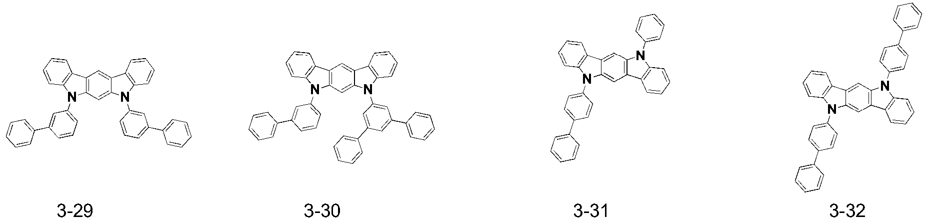

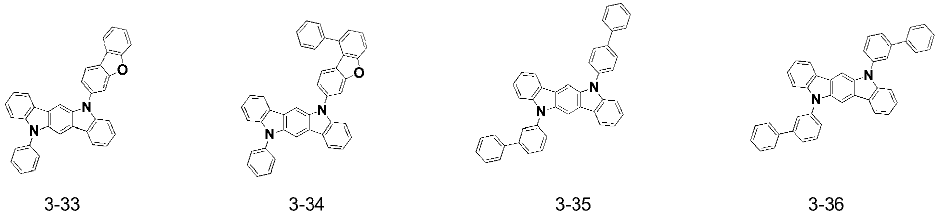

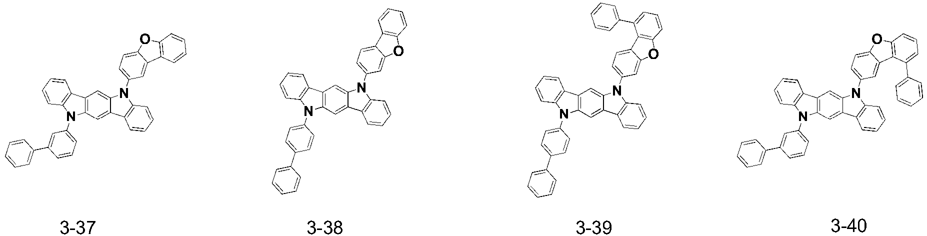

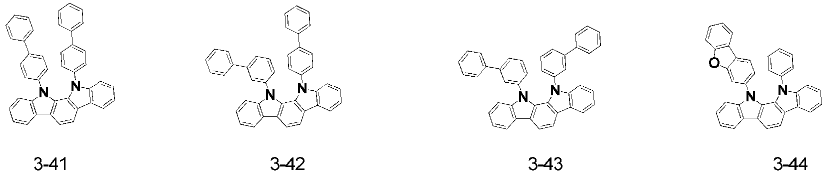

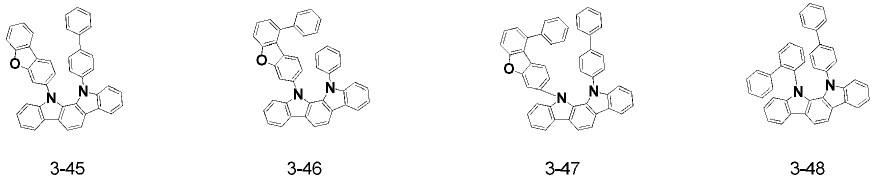

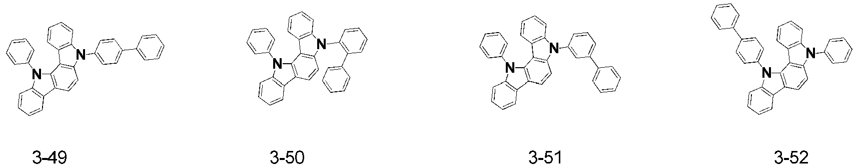

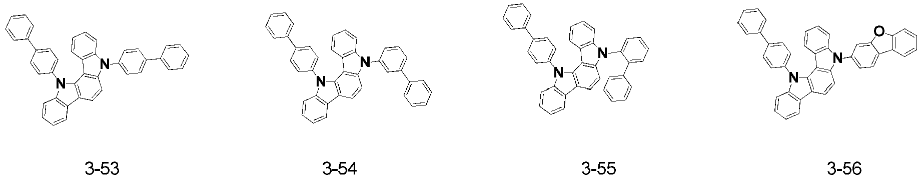

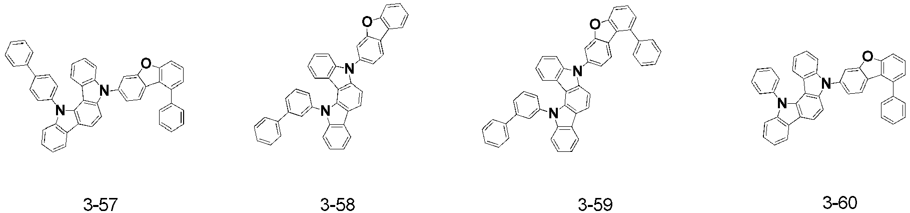

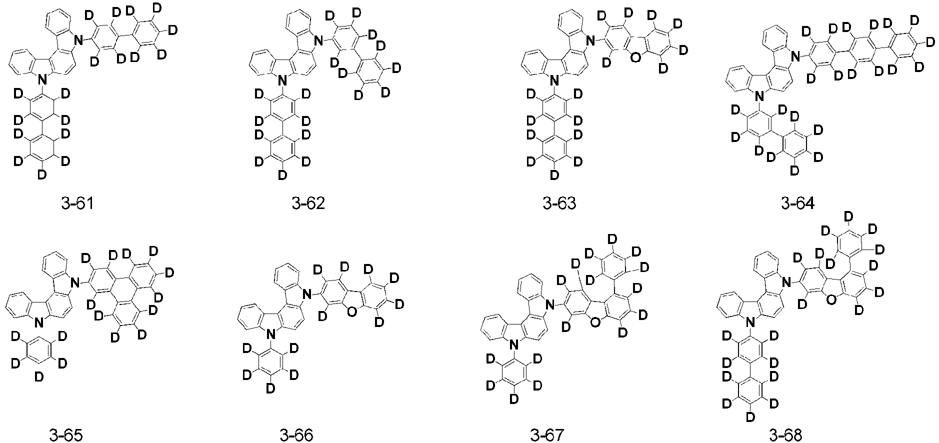

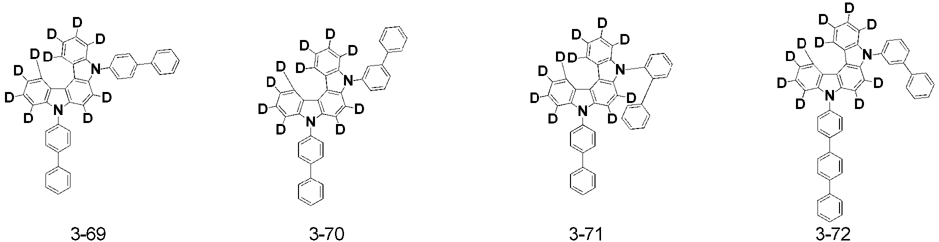

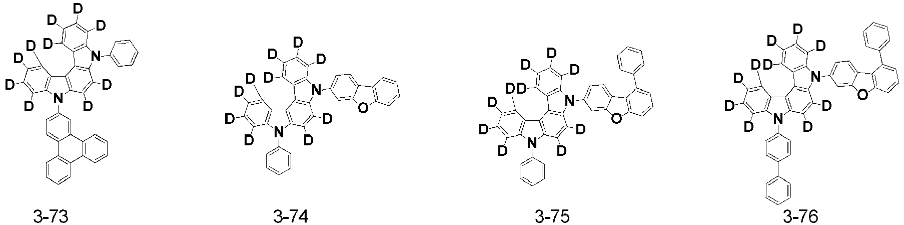

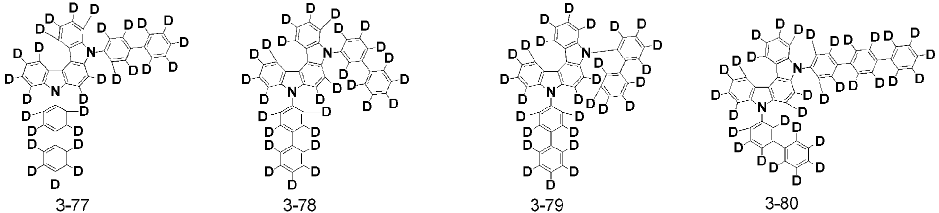

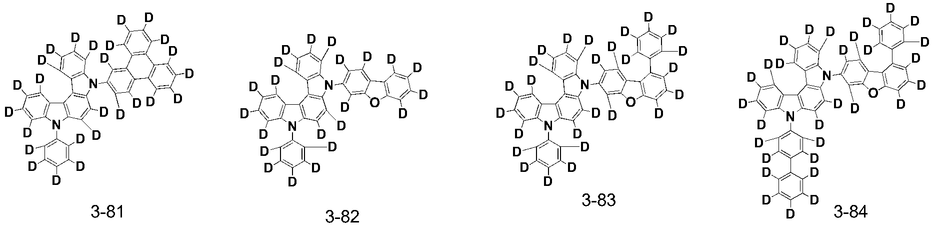

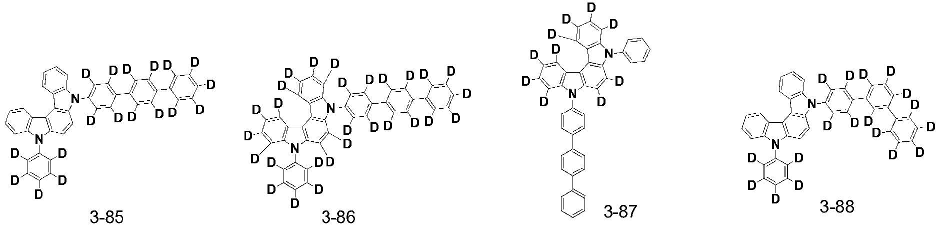

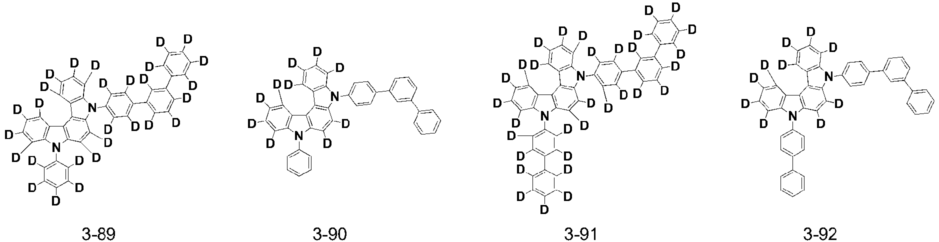

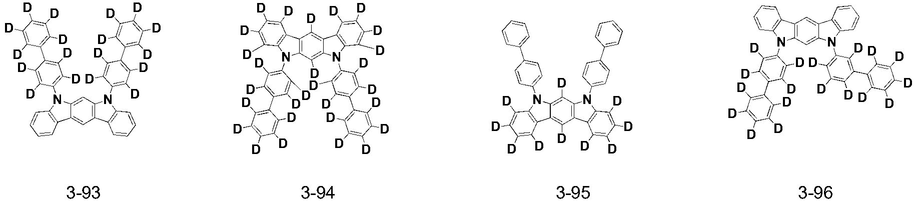

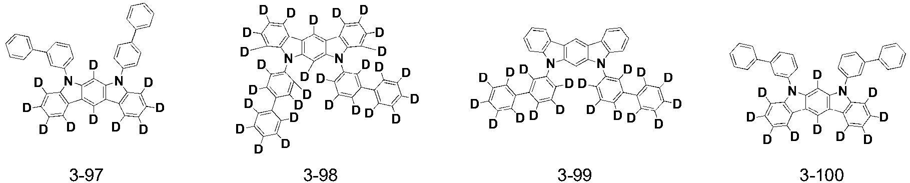

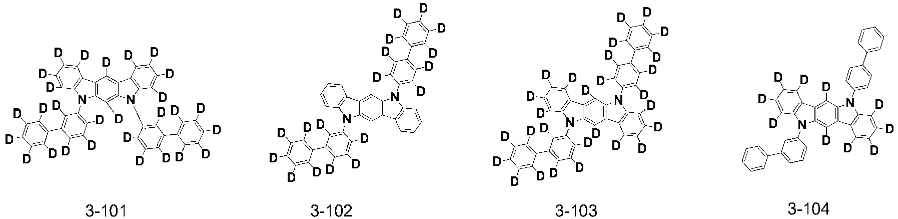

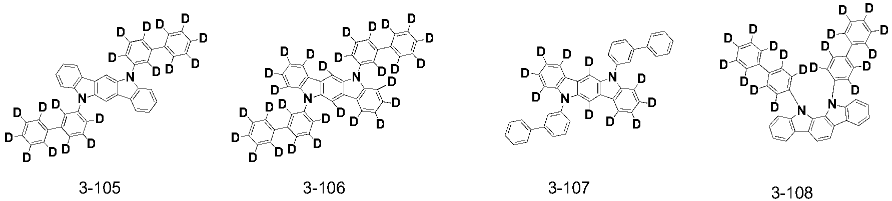

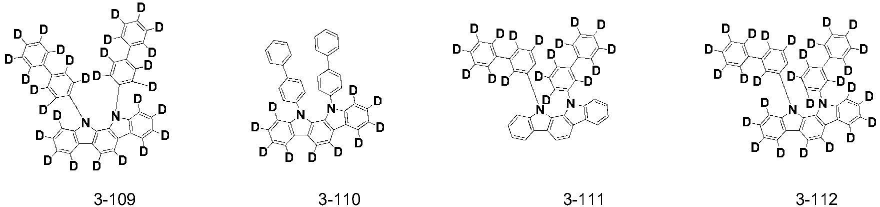

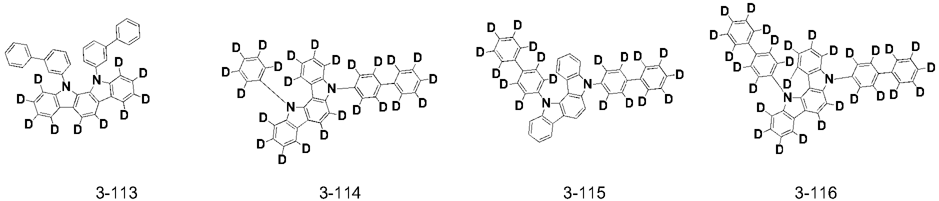

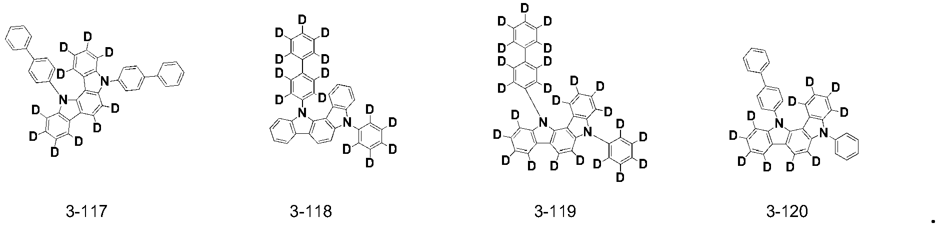

14. An organic light-emitting device according to claim 10,
wherein the heterocyclic compound represented by Chemical Formula 2 or the heterocyclic compound represented by Chemical Formula 3 does not include deuterium as a substituent, or the deuterium content relative to the total number of hydrogen atoms and deuterium atoms is 1% to 100%.

15. An organic light-emitting device according to claim 7,
wherein the organic light-emitting device further include one or more layers selected from the group consisting of a emission layer, a hole injection layer, a hole transport layer, an electron injection layer, an electron transport layer, an electron blocking layer, and a hole blocking layer.

16. A composition for an organic material layer comprising the heterocyclic compound represented by Chemical Formula 1 according to claim 1 and the heterocyclic compound represented by Chemical Formula 2 below or the heterocyclic compound represented by Chemical Formula 3 below: 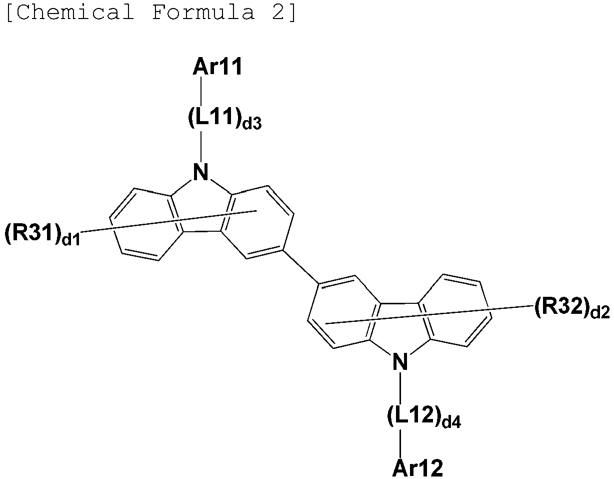 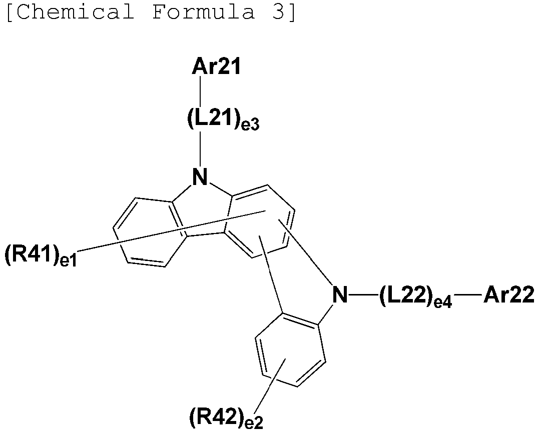 in Chemical Formulas 2 and 3,
R31, R32, R41, and R42 are the same as or different from each other, and each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - P(=O)R201R202; -SiR201R202R203; and -NR201R202; or two or more adjacent groups may be bonded to each other to form a substituted or unsubstituted C6 to C60 aromatic hydrocarbon ring or a substituted or unsubstituted C2 to C60 heterocyclic ring, wherein R201, R202, and R203 are the same as or different from each other, and each independently a substituted or unsubstituted C1 to C60 alkyl group; a substituted or unsubstituted C6 to C60 aryl group; or a substituted or unsubstituted C2 to C60 heteroaryl group,
d1 is an integer of 0 to 7, and when d1 is 2 or more, R31 are the same as or different from each other,
d2 is an integer of 0 to 7, and when d2 is 2 or more, R32 are the same as or different from each other,
e1 is an integer of 0 to 6, and when e1 is 2 or more, R41 are the same as or different from each other,
e2 is an integer of 0 to 4, and when e2 is 2 or more, R42 are the same as or different from each other,
Ar11, Ar12, Ar21, and Ar22 are the same as or different from each other, and each independently a cyano group; - SiR201R202R203; a substituted or unsubstituted C6 to C60 aryl group; or a substituted or unsubstituted C2 to C60 heteroaryl group, wherein R201, R202, and R203 are the same as or different from each other, and each independently a substituted or unsubstituted C1 to C60 alkyl group; a substituted or unsubstituted C6 to C60 aryl group; or a substituted or unsubstituted C2 to C60 heteroaryl group,
L11, L12, L21, and L22 are the same as or different from each other, and each independently a direct bond; a substituted or unsubstituted C6 to C60 arylene group; or a substituted or unsubstituted C2 to C60 heteroarylene group,
d3 is an integer of 0 to 5, and when d3 is 2 or more, L11 are the same as or different from each other,
d4 is an integer of 0 to 5, and when d4 is 2 or more, L12 are the same as or different from each other,
e3 is an integer of 0 to 5, and when e3 is 2 or more, L21 are the same as or different from each other,
e4 is an integer of 0 to 5, and when e4 is 2 or more, L22 are the same as or different from each other.

17. A composition for an organic material layer according to claim 16,
wherein a weight ratio of the heterocyclic compound represented by Chemical Formula 1 to the heterocyclic compound represented by Chemical Formula 2 or the heterocyclic compound represented by Chemical Formula 3 is from 1:9 to 9:1.
